# EUROPEAN PATENT APPLICATION

(11) **EP 4 234 015 A2**
(43) Date of publication of application: **30.08.2023**
(21) Application number: 23162149.1
(22) Date of filing: 17.03.2014
(51) Int. Cl.: A61P 5/26

(54) **METHODS OF TREATING TESTOSTERONE DEFICIENCY**

(30) Priority: 15.03.2013 US 201361794055 P
(62) Divisional of application: 14765688.8
(71) Applicant: Tolmar, Inc., Fort Collins, CO 80526 (US)
(72) Inventor: DUDLEY, Robert E., North Brook, IL, 60062 (US); CONSTANTINIDES, Panayotis P., North Brook, IL, 60062 (US); LONGSTRETH, James A., North Brook, IL, 60062 (US)
(74) Representative: Steffan & Kiehne Patentanwälte PartG mbB

(57) **Abstract**

Methods of treating a testosterone deficiency or its symptoms with a pharmaceutical formulation of testosterone esters are provided. The methods are designed toprovide optimum serum testosterone levels over an extended period.

## Description

This application claims the benefit of priority of United States Provisional Application No. 61/794,055, filed March 15, 2013, the disclosure of which is hereby incorporated by reference as if written herein in its entirety.

The present invention relates to treatments for testosterone deficiency and, in particular, methods utilizing oral formulations of testosterone esters that optimize the serum testosterone concentration during chronic treatment.

Testosterone (T) is a primary androgenic hormone produced in the interstitial cells of the testes and is responsible for normal growth, development and maintenance of male sex organs and secondary sex characteristics (e.g., deepening voice, muscular development, facial hair, etc.). Throughout adult life, testosterone is necessary for proper functioning of the testes and its accessory structures, prostate and seminal vesicle; for sense of well-being; and for maintenance of libido, erectile potency.

Testosterone deficiency--insufficient secretion of T characterized by low serum T concentrations--can give rise to medical conditions (e.g., hypogonadism) in males. Symptoms associated with male hypogonadism include impotence and decreased sexual desire, fatigue and loss of energy, mood depression, regression of secondary sexual characteristics, decreased muscle mass, and increased fat mass. Furthermore, hypogonadism in men is a risk factor for osteoporosis, metabolic syndrome, type II diabetes and cardiovascular disease.

Various testosterone replacement therapies are commercially available for the treatment of male hypogonadism. Pharmaceutical preparations include both testosterone and testosterone derivatives in the form of intramuscular injections, implants, oral tablets of alkylated T (e.g., methyltestosterone), topical gels, or topical patches. All of the current T therapies, however, fail to adequately provide an easy and clinically effective method of delivering T. For example, intramuscular injections are painful and are associated with significant fluctuations in serum T levels between doses; T patches are generally associated with levels of T in the lower range of normal (i.e., clinically ineffective) and often cause substantial skin irritation; and T gels have been associated with unsafe transfer of T from the user to women and children. As well, the sole "approved" oral T therapy, methyltestosterone, is associated with a significant occurrence of liver toxicity. Over time, therefore, the current methods of treating testosterone deficiency suffer from poor compliance and thus unsatisfactory treatment of men with low T. For example, in a recently published study, patient adherence to topical T replacement therapy at 6 months was only 34.7% and by 12 months, only 15.4% of patients continued on topical T therapy (Medication Adherence and Treatment Patterns for Hypogonadal Patients Treated with Topical Testosterone Therapy: A Retrospective Medical Claims Analysis. Michael Jay Schoenfeld, Emily Shortridge, Zhanglin Cui and David Muram, Journal of Sexual Medicine March 2013).

Testosterone and its short-chain aliphatic esters are poorly bioavailable prodrugs of testosterone--owing to extensive first pass intestinal and hepatic metabolism. On the other end, long-chain aliphatic esters of testosterone having 16 or more carbons although bioavailable are undergoing very slow hydrolysis in vivo to release effective amounts of free --owing to extensive first pass intestinal and hepatic metabolism. On the other end, long-chain aliphatic esters of testosterone having 16 or more carbons, although bioavailable, undergo very slow hydrolysis in vivo and do not release effective amounts of free testosterone. Thus, with testosterone aliphatic ester prodrugs an optimum chain length is required for improved bioavailability, plasma hydrolysis and free testosterone release. For example, testosterone and testosterone esters with aliphatic side chains of less than 10 carbons in length are primarily absorbed via the portal circulation resulting in substantial, if not total, first pass metabolism. Fatty acid esters of medium and long chain fatty acids (i.e., 11 or more carbons) can be absorbed by intestinal lymphatics, but the longer the fatty acid chain length, the slower the rate and extent of hydrolysis of the ester by in vivo esterases to liberate testosterone thus resulting in poor (i.e., clinically ineffective) pharmacological activity.

Other than selection of a testosterone ester with an optimum side chain length, the formulation of the resulting testosterone ester presents unique challenges. The gastrointestinal environment is decidedly aqueous in nature, which requires that drugs must be solubilized for absorption. However, testosterone and particularly its esters are insoluble in water and aqueous media, and even if the T or T ester is solubilized initially in the formulation, the formulation must be able to maintain the drug in a soluble or dispersed form in the intestine without precipitation or, otherwise, coming out of solution. Simulated intestinal fluids are frequently employed to optimize the formulation in vitro and correlate the in vitro behavior to in vivo performance as reflected in the pharmacokinetic parameters. Furthermore, an oral T formulation must, effectively release T or T ester according to a desired release profile. Hence, an effective formulation of T or T ester must balance good solubility with optimum release and satisfaction of a targeted plasma or serum concentration profile and therapeutic index requirements for testosterone therapy.

For these reasons, among others, no oral formulation of testosterone or testosterone esters has been approved by the United States Food and Drug Administration (FDA) to date. In fact, the only oral testosterone product ever approved to date by the FDA is methyltestosterone (in which a methyl group covalently bound to a testosterone "nucleus" at the C-17 position to inhibit hepatic metabolism; note, also, that methyltestosterone is a chemical derivative and not a prodrug of testosterone) and this approval occurred several decades ago. Unfortunately, use of methyltestosterone has been associated with a significant incidence of liver toxicity, and it is rarely prescribed to treat men with low testosterone.

As noted above, fatty acid esters of testosterone provide yet another mode of potential delivery of testosterone to the body (i.e., as a "prodrug"). Once absorbed, testosterone can be liberated from its ester via the action of non-specific tissue and plasma esterases. Furthermore, by increasing the relative hydrophobicity of the testosterone moiety and the lipophilicity of the resulting molecule as determined by its n-octanol-water partition coefficient (log P) value, such prodrugs can be absorbed, primarily via the intestinal lymphatics, thus reducing first-pass metabolism by the liver. In general, lipophilic compounds having a log P value of at least 5 and oil (triglyceride) solubility of at least 50 mg/mL are transported primarily via the lymphatic system.

Despite their promise, prodrugs of testosterone, including testosterone esters, have not been formulated in a manner to achieve effective and sustained serum testosterone levels at eugonadal levels (i.e., average serum T concentration falling in the range of about 300-1100 ng/dL). In fact, an orally administered pharmaceutical preparation of a testosterone prodrug, including testosterone esters, has yet to be approved by the FDA.

Thus, there remains a need for an oral formulation of a testosterone ester, which provides optimum serum testosterone levels that are clinically effective to treat hypogonadal men (i.e., those with a serum T concentration of <300 ng/dL) over an extended period.

Thus, in various embodiments, the present invention provides a method of treating chronic testosterone deficiency in a subject in need thereof comprising the steps of:
a) administering daily to the subject a dose of an oral pharmaceutical composition comprising a testosterone ester solubilized in a carrier comprising at least one lipophilic surfactant and at least one hydrophilic surfactant;
b) measuring the serum testosterone concentration in the subject; and
c) increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL.

In certain embodiments, the steps a.- c. are repeated until the serum testosterone concentration in the subject is between about 250 and about 700 ng/dL.

In various embodiments, the initial amount of testosterone ester in the oral pharmaceutical composition is equivalent to about 200 mg of testosterone. In certain embodiments, the oral pharmaceutical composition comprises testosterone undecanoate. In particular embodiments, the oral pharmaceutical composition administered comprises about 317mg of testosterone undecanoate.

In various embodiments, the amount of testosterone ester in the administered oral pharmaceutical composition is increased by the equivalent of about 25 to about 50 mg of testosterone when the serum testosterone concentration in the subject is less than about 250 ng/dL, and decreased by the equivalent of about 25 to about 50 mg of testosterone when the serum testosterone concentration in the subject is greater than about 700 ng/dL. In certain embodiments, the dose of testosterone undecanoate in the administered oral pharmaceutical composition is increased by about 40 to about 80 mg measured serum testosterone concentration in the subject is less than about 250 ng/dL, and decreased by about 40 to about 80 mg when the measured serum testosterone concentration in the subject is greater than about 700 ng/dL.

In various embodiments, the oral pharmaceutical composition is administered twice daily.

In various embodiments, the serum testosterone concentration is measured two to six hours after administering the oral pharmaceutical composition. In certain embodiments, the serum testosterone concentration is measured three to five hours after administering the oral pharmaceutical composition.

In various embodiments, the serum testosterone concentration is measured via a radioimmunoassay, an immunometric assay, or a liquid chromatography tandem mass spectrometry (LC-MS/MS) assay.

In various embodiments, the serum testosterone concentration is measured after at least fourteen days of daily treatment with the oral pharmaceutical composition. In certain embodiments, the serum testosterone concentration is measured after at least thirty days of daily treatment with the oral pharmaceutical composition.

In various embodiments, the oral pharmaceutical composition is administered within about 30 minutes of consuming a meal wherein at least about 20 percent of the calories are derived from fat.

In various embodiments, the oral pharmaceutical composition comprises about 10-20 percent by weight of solubilized testosterone ester, about 5-20 percent by weight of hydrophilic surfactant, about 50-70 percent by weight of lipophilic surfactant; and about 10-15 percent by weight of digestible oil, wherein the oral pharmaceutical composition is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

In various embodiments, the testosterone ester is a short-chain (C₂-C₆) or a medium-chain (C₇-C₁₃) fatty acid ester. In certain embodiments, the testosterone ester is a medium-chain fatty acid ester selected from the group consisting of testosterone cypionate, testosterone octanoate, testosterone enanthate, testosterone decanoate, and testosterone undecanoate, or combinations thereof. In particular embodiments, the testosterone ester is testosterone undecanoate.

In various embodiments, the hydrophilic surfactant exhibits an HLB of 10 to 45.

In certain embodiments, the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof. In particular embodiments, the hydrophilic surfactant is a hydrogenated castor oil ethoxylate.

In various embodiments, the lipophilic surfactant exhibits an HLB of less than 10. In certain embodiments, the lipophilic surfactant exhibits an HLB of less than 5. In particular embodiments, the lipophilic surfactant exhibits an HLB of 1 to 2.

In various embodiments, the lipophilic surfactant is a fatty acid selected from the group consisting of octanoic acid, decanoic acid, undecanoic acid, lauric acid, myristic acid, palmitic acid, pamitoleic, stearic acid, oleic acid, linoleic acid, alpha- and gamma linolenic acid, arachidonic acid, glyceryl monolinoleate and combinations thereof.

In various embodiments, the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil.

In various embodiments, the oral pharmaceutical composition comprises one or more additional lipid-soluble therapeutic agents. In certain embodiments, the additional lipid-soluble therapeutic agents are selected from the group consisting of a synthetic progestin, an inhibitor of type-I and/or type II 5α-reductase, an inhibitor of CYP3A4, finasteride, dutasteride and combinations thereof. In particular embodiments, the one or more additional lipid-soluble therapeutic agents comprises a second testosterone ester.

In various embodiments, the oral pharmaceutical composition is filled into a hard or soft gelatin capsule.

In various embodiments, the oral pharmaceutical composition is a liquid, semi-solid or solid dosage form.

In various embodiments, the oral pharmaceutical composition exhibits a percent (%) in vitro dissolution profile in 5% Triton X-100 solution in phosphate buffer, pH 6.8, indicating release from the composition of substantially all of the solubilized testosterone ester within about 1 hour.

In certain embodiments, the oral pharmaceutical composition comprises: about 10-20 percent by weight of solubilized testosterone undecanoate, about 5-20 percent by weight of a hydrogenated castor oil ethoxylate, about 50-70 percent by weight of oleic acid; and about 10-15 percent by weight of digestible oil, wherein the oral pharmaceutical composition is free of ethanol and exhibits a percent (%) in vitro dissolution profile in 5% Triton X-100 solution in phosphate buffer, pH 6.8 that indicates release from the composition of substantially all of the solubilized testosterone ester within about 2 hours.

In certain embodiments, the oral pharmaceutical composition comprises: about 15-20 percent by weight of solubilized testosterone ester, about 5-20 percent by weight of hydrophilic surfactant, about 50-70 percent by weight of lipophilic surfactant; and about 10-15 percent by weight of digestible oil, wherein the oral pharmaceutical composition is free of ethanol and exhibits a percent (%) in vitro dissolution profile in 5% Triton X-100 solution in phosphate buffer, pH 6.8 that indicates release from the composition of substantially all of the solubilized testosterone ester within about 2 hours.

In certain embodiments, the oral pharmaceutical composition comprises: about 15-20 percent by weight of solubilized testosterone ester, about 5-20 percent by weight of hydrophilic surfactant, about 50-70 percent by weight of lipophilic surfactant; and about 1-10 percent by weight of polyethylene glycol 8000, wherein the oral pharmaceutical composition is free of ethanol and exhibits a percent (%) in vitro dissolution profile in 5% Triton X-100 solution in phosphate buffer, pH 6.8 that indicates release from the composition of substantially all of the solubilized testosterone ester within about 2 hours.

In various embodiments, the testosterone ester is a short-chain (C₂-C₆) or a medium-chain (C₇-C₁₃) fatty acid ester. In certain embodiments, the testosterone ester is a medium-chain fatty acid ester selected from the group consisting of testosterone cypionate, testosterone octanoate, testosterone enanthate, testosterone decanoate, and testosterone undecanoate, or combinations thereof. In particular embodiments, the testosterone ester is testosterone undecanoate.

In various embodiments, the hydrophilic surfactant exhibits an HLB of 10 to 45.

In certain embodiments, the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof. In particular embodiments, the hydrophilic surfactant is polyoxyethylene (40) hydrogenated castor oil.

In various embodiments, the lipophilic surfactant exhibits an HLB of less than 10.

In various embodiments, the lipophilic surfactant is a fatty acid selected from the group consisting of octanoic acid, decanoic acid, undecanoic acid, lauric acid, myristic acid, palmitic acid, pamitoleic, stearic acid, oleic acid, linoleic acid, alpha- and gamma linolenic acid, arachidonic acid, glyceryl monolinoleate and combinations thereof.

In various embodiments, the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil.

In various embodiments, the oral pharmaceutical composition comprises one or more additional lipid-soluble therapeutic agents. In certain embodiments, the additional lipid-soluble therapeutic agents are selected from the group consisting of a synthetic progestin, an inhibitor of type-I and/or type II 5α-reductase, an inhibitor of CYP3A4, finasteride, dutasteride and combinations thereof. In particular embodiments, the one or more additional lipid-soluble therapeutic agents comprises a second testosterone ester.

In various embodiments, the oral pharmaceutical composition is filled into a hard or soft gelatin capsule.

In various embodiments, the oral pharmaceutical composition is a liquid, semi-solid or solid dosage form

In certain embodiments, the oral pharmaceutical composition exhibits a percent (%) in vitro dissolution profile in 5% Triton X-100 solution in phosphate buffer, pH 6.8, indicating release from the composition of substantially all of the solubilized testosterone ester within about 1 hour.

In various embodiments, the oral pharmaceutical composition comprises: about 15-20 percent by weight of solubilized testosterone undecanoate, about 5-20 percent by weight of a hydrogenated castor oil ethoxylate, about 50-70 percent by weight of oleic acid; and about 10-15 percent by weight of digestible oil, wherein the oral pharmaceutical composition is free of ethanol and exhibits a percent (%) in vitro dissolution profile in 5% Triton X-100 solution in phosphate buffer, pH 6.8,, which indicates release from the composition of substantially all of the solubilized testosterone ester within about 2 hours.

In various embodiments, the oral pharmaceutical composition comprises: about 15-20 percent by weight of solubilized testosterone ester, about 5-20 percent by weight of hydrophilic surfactant, about 50-70 percent by weight of a lipophilic surfactant which is a C₁₄-C₂₄ fatty acid; and about 10-15 percent by weight of digestible oil, wherein the oral pharmaceutical composition exhibits a percent (%) in vitro dissolution profile in 5% Triton X-100 solution in phosphate buffer, pH 6.8 that indicates release from the composition of substantially all of the solubilized testosterone ester within about 2 hours.

In various embodiments, the testosterone ester is a short-chain (C₂-C₆) or a medium-chain (C₇-C₁₃) fatty acid ester. In certain embodiments, the testosterone ester is a medium-chain fatty acid ester selected from the group consisting of testosterone cypionate, testosterone octanoate, testosterone enanthate, testosterone decanoate, and testosterone undecanoate, or combinations thereof. In particular embodiments, the testosterone ester is testosterone undecanoate.

In various embodiments, the hydrophilic surfactant exhibits an HLB of 10 to 45.

In certain embodiments, the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof. In particular embodiments, the hydrophilic surfactant is a hydrogenated castor oil ethoxylate.

In various embodiments, the lipophilic surfactant exhibits an HLB of less than 10. In certain embodiments, the lipophilic surfactant exhibits an HLB of less than 5. In particular embodiments, the lipophilic surfactant exhibits an HLB of 1 to 2.

In various embodiments, the lipophilic surfactant is a fatty acid selected from the group consisting of octanoic acid, decanoic acid, undecanoic acid, lauric acid, myristic acid, palmitic acid, pamitoleic, stearic acid, oleic acid, linoleic acid, alpha- and gamma linolenic acid, arachidonic acid, glyceryl monolinoleate and combinations thereof.

In various embodiments, the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil.

In various embodiments, the oral pharmaceutical composition comprises one or more additional lipid-soluble therapeutic agents. In certain embodiments, the additional lipid-soluble therapeutic agents are selected from the group consisting of a synthetic progestin, an inhibitor of type-I and/or type II 5α-reductase, an inhibitor of CYP3A4, finasteride, dutasteride and combinations thereof. In particular embodiments, the one or more additional lipid-soluble therapeutic agents comprises a second testosterone ester.

In various embodiments, the oral pharmaceutical composition is filled into a hard or soft gelatin capsule.

In various embodiments, the oral pharmaceutical composition is a liquid, semi-solid or solid dosage form.

In various embodiments, the oral pharmaceutical composition exhibits a percent (%) in vitro dissolution profile in 5% Triton X-100 solution in phosphate buffer, pH 6.8, indicating release from the composition of substantially all of the solubilized testosterone ester within about 1 hour.

In various embodiments, the oral pharmaceutical composition comprises: about 15-20 percent by weight of solubilized testosterone undecanoate, about 5-20 percent by weight of a hydrogenated castor oil ethoxylate, about 50-70 percent by weight of oleic acid; and about 10-15 percent by weight of digestible oil, wherein the oral pharmaceutical composition exhibits a percent (%) in vitro dissolution profile in 5% Triton X-100 solution in phosphate buffer, pH 6.8, which indicates release from the composition of substantially all of the solubilized testosterone ester within about 2 hours.

In various embodiments, the oral pharmaceutical composition comprises: about 15-20 percent by weight of solubilized testosterone ester, about 5-20 percent by weight of hydrophilic surfactant, and greater than about 50 percent by weight of lipophilic surfactant that is a C₁₄-C₂₄ fatty acid.

In certain embodiments, the oral pharmaceutical composition further comprises one or more digestible oils.

In certain embodiments, the oral pharmaceutical composition exhibits a percent (%) in vitro dissolution profile in 5% Triton X-100 solution in phosphate buffer, pH 6.8, which indicates release from the composition of substantially all of the solubilized testosterone ester within about 2 hours.

In various embodiments, the testosterone ester is a short-chain (C₂-C₆) or a medium-chain (C₇-C₁₃) fatty acid ester. In certain embodiments, the testosterone ester is a medium-chain fatty acid ester selected from the group consisting of testosterone cypionate, testosterone octanoate, testosterone enanthate, testosterone decanoate, and testosterone undecanoate, or combinations thereof. In particular embodiments, the testosterone ester is testosterone undecanoate.

In various embodiments, the hydrophilic surfactant exhibits an HLB of 10 to 45.

In certain embodiments, the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof. In particular embodiments, the hydrophilic surfactant is a hydrogenated castor oil ethoxylate.

In various embodiments, the lipophilic surfactant exhibits an HLB of less than 10. In certain embodiments, the lipophilic surfactant exhibits an HLB of less than 5. In particular embodiments, the lipophilic surfactant exhibits an HLB of 1 to 2.

In various embodiments, the lipophilic surfactant is a fatty acid selected from the group consisting of octanoic acid, decanoic acid, undecanoic acid, lauric acid, myristic acid, palmitic acid, pamitoleic, stearic acid, oleic acid, linoleic acid, alpha- and gamma linolenic acid, arachidonic acid, glyceryl monolinoleate and combinations thereof. In particular embodiments, the lipophilic surfactant is oleic acid. In particular embodiments, the lipophilic surfactant comprises 50-80 percent by weight of the composition.

In various embodiments, the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil.

In various embodiments, the oral pharmaceutical composition comprises one or more additional lipid-soluble therapeutic agents. In certain embodiments, the additional lipid-soluble therapeutic agents are selected from the group consisting of a synthetic progestin, an inhibitor of type-I and/or type II 5α-reductase, an inhibitor of CYP3A4, finasteride, dutasteride and combinations thereof. In particular embodiments, the one or more additional lipid-soluble therapeutic agents comprises a second testosterone ester.

In various embodiments, the oral pharmaceutical composition is filled into a hard or soft gelatin capsule.

In various embodiments, the oral pharmaceutical composition is a liquid, semi-solid or solid dosage form.

In certain embodiments, the composition is free of monohydric alcohol. In certain embodiments, the monohydric alcohol is chosen from C₂-C₁₈ aliphatic or aromatic alcohol. In particular embodiments, the monohydric alcohol is chosen from ethanol and benzyl alcohol.

In various embodiments, the oral pharmaceutical composition comprises testosterone undecanote solubilized in a carrier comprising at least one lipophilic surfactant and at least one hydrophilic surfactant in a total lipophilic surfactant to total hydrophilic surfactant ratio (w/w) falling in the range of about 6:1 to 3.5:1, which composition, upon once- or twice-daily oral administration, provides an average serum testosterone concentration at steady state falling in the range of about 300 to about 1100 ng/dL.

In particular embodiments, the oral pharmaceutical composition comprises at least one hydrophilic surfactant comprises Cremophor RH 40 (polyoxyethyleneglycerol trihydroxystearate).

In particular embodiments, the lipophilic surfactant comprises oleic acid. In particular embodiments, the oral pharmaceutical composition comprises about 18 to 22 percent by weight of a solubilized testosterone undecanoate. In particular embodiments, the testosterone undecanoate is solubilized in a carrier substantially free of ethanol. In particular embodiments, the oral pharmaceutical composition comprises 15 to 17 percent by weight of the at least one hydrophilic surfactant. In particular embodiments, the oral pharmaceutical composition comprises 50 to 55 percent by weight of the at least one lipophilic surfactant.

Thus, in particular embodiments, the present invention provide a method of treating chronic testosterone deficiency in a subject in need thereof comprising the steps of: administering daily to the subject a morning dose and an evening dose of an oral pharmaceutical composition comprising testosterone undecanoate, wherein each dose is administered within about 30 minutes of consuming a meal, for a period of at least thirty days, measuring the serum testosterone concentration in the subject about three to five hours following the morning administration of the oral pharmaceutical composition, increasing each dose of testosterone undecanoate administered in step a. by about 80 mg when the measured serum testosterone concentration in the subject is less than about 250 ng/dL, decreasing each dose of testosterone undecanoate administered in step a. by about 80 mg when the measured serum testosterone concentration in the subject is greater than about 700 ng/dL, and maintaining each dose of testosterone undecanoate administered in step a. when the measured serum testosterone concentration in the subject is between about 250 ng/dL and 700 ng/dL; and repeating steps a. - c. until the serum testosterone concentration in the subject is between about 250 and 700 ng/dL. In particular embodiments, the oral pharmaceutical composition comprises about 19.8 percent by weight of solubilized testosterone undecanoate, about 51.6 percent by weight of oleic acid, about 16.1 percent by weight of polyoxyethylene (40) hydrogenated castor oil, about 10 percent by weight of borage seed oil, about 2.5 percent by weight of peppermint oil, and about 0.03 percent by weight of butylated hydroxytoluene (BHT). In particular embodiments, each morning and evening dose initially comprises about 317 mg of testosterone undecanoate.

The oral pharmaceutical compositions provide optimum drug release without compromising the solubilization of the active ingredients. In various embodiments, the oral pharmaceutical composition exhibits a percent (%) in vitro dissolution profile in 5% Triton X-100 solution in phosphate buffer, pH 6.8, indicating release from the composition of substantially all of the solubilized testosterone ester within about 3 hours, preferably within about 2 hours, and more preferable, release occurs within about 1 hour.

Dietary fat content modulates serum T levels. Thus, in various embodiments, the oral pharmaceutical composition is administered with a meal that at least 20 percent of the calories are derived from fat.

### BRIEF DESCRIPTION OF THE DRAWING(S)

Figure 1 depicts Observed C_{avg} Values Compared to a Theoretical Normal Distribution.
Figure 2 depicts Observed C_{avg} Values Compared to Theoretical Log-Normal Distribution.
Figure 3 depicts a Schematic of the Distribution of C_{avg} Values in a Very Large Population of Patients after Administration of Identical Doses of TU to all Patients.
Figure 4 depicts Individual and Mean Baseline T Concentrations, by Treatment Period.
Figure 5 depicts a Relationship between C_{avg} and Cₘₐₓ on Day 7 of Dosing with TU.
Figure 6 depicts a schematic view of the interplay of C_{avg}, Cₘₐₓ and the variability in Cₘₐₓ.
Figure 7 C_{avg} and Cₘₐₓ Distributions (Normal) with Demarcations for Selected Threshold Concentrations. Left curve is C_{avg}, and left two dashed lines indicate limits of normal T range; right curve is Cₘₐₓ, and right three dashed lines critical Cₘₐₓ thresholds.
Figure 8 depicts a C_{avg} and Cₘₐₓ Distributions (Log-Normal) with Demarcations for Selected Threshold Concentrations. Left curve is C_{avg}, and left two dashed lines indicate limits of normal T range; right curve is Cₘₐₓ, and right three dashed lines critical Cₘₐₓ thresholds.
Figure 9A Correlation between C_{avg} & C(0); Figure 9B shows the Contingency Table Overlaid on Correlation Relationship. In each of these figures, the triangles represent formulation A batch AA, the diamonds represent formulation A batch BB, and the dashed line represents the regression.
Figure 10A shows Correlation between C_{avg} & C(1); Figure 10B shows the Contingency Table Overlaid on Correlation Relationship. In each of these figures, the triangles represent formulation A batch AA, the diamonds represent formulation A batch BB, and the dashed line represents the regression.
Figure 11A shows the Correlation between C_{avg} & C(1.5); Figure 11B shows the Contingency Table Overlaid on Correlation Relationship. In each of these figures, the triangles represent formulation A batch AA, the diamonds represent formulation A batch BB, and the dashed line represents the regression.
Figure 12A shows Correlation between C_{avg} & C(2); Figure 12B shows the Contingency Table Overlaid on Correlation Relationship. In each of these figures, the triangles represent formulation A batch AA, the diamonds represent formulation A batch BB, and the dashed line represents the regression.
Figure 13A provides Correlation between C_{avg} & C(3); Figure 13B shows the Contingency Table Overlaid on Correlation Relationship. In each of these figures, the triangles represent formulation A batch AA, the diamonds represent formulation A batch BB, and the dashed line represents the regression.
Figure 14A depicts Correlation between C_{avg} & C(4); Figure 14B shows show the Contingency Table Overlaid on Correlation Relationship. In each of these figures, the triangles represent formulation A batch AA, the diamonds represent formulation A batch BB, and the dashed line represents the regression.
Figure 15A depicts Correlation between C_{avg} & C(5); Figure 15B shows show the Contingency Table Overlaid on Correlation Relationship. In each of these figures, the triangles represent formulation A batch AA, the diamonds represent formulation A batch BB, and the dashed line represents the regression.
Figure 16A Correlation between C_{avg} & C(6); Figure 16B shows the Contingency Table Overlaid on Correlation Relationship. In each of these figures, the triangles represent formulation A batch AA, the diamonds represent formulation A batch BB, and the dashed line represents the regression.
Figure 17A Correlation between C_{avg} & C(6); Figure 17B shows the Contingency Table Overlaid on Correlation Relationship. In each of these figures, the triangles represent formulation A batch AA, the diamonds represent formulation A batch BB, and the dashed line represents the regression.
Figure 18A Correlation between C_{avg} & C(8) ; Figure 18B shows the Contingency Table Overlaid on Correlation Relationship. In each of these figures, the triangles represent formulation A batch AA, the diamonds represent formulation A batch BB, and the dashed line represents the regression.
Figure 19A shows Correlation between C_{avg} & C(12) ; Figure 19B shows the Contingency Table Overlaid on Correlation Relationship. In each of these figures, the triangles represent formulation A batch AA, the diamonds represent formulation A batch BB, and the dashed line represents the regression.
Figure 20 depicts a steady-state pharmacokinetic profile of the serum concentration of testosterone upon ingestion of a formulation of TP, which maximizes diurnal variation while producing an early Tₘₐₓ, preferably compatible with early morning, once-daily dosing
Figure 21 depicts a steady-state pharmacokinetic profile of the serum concentration of testosterone upon ingestion of a formulation of TP which maximizes diurnal variation while producing a late Tₘₐₓ, preferably compatible with night-time, once-daily dosing.
Figure 22 depicts a steady-state pharmacokinetic profile of the serum concentration of testosterone upon ingestion of a formulation of TP which provides physiological diurnal variation and an early Tₘₐₓ, preferably compatible with early morning, once-daily dosing.
Figure 23 depicts a steady-state pharmacokinetic profile of the serum concentration of testosterone upon ingestion of a formulation of TP, which provides physiological diurnal variation and a delayed Tₘₐₓ, preferably compatible with early morning, once-daily dosing.
Figure 24 depicts a steady-state pharmacokinetic profile of the serum concentration of testosterone upon ingestion of a formulation of TP, which provides a short elimination half-life and an early Tₘₐₓ, preferably compatible with maximal patient activity soon after waking and twice-daily dosing.
Figure 25 depicts a steady-state pharmacokinetic profile of the serum concentration of testosterone upon ingestion of a formulation of TP, which provides a relatively short elimination half-life and a delayed Tₘₐₓ with maximal activity about waking time. One of the twice-daily doses is preferably scheduled before bedtime.
Figure 26 depicts a steady-state pharmacokinetic profile of the serum concentration of testosterone upon ingestion of a formulation of TP, which provides and intermediate elimination half-life and a Tₘₐₓ preferably compatible with maximal activity soon after walking while reducing the extent of fluctuation to the physiological level with twice-daily dosing.
Figure 27 depicts a steady-state pharmacokinetic profile of the serum concentration of testosterone upon ingestion of a formulation of TP, which provides a longer elimination half-life and a delayed Tₘₐₓ, preferably compatible with maximal activity about awakening time following bedtime administration. This formulation reduces the extent of fluctuation to the physiological levels of testosterone with twice-daily dosing.
Figure 28 shows dissolution curves of TP from three formulations (9, 23 and 24 the compositions of which are listed in Table 2) in a phosphate buffered dissolution medium incorporating TritonX-100 as a surfactant in accordance with the present invention.
Figure 29 shows dissolution curves of TP from three formulations (47, 50, 51 and 54 the compositions of which are listed in Table 3) in a phosphate buffered dissolution medium incorporating Triton X-100 as a surfactant in accordance with the present invention.
Figure 30 provides the mean steady-state profile of treatment with three regimens for seven days.
Figure 31 shows the mean steady-state serum T and DHT Levels after seven days of BID administration of formulation 54.
Figure 32 provides a simulated mean steady-state profile of formulation 50 with respect to the observed profile for formulation 54 (both administered BID for seven days).
Figure 33 shows representative in vitro dissolution profiles for various TP formulations in phosphate buffer (PBS).

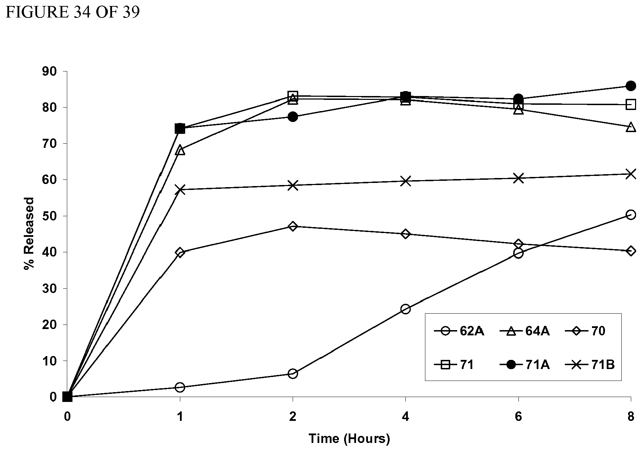

Figure 34 shows representative in vitro dissolution profiles for various TP formulations in fed-state simulated intestinal fluid (FeSSIF).
Figure 35 provides serum T levels over a 24 hour period of once or twice daily oral dosing of a TU formulation of the invention.
Figure 36 shows a serum T response over time in hypogonadal men upon administration of a formulation of the invention vs. a conventional oral TU formulation comprising TU in oleic acid (Restandol).
Figure 37 provides Tₘₐₓ values of serum T levels in subjects having consumed meals of varying fat content (as a percentage by weight) prior to oral administration of a TU formulation of the invention.
Figure 38 provides Cₘₐₓ values of serum T levels in subjects having consumed meals of varying fat content (as a percentage by weight) prior to oral administration of a TU formulation of the invention.
Figure 39 provides area under the curve (AUC) values of serum T levels in subjects having consumed meals of varying fat content (as a percentage by weight) prior to oral administration of a TU formulation of the invention

### DETAILED DESCRIPTION OF THE INVENTION

### Abbreviations and Definitions

To facilitate understanding of the invention, a number of terms and abbreviations as used herein are defined below as follows:

When introducing elements of the present invention or the particular embodiment(s) thereof, the articles "a", "an", "the" and "said" are intended to mean that there are one or more of the elements. The terms "comprising", "including" and "having" are intended to be inclusive and mean that there may be additional elements other than the listed elements.

The term "and/or" when used in a list of two or more items, means that any one of the listed items can be employed by itself or in combination with any one or more of the listed items. For example, the expression "A and/or B" is intended to mean either or both of A and B, i.e. A alone, B alone or A and B in combination. The expression "A, B and/or C" is intended to mean A alone, B alone, C alone, A and B in combination, A and C in combination, B and C in combination or A, B, and C in combination.

The term "about," as used herein, is intended to qualify the numerical values that it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

### Methods

Certain embodiments as disclosed herein provide methods of treating testosterone deficiency or its symptoms and, in particular, optimize the serum testosterone concentration during chronic treatment.

The present invention provides methods of administering oral pharmaceutical formulations comprising testosterone esters that provide average steady state serum levels (concentrations) of testosterone, which fall within a desired "normal" or eugonadalrange (i.e., about 300-1100 ng/dL) while avoiding the high Cₘₐₓ values that are considered by the United States Food and Drug Administration to be undesirable as summarized in Table 1.

**Table 1.Exposure Categories, and Proposed Limits, for T Replacement**

| Concentration Range | Percent of Population |
|---|---|
| C_{avg}< 300 ng/dL | < 25%* |
| 300 ng/dL ≤ C_{avg} ≤ 1000 ng/dL | ≥ 75% |
| C_{avg}> 1000 ng/dL | < 25%* |
| | |
| Cₘₐₓ ≤ 1500 ng/dL | ≥ 85% |
| Cₘₐₓ> 1500 ng/dL | < 15% |
| Cₘₐₓ> 1800 ng/dL | <5% |
| Cₘₐₓ> 2500 ng/dL | 0% |

| | |
|---|---|
| * The patients whose C_{avg} does not fall within the normal range for T can have C_{avg} values either above or below the normal range, but the sum of both populations should not exceed 25%. | |

For instance, FDA approval guidelines state that less than 85% of treated subjects may have a Cₘₐₓ value of 1500 ng/dL or greater, and that none may have a Cₘₐₓ value exceeding 2500 ng/dL. Less than 5% of treated subjects may have a Cₘₐₓ value falling in the range of 1800-2500 ng/dL.

Modeling studies suggest that 200 mg BID dosing of T (as a testosterone ester) is likely to have a high success rate in terms of C_{avg} being in the normal range, and Cₘₐₓ concentrations not being excessively high, at least after dose titration, and that over-responders, and most of the under-responders can have their serum T C_{avg} concentration brought into the normal range without exceeding the Cₘₐₓ limitations noted in the guidelines

Thus, in various embodiments, the present invention provides a method of treating chronic testosterone deficiency or it symptoms comprising the steps of:
a. administering to a subject in need thereof an initial amount of oral pharmaceutical composition comprising a testosterone ester solubilized in a carrier comprising at least one lipophilic surfactant and at least one hydrophilic surfactant;
b. measuring the serum testosterone concentration in the subject; and
c. administering an increased amount of the oral pharmaceutical composition to the subject when the serum testosterone concentration in the subject is less than 250 ng/dL, and administering a decreased amount of the oral pharmaceutical composition to the subject when the serum testosterone concentration in the subject is greater than 700 ng/dL

The administered oral pharmaceutical compositions comprise a hydrophobic testosterone ester dissolved in a lipophilic surfactant and a hydrophilic surfactant. A lipophilic surfactant as defined herein has a hydrophilic-lipophilic balance (HLB) less than 10, and preferably less than 5. A hydrophilic surfactant as defined herein has an HLB of greater than 10. (HLB is an empirical expression for the relationship of the hydrophilic and hydrophobic groups of a surface-active amphiphilic molecule, such as a surfactant). It is used to index surfactants and its value varies from about 1 to about 45. The higher the HLB, the more water-soluble the surfactant. The compositions are designed to be self-emulsifying drug delivery systems (SEDDS) and iterations thereof such as self-microemulsified drug delivery systems (SMEDDS) and self-nanoemulsified drug delivery systems (SNEDDS) so that a testosterone ester-containing emulsion, microemulsion, nanoemulsion (or dispersion) is formed upon mixing with intestinal fluids in the gastrointestinal tract.

In various embodiments, the testosterone ester is a short-chain (C₂-C₆) or a medium-chain (C₇-C₁₃) fatty acid ester located on the C-17 of the testosterone molecule. In certain embodiments, the testosterone ester is testosterone cypionate, testosterone octanoate, testosterone enanthate, testosterone decanoate, or testosterone undecanoate. In particular embodiments, the testosterone ester is testosterone undecanoate. For calculation purposes, 1 mg of T is equivalent to: 1.39 mg T-enanthate; 1.58 mg T-undecanoate; 1.43 mg T-cypionate, and 1.83 mg T-palmitate.

In various embodiments, the lipophilic surfactant exhibits an HLB of less than 10, preferably less than 5, and more preferably, the lipophilic surfactant exhibits an HLB of 1 to 2. Certain lipophilic surfactants suitable in oral compositions of the present invention include fatty acids (C₆-C₂₄, preferably C₁₀-C₂₄, more preferably C₁₄-C₂₄), for example, octanoic acid, decanoic acid, undecanoic acid, lauric acid, myristic acid, palmitic acid, palmitoleic, stearic acid, oleic acid, linoleic acid, alpha- and gamma- linolenic acid, arachidonic acid or combinations thereof. In a particular embodiment, the lipophilic surfactant is oleic acid.

Other suitable lipophilic surfactants include:
- Mono- and/or di-glycerides of fatty acids, such as Imwitor 988 (glyceryl mono-/di-caprylate), Imwitor 742 (glyceryl mono-di-caprylate/caprate), Imwitor 308 (glyceryl mono-caprylate), Imwitor 191 (glyceryl monostearate), Softigen 701 (glyceryl mono-/di-ricinoleate), Capmul MCM (glyceryl caprylate/caprate), Capmul MCM(L) (liquid form of Capmul MCM), Capmul GMO (glyceryl mono-oleate), Capmul GDL (glyceryl dilaurate), Maisine (glyceryl mono-linoleate), Peceol (glyceryl mono-oleate), Myverol 18-92 (distilled monoglycerides from sunflower oil) and Myverol 18-06 (distilled monoglycerides from hydrogenated soybean oil), Precirol ATO 5 (glyceryl palmitostearate) and Gelucire 39/01 (semisynthetic glycerides, i.e., C12-18 mono-, di- and tri-glycerides);
- Acetic, succinic, lactic, citric and/or tartaric esters of mono- and/or di-glycerides of fatty acids, for example, Myvacet 9-45 (distilled acetylated monoglycerides), Miglyol 829 (caprylic/capric diglyceryl succinate), Myverol SMG (mono/di-succinylated monoglycerides), Imwitor 370 (glyceryl stearate citrate), Imwitor 375 (glyceryl monostearate/citrate/lactate) and Crodatem T22 (diacetyl tartaric esters of monoglycerides);
- Propylene glycol mono- and/or di-esters of fatty acids, for example, Lauroglycol (propylene glycol monolaurate), Mirpyl (propylene glycol monomyristate), Captex 200 (propylene glycol dicaprylate/dicaprate), Miglyol 840 (propylene glycol dicaprylate/dicaprate) and Neobee M-20 (propylene glycol dicaprylate/dicaprate);
- Polyglycerol esters of fatty acids such as Plurol oleique (polyglyceryl oleate), Caprol ET (polyglyceryl mixed fatty acids) and Drewpol 10.10.10 (polyglyceryl oleate);
- Castor oil ethoxylates of low ethoxylate content (HLB<10) such as Etocas 5 (5 moles of ethylene oxide reacted with 1 mole of castor oil) and Sandoxylate 5 (5 moles of ethylene oxide reacted with 1 mole of castor oil;
- Acid and ester ethoxylates formed by reacting ethylene oxide with fatty acids or glycerol esters of fatty acids (HLB<10) such as Crodet 04 (polyoxyethylene (4) lauric acid), Cithrol 2MS (polyoxyethylene (2) stearic acid), Marlosol 183 (polyoxyethylene (3) stearic acid) and Marlowet G12DO (glyceryl 12 EO dioleate). Sorbitan esters of fatty acids, for example, Span 20 (sorbitan monolaurate), Crill 1 (sorbitan monolaurate) and Crill 4 (sorbitan mono-oleate);
- Transesterification products of natural or hydrogenated vegetable oil triglyceride and a polyalkylene polyol (HLB<10), e.g. Labrafil M1944CS (polyoxyethylated apricot kernel oil), Labrafil M2125CS (polyoxyethylated corn oil) and Gelucire 37/06 (polyoxyethylated hydrogenated coconut);
- Alcohol ethyoxylates (HLB<10), e.g. Volpo N3 (polyoxyethylated (3) oleyl ether), Brij 93 (polyoxyethylated (2) oleyl ether), Marlowet LA4 (polyoxyethylated (4) lauryl ether); and
- Pluronics, for example, Polyoxyethylene-polyoxypropylene co-polymers and block co-polymers (HLB<10) e.g. Synperonic PE L42 (HLB=8) and Synperonic PE L61 (HLB=3)

In various embodiments, the lipophilic surfactant is glyceryl monolinoleate.

In various embodiments, the hydrophilic surfactant exhibits an HLB of 10 to 45. Hydrophilic surfactants with an HLB value between 10-15 are particularly preferred. A hydrophilic surfactant component may be necessary to achieve desirable dispersability of the formulation in the GI tract and release of the drug. That is, a hydrophilic surfactant, in addition to serving as a secondary solvent, may be required to release the drug from the lipid carrier matrix, or primary solvent. The levels (amounts) of the high HLB surfactant can be adjusted to provide optimum drug release without compromising the solubilization of the active ingredient. In certain embodiments, the hydrophilic surfactant is a polyoxyethylene sorbitan fatty acid ester, hydrogenated castor oil ethoxylate, PEG mono- and diester of palmitic and stearic acid, fatty acid ethoxylate, or combinations thereof. In a particular embodiment, the hydrophilic surfactant is a hydrogenated castor oil ethoxylate. In another particular embodiment, the hydrophilic surfactant is Cremophor RH 40 (polyoxyethyleneglycerol trihydroxystearate).

In various embodiments, the oral pharmaceutical composition further includes digestible oil. A digestible oil is defined herein as an oil that is capable of undergoing de-esterification or hydrolysis in the presence of pancreatic lipase in vivo under normal physiological conditions. Specifically, digestible oils may be complete glycerol triesters of medium chain (C₇-C₁₃) or long chain (C₁₄-C₂₂) fatty acids with low molecular weight (up to C₆) mono-, di- or polyhydric alcohols. Some examples of digestible oils for use the oral pharmaceutical composition include: vegetable oils (e.g., soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond, borage, peppermint and apricot kernel oils) and animal oils (e.g., fish liver oil, shark oil and mink oil). In certain embodiments, the digestible oil is a vegetable oil. In certain embodiments, the vegetable oil is soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond oil, borage oil, peppermint oil, apricot kernel oil, or combinations thereof. Particularly preferred digestible oils are those with high gamma-linolenic acid (GLA) content such as, black currant oil, primrose oil and borage oil, as well as any other digestible oil that can be enriched in GLA acid through enzymatic processes.

In other embodiments of the present invention, methods and compositions for modulating (i.e., sustaining) the rate of available serum testosterone by incorporating component(s) that may biochemically modulate (1) testosterone ester absorption, (2) testosterone ester metabolism to testosterone, and/or (3) metabolism of testosterone to dihydrotestosterone (DHT). For example, the inclusion of medium to long chain fatty acid esters can enhance testosterone ester absorption. In this way, more testosterone ester may stave off hydrolysis in the gut and enter the blood stream. In other words, the fatty acid ester may competitively inhibit esterases that would otherwise metabolize the testosterone ester. Examples of other esters or combinations thereof include botanical extracts or benign esters used as food additives (e.g., propylparaben, octylacetate and ethylacetate).

Other components that can modulate testosterone ester absorption include "natural" and synthetic inhibitors of 5α-reductase, which is an enzyme present in enterocytes and other tissues that catalyzes the conversion of T to DHT. Complete or partial inhibition of this conversion may both increase and sustain increased serum levels of T after oral dosing with testosterone ester while concomitantly reducing serum DHT levels. Borage oil, which contains a significant amount of the 5α-reductase inhibitor, gamma-linolenic acid (GLA), is an example of a "natural" modulator of testosterone ester metabolism. Other than within borage oil, of course, GLA could be added directly as a separate component of a testosterone ester formulation of the invention. Furthermore, any digestible oil as listed above can be enzymatically enriched in GLA. Many natural inhibitors of 5α-reductase are known in the art (e.g., epigallocatechin gallate, a catechin derived primarily from green tea and saw palmetto extract from berries of the Serenoa repens species, phytosterols and lycopene), all of which may be suitable in the present invention. Non-limiting examples of synthetic 5α-reductase inhibitors suitable for use in the present invention include compounds such as finasteride, dutasteride and the like.

In various embodiments, the oral pharmaceutical composition further includes one or more additional lipid-soluble therapeutic agents. In certain embodiments, the agent is a second testosterone ester, a synthetic progestin, an inhibitor of type-I and/or type II 5α-reductase, an inhibitor of CYP3A4, finasteride, dutasteride, or combinations thereof. In a particular embodiment, the agent is borage oil. In another particular embodiment, the agent is peppermint oil and related substances such as menthol and menthol esters. In another particular embodiment, the agent is a second testosterone ester.

Optional cosolvents suitable with the oral pharmaceutical composition are, for example, water, short chain mono-, di-, and polyhydric alcohols, such as ethanol, benzyl alcohol, glycerol, propylene glycol, propylene carbonate, polyethylene glycol (PEG) with an average molecular weight of about 200 to about 10,000, diethylene glycol monoethyl ether (e.g., Transcutol HP), and combinations thereof. In particular, such cosolvents, especially monohydric alcohols, are excluded altogether. Thus, in various embodiments, the oral pharmaceutical compositions are free of monohydric alcohols. In certain embodiments, the monohydric alcohols are C₂-C₁₈ aliphatic or aromatic alcohols. In particular embodiments, the compositions are free of ethyl or benzyl alcohols.

In particular embodiments, the compositions contain between 0% and 10% (w/w) of polyethylene glycol with an average molecular weight of about 8,000 (PEG-8000). In particular embodiments, the compositions contain between 5% and 10% (w/w) of PEG-8000.

The oral pharmaceutical compositions administered in the present invention are preferably liquid or semi-solid at ambient temperatures. Furthermore, these pharmaceutical compositions can be transformed into solid dosage forms through adsorption onto solid carrier particles, such as silicon dioxide, calcium silicate or magnesium aluminometasilicate to obtain free-flowing powders that can be either filled into hard capsules or compressed into tablets. Hence, the term "solubilized" herein, should be interpreted to describe an active pharmaceutical ingredient (API), which is dissolved in a liquid solution or which is uniformly dispersed in a solid carrier. In addition, sachet type dosage forms can be formed and used. In various embodiments, the oral pharmaceutical composition is filled into a hard or soft gelatin capsule.

An embodiment of the oral pharmaceutical composition comprises:
a) 10-20 percent by weight of solubilized testosterone ester;
b) 5-20 percent by weight of hydrophilic surfactant;
c) 50-70 percent by weight of lipophilic surfactant; and
d) 10-15 percent by weight of digestible oil,
that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

In certain embodiments, the testosterone ester is a short-chain (C₂-C₆) or a medium-chain (C₇-C₁₃) fatty acid ester. In certain embodiments, the testosterone ester is testosterone cypionate, testosterone octanoate, testosterone enanthate, testosterone decanoate, or testosterone undecanoate. In a particular embodiment, the testosterone ester is testosterone undecanoate.

In some embodiments, the hydrophilic surfactant exhibits an HLB of 10 to 45, and is a polyoxyethylene sorbitan fatty acid ester, hydrogenated castor oil ethoxylate, PEG mono- and di-esters of palmitic and stearic acid, fatty acid ethoxylate, or a combination thereof. In particular, the hydrophilic surfactant is a hydrogenated castor oil ethoxylate.

In a certain embodiments, the lipophilic surfactant exhibits an HLB of less than 10, more preferably less than 5, and most preferably between 1 and 2. In certain embodiments, the lipophilic surfactant is octanoic acid, decanoic acid, undecanoic acid, lauric acid, myristic acid, palmitic acid, stearic acid, oleic acid, linoleic acid, or alpha- and gamma- linolenic acid and arachidonic acid.

In certain embodiments, the digestible oil is soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond oil, borage oil, peppermint oil, or apricot kernel oil. In some embodiments, the oral pharmaceutical composition contains one or more additional lipid-soluble therapeutic agents. In certain embodiments, these agents are synthetic progestins, inhibitors of type-I and/or type II 5α-reductase, inhibitors of CYP3A4, finasteride, dutasteride and combinations thereof. In a particular embodiment, the compositions include borage oil. In another particular embodiment, the compositions include peppermint oil.

In yet another particular embodiment, the compositions include a second testosterone ester.

In certain embodiments, the oral pharmaceutical composition exhibits a percent (%) in vitro dissolution profile 5% Triton X-100 solution in phosphate buffer, pH 6.8, and indicating release from the composition of substantially all of the solubilized testosterone ester within about 1 hour.

A specific embodiment of the oral pharmaceutical composition comprises:
a) 10-20 percent by weight of solubilized testosterone undecanoate;
b) 5-20 percent by weight hydrogenated castor oil ethoxylate;
c) 50-70 percent by weight of oleic acid; and
d) 10-15 percent by weight of digestible oil,
that is free of ethanol, and exhibits a percent (%) in vitro dissolution profile 5% Triton X-100 solution in phosphate buffer, pH 6.8, that indicates release from the composition of substantially all of the solubilized testosterone ester within about 2 hours.

Another embodiment of the oral pharmaceutical composition comprises:
a) 15-20 percent by weight of solubilized testosterone ester;
b) 5-20 percent by weight of hydrophilic surfactant;
b) 50-70 percent by weight of lipophilic surfactant; and
c) 10-15 percent by weight of digestible oil,
that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

In certain embodiments, the testosterone ester is a short-chain (C₂-C₆) or a medium-chain (C₇-C₁₃) fatty acid ester. In certain embodiments, the testosterone ester is testosterone cypionate, testosterone octanoate, testosterone enanthate, testosterone decanoate, or testosterone undecanoate. In a particular embodiment, the testosterone ester is testosterone undecanoate.

In some embodiments, the hydrophilic surfactant exhibits an HLB of 10 to 45, and is a polyoxyethylene sorbitan fatty acid ester, hydrogenated castor oil ethoxylate, PEG mono- and di-esters of palmitic and stearic acid, fatty acid ethoxylate, or a combination thereof. In particular, the hydrophilic surfactant is a hydrogenated castor oil ethoxylate.

In a certain embodiments, the lipophilic surfactant exhibits an HLB of less than 10, more preferably less than 5, and most preferably between 1 and 2. In certain embodiments, the lipophilic surfactant is octanoic acid, decanoic acid, undecanoic acid, lauric acid, myristic acid, palmitic acid, palmitoleic, stearic acid, oleic acid, linoleic acid, alpha- and gamma-linolenic acid, arachidonic acid, glyceryl monolinoleate and combinations thereof.

In certain embodiments, the digestible oil is soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond oil, borage oil, peppermint oil, or apricot kernel oil.

In some embodiments, the oral pharmaceutical composition contains one or more additional lipid-soluble therapeutic agents. In certain embodiments, these agents are synthetic progestins, inhibitors of type-I and/or type II 5α-reductase, inhibitors of CYP3A4, finasteride, dutasteride and combinations thereof. In a particular embodiment, the compositions include borage oil. In another particular embodiment, the compositions include peppermint oil.

In yet another particular embodiment, the compositions include a second testosterone ester.

In certain embodiments, the oral pharmaceutical composition exhibits a percent (%) in vitro dissolution profile in 5% Triton X-100 solution in phosphate buffer, pH 6.8, and indicating release from the composition of substantially all of the solubilized testosterone ester within about 1 hour.

A specific embodiment of the oral pharmaceutical composition comprises: a) 15-20 percent by weight of solubilized testosterone undecanoate; b) 5-20 percent by weight hydrogenated castor oil ethoxylate; b) 50-70 percent by weight of oleic acid; and c) 10-15 percent by weight of digestible oil, that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

Another embodiment of the oral pharmaceutical composition comprises:
a) 15-20 percent by weight of solubilized testosterone ester;
b) 5-20 percent by weight of hydrophilic surfactant;
c) 30-70 percent by weight of lipophilic surfactant; and
d) 10-15 percent by weight of digestible oil,
that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours. In certain embodiments, the testosterone ester is a short-chain (C₂-C₆) or a medium-chain (C₇-C₁₃) fatty acid ester.

In certain embodiments, the testosterone ester is testosterone cypionate, testosterone octanoate, testosterone enanthate, testosterone decanoate, or testosterone undecanoate. In a particular embodiment, the testosterone ester is testosterone undecanoate.

In some embodiments, the hydrophilic surfactant exhibits an HLB of 10 to 45, and is a polyoxyethylene sorbitan fatty acid ester, hydrogenated castor oil ethoxylate, PEG mono- and di-esters of palmitic and stearic acid, fatty acid ethoxylate, or a combination thereof. In particular, the hydrophilic surfactant is a hydrogenated castor oil ethoxylate.

In a certain embodiments, the lipophilic surfactant exhibits an HLB of less than 10, more preferably less than 5, and most preferably between 1 and 2. In certain embodiments, the lipophilic surfactant is octanoic acid, decanoic acid, undecanoic acid, lauric acid, myristic acid, palmitic acid, stearic acid, oleic acid, linoleic acid, or linolenic acid.

In certain embodiments, the digestible oil is soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond oil, borage oil, peppermint oil, or apricot kernel oil.

In some embodiments, the oral pharmaceutical composition contains one or more additional lipid-soluble therapeutic agents. In certain embodiments, these agents are synthetic progestins, inhibitors of type-I and/or type II 5α-reductase, inhibitors of CYP3A4, finasteride, dutasteride and combinations thereof. In a particular embodiment, the compositions include borage oil. In another particular embodiment, the compositions include peppermint oil.

In yet another particular embodiment, the compositions include a second testosterone ester.

In certain embodiments, the oral pharmaceutical composition exhibits a percent (%) in vitro dissolution profile in 5% Triton X-100 solution in phosphate buffer, pH 6.8, and indicating release from the composition of substantially all of the solubilized testosterone ester within about 1 hour.

A specific embodiment of the oral pharmaceutical composition comprises:
a) 15-20 percent by weight of solubilized testosterone undecanoate;
b) 5-20 percent by weight hydrogenated castor oil ethoxylate;
c) 30-70 percent by weight of oleic acid; and
d) 10-15 percent by weight of digestible oil, that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

Another embodiment of the oral pharmaceutical composition comprises:
a) 15-20 percent by weight of solubilized testosterone ester;
b) 5-20 percent by weight of hydrophilic surfactant;
c) >50 percent by weight of lipophilic surfactant; and
d) 10-15 percent by weight of digestible oil, that is free of ethanol,
and exhibits a percent (%) in vitro dissolution profile in 5% Triton X-100 solution in phosphate buffer, pH 6.8, that indicates release from the composition of substantially all of the solubilized testosterone ester within about 2 hours.

In certain embodiments, the testosterone ester is a short-chain (C₂-C₆) or a medium-chain (C₇-C₁₃) fatty acid ester. In certain embodiments, the testosterone ester is testosterone cypionate, testosterone octanoate, testosterone enanthate, testosterone decanoate, or testosterone undecanoate. In a particular embodiment, the testosterone ester is testosterone undecanoate.

In some embodiments, the hydrophilic surfactant exhibits an HLB of 10 to 45, and is a polyoxyethylene sorbitan fatty acid ester, hydrogenated castor oil ethoxylate, PEG mono- and di-esters of palmitic and stearic acid, fatty acid ethoxylate, or a combination thereof. In particular, the hydrophilic surfactant is a hydrogenated castor oil ethoxylate.

In a certain embodiments, the lipophilic surfactant exhibits an HLB of less than 10, more preferably less than 5, and most preferably between 1 and 2. In certain embodiments, the lipophilic surfactant is octanoic acid, decanoic acid, undecanoic acid, lauric acid, myristic acid, palmitic acid, stearic acid, oleic acid, linoleic acid, or linolenic acid.

In certain embodiments, the digestible oil is soybean oil, safflower seed oil, corn oil, olive oil, castor oil, cottonseed oil, arachis oil, sunflower seed oil, coconut oil, palm oil, rapeseed oil, evening primrose oil, grape seed oil, wheat germ oil, sesame oil, avocado oil, almond oil, borage oil, peppermint oil, or apricot kernel oil.

In some embodiments, the oral pharmaceutical composition contains one or more additional lipid-soluble therapeutic agents. In certain embodiments, these agents are synthetic progestins, inhibitors of type-I and/or type II 5α-reductase, inhibitors of CYP3A4, finasteride, dutasteride and combinations thereof. In a particular embodiment, the compositions include borage oil. In another particular embodiment, the compositions include peppermint oil.

In yet another particular embodiment, the compositions include a second testosterone ester.

In certain embodiments, the oral pharmaceutical composition exhibits a percent (%) in vitro dissolution profile in 5% Triton X-100 solution in phosphate buffer, pH 6.8, and indicating release from the composition of substantially all of the solubilized testosterone ester within about 1 hour.

A specific embodiment of the oral pharmaceutical composition comprises:
a) 15-20 percent by weight of solubilized testosterone undecanoate;
b) 5-20 percent by weight hydrogenated castor oil ethoxylate;
c) 30-70 percent by weight of oleic acid; and
d) 10-15 percent by weight of digestible oil,
that is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

In various embodiments, the oral pharmaceutical composition is administered once or twice daily. In certain embodiments, the serum testosterone concentration is measured three to five hours after administering the oral pharmaceutical composition. In certain embodiments, the serum testosterone concentration is measured after fourteen days of daily treatment with the oral pharmaceutical composition.

In various embodiments, the serum testosterone concentration is measured via radioimmunoassay, immunometric assays, or liquid chromatography tandem mass spectrometry (LC-MS/MS) assays. In particular, the serum testosterone concentration is measured via a liquid chromatography tandem mass spectrometry (LC-MS/MS) assay.

In various embodiments, the amount of the oral pharmaceutical composition administered is increased by the equivalent of about 50 mg of testosterone when the serum testosterone concentration in the subject is less than 250 ng/dL, and decreased by the equivalent of about 50 mg of testosterone when the serum testosterone concentration in the subject is greater than 700 ng/dL. In certain embodiments, the steps a.- c. are repeated until the serum testosterone concentration in the subject is between 250 and 700 ng/dL.

In a particular embodiment, the present invention provides a method of treating chronic testosterone deficiency or it symptoms comprising the steps of:
a) administering daily to a subject in need thereof an oral pharmaceutical composition comprising 475 mg of testosterone undecanoate solubilized in a carrier comprising oleic acid, polyoxyethyelene (40) hydrogenated castor oil, borage seed oil, and peppermint oil, for a period of at least fourteen days;
b) measuring the serum testosterone concentration in the subject three to five hours following the daily administration of the oral pharmaceutical composition;
c) increasing the amount of testosterone undecanoate administered daily to the subject by 50 mg when the serum testosterone concentration in the subject is less than 250 ng/dL, and decreasing the amount of testosterone undecanoate administered daily to the subject by 50 mg when the serum testosterone concentration in the subject is greater than 700 ng/dL; and
d) repeating steps a.- c. until the serum testosterone concentration in the subject is between 250 and 700 ng/dL.

The oral pharmaceutical compositions provide optimum drug release without compromising the solubilization of the active ingredients. In various embodiments, the oral pharmaceutical composition exhibits a percent (%) in vitro dissolution profile in 5% Triton X-100 solution in phosphate buffer, pH 6.8, indicating release from the composition of substantially all of the solubilized testosterone ester within about 3 hours, preferably within about 2 hours, and more preferable, release occurs within about 1 hour.

Dietary fat content modulates serum T levels. Thus, in various embodiments, the oral pharmaceutical composition is administered with a meal that at least 20 percent of the calories are derived from fat.

In an embodiment, the initial amount of testosterone ester in the oral pharmaceutical composition is administered in one or more capsules.

In an embodiment, the initial amount of testosterone ester in the oral pharmaceutical composition is administered in two capsules.

In an embodiment, the oral pharmaceutical composition comprises:
a. 10-20 percent by weight of solubilized testosterone ester;
b. about 5-20 percent by weight of hydrophilic surfactant;
c. about 50-70 percent by weight of lipophilic surfactant; and
d. about 1-10 percent by weight of polyethylene glycol 8000,
wherein the oral pharmaceutical composition is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.

In an embodiment, said composition comprises 15-20 by weight of solubilized testosterone ester.

In an embodiment, said testosterone ester is testosterone undecanoate.

In an embodiment, said hydrophilic surfactant is a hydrogenated castor oil ethoxylate.

In an embodiment, said lipophilic surfactant is glyceryl monolinoleate.

In an embodiment, said oral pharmaceutical composition comprises:
a. 15 percent by weight of solubilized testosterone undecanoate;
b. 16 percent by weight of polyoxyethylene (40) hydrogenated castor oil;
c. 63 percent by weight of glyceryl monolinoleate; and
d. 6 percent by weight of polyethylene glycol 8000.

Provided herein is a method of treating a population of humans suffering from chronic testosterone deficiency comprising the steps of:
a. administering daily to the subject a dose of an oral pharmaceutical composition comprising a testosterone ester solubilized in a carrier comprising at least one lipophilic surfactant and at least one hydrophilic surfactant;
b. measuring the serum testosterone concentration in the subject; and
c. increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL,
wherein, after treatment, less than 25% of the population has a serum testosterone C_{avg} below 300 ng/dL, less than 25% of the population has a serum testosterone C_{avg} above 1000 ng/dL, and 75% of the population has a serum testosterone C_{avg} between 300 ng/dL and 1000 ng/dL.

Disclosed herein is a method of treating a population of humans suffering from chronic testosterone deficiency comprising the steps of:
a. administering daily to the subject a dose of an oral pharmaceutical composition comprising a testosterone ester solubilized in a carrier comprising at least one lipophilic surfactant and at least one hydrophilic surfactant;
b. measuring the serum testosterone concentration in the subject; and
c. increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL,
wherein, after treatment, less than 85% of the population has a serum testosterone Cₘₐₓ below 1500 ng/dL, less than 15% of the population has a serum testosterone Cₘₐₓ above 1500 ng/dL, less than 5% of the population has a serum testosterone Cₘₐₓ above 1800 ng/dL, and 0% of the population has a serum testosterone Cₘₐₓ above 2500 ng/dL.

After reading this description, it will become apparent to one skilled in the art how to implement the invention in various alternative embodiments and alternative applications. However, although various embodiments of the present invention will be described herein, it is understood that these embodiments are presented by way of example only, and not limitation. As such, this detailed description of various alternative embodiments should not be construed to limit the scope or breadth of the present invention as set forth in the appended claims.

Specific embodiments of the instant invention will now be described in non-limiting examples.

### Example - Titration Dosing Modeling Studies

The objective of the modeling effort was to predict the fractions of the treated patient population likely to have their serum testosterone (T) C_{avg} or Cₘₐₓ values fall within designated desired ranges if the patients were to be dosed with an oral testosterone undecanoate (TU) product according to proposed treatment regimens for a pivotal Phase III clinical trial.

### Methods

The fractions of the modeled patient population having their serum T C_{avg} and Cₘₐₓ values falling within and/or outside of pre-specified limits and categories, as predicted from the probability model, were monitored and tabulated. The categories of interest were those identified by the FDA in its proposed guidelines for safe and effective hormone replacement therapy, as summarized in Table 2.

**Table 2. Exposure Categories, and Proposed Limits, for T Replacement**

| Concentration Range | Percent of Population |
|---|---|
| C_{avg}< 300 ng/dL | < 25%* |
| 300 ng/dL ≤ C_{avg} ≤ 1000 ng/dL | ≥ 75% |
| C_{avg}> 1000 ng/dL | < 25%* |
| | |
| Cₘₐₓ ≤ 1500 ng/dL | ≥ 85% |
| Cₘₐₓ> 1500 ng/dL | < 15% |
| Cₘₐₓ> 1800 ng/dL | < 5% |
| Cₘₐₓ> 2500 ng/dL | 0% |

| | |
|---|---|
| * The patients whose C_{avg} does not fall within the normal range for T can have C_{avg} values either above or below the normal range, but the sum of both populations should not exceed 25%. | |

The probability model was based on the pharmacokinetic results obtained from the hypogonadal patient population that participated in the two multidose TU treatments in study LOT-A. As noted previously, 29 hypogonadal males participated in the study, 28 of them completed Treatment Period 1 (300 mg T, as TU, BID) and 24 of them completed Treatment Period 3 (200 mg T, as TU, BID).

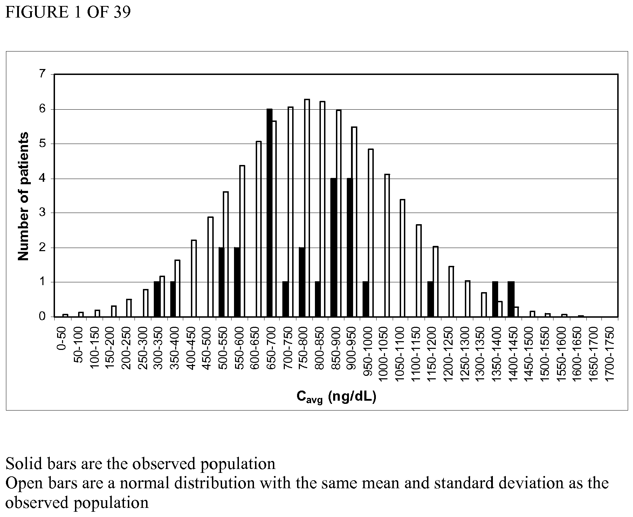

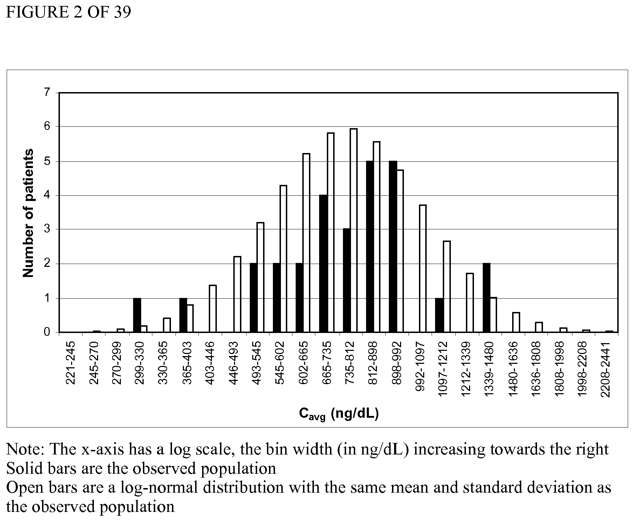

Initially, the distribution of the calculated values of C_{avg} in the treated population was characterized by a mean and standard deviation assuming that the distribution fit either a normal distribution, or a log-normal distribution. Since neither distribution resulted in an obviously superior fit to the observed data, the subsequent steps in the modeling were conducted using both alternatives (Figure 1 and Figure 2). The relationship between serum T C_{avg} and the administered dose of T was identified using the dose proportionality of the 200 mg BID and 300 mg BID treatments (Table 4).

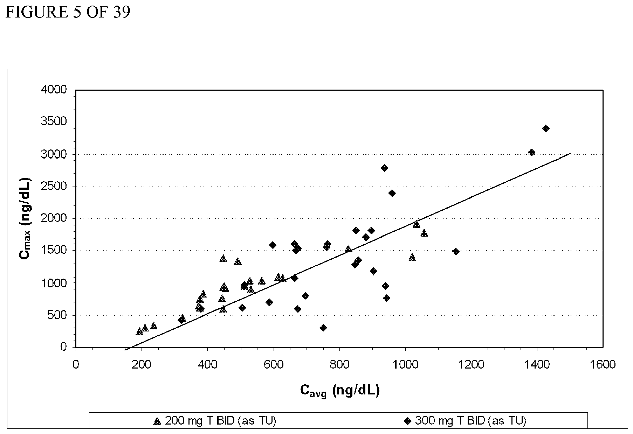

Second, a relationship was identified between serum T C_{avg} and Cₘₐₓ using linear regression, such that given an hypothesized C_{avg} value, an expected value of Cₘₐₓ could be determined (Figure 5).

Third, the variability of serum T Cₘₐₓ about its mean value (the regressed value noted above) was added to the model, creating a joint distribution function for Cₘₐₓ that tied Cₘₐₓ to the administered dose, using serum T C_{avg} as an intermediary. Cₘₐₓ variability was assumed to be normally distributed and characterized by its mean and standard deviation.

Fourth, the fraction of a treated population that fell within one of the 7 bins identified in Table 1 was calculated by:
1. Subdividing the serum T C_{avg} distribution into approximately 170 bins
2. Determining the fraction of the population within each bin
3. Summing across all bins that met each of the serum T C_{avg} criteria (Table 1) to determine the fractions of the total population in each of the three C_{avg} related categories
4. In addition, for the serum T Cₘₐₓ related items, taking each of the approximately 170 C_{avg} bins in turn and calculating, using the individualized Cₘₐₓ distributions, the fractions of that slice that met each of the four criteria related to Cₘₐₓ as noted in Table 1, above.
5. Summing across all the serum T C_{avg} bins the fractions of the population that met the serum T Cₘₐₓ criteria, thus determining the fractions of the total population that met each of the four Cₘₐₓ criteria (Table 1).

Using the above procedure the results of the probability model were explored:
1. To identify a recommended dose of TU to be used in the pivotal Phase III trial
2. To test a proposed titration scheme
3. To evaluate the robustness of the modeling procedures to the assumptions concerning:
   a. The choice of population distributions (normal vs. log normal)
   b. The regression relationship between C_{avg} and Cₘₐₓ
   c. The choice of coefficients of variation (CV) describing the population variability
   d. The impact of erroneously estimating the endogenous T levels.

### Results

### Frequency Distribution of C_{avg}

Figure 1 and Figure 2 show the observed distribution for C_{avg} values in the 24 patients that received 200 mg BID of T, as TU. Superimposed on the distribution of observed values in Figure 1 is a theoretical normal distribution profile with the same mean and standard deviation, and superimposed on the observed values in Figure 2 is a theoretical log-normal distribution with the same mean and standard deviation as the log-transformed C_{avg} values. Neither distribution produced a visually superior fit. Because the underlying distribution for the C_{avg} values was still in doubt, the remaining modeling was done twice, i.e., using each of the assumptions, and the final results were examined for sensitivity to this potentially significant assumption.

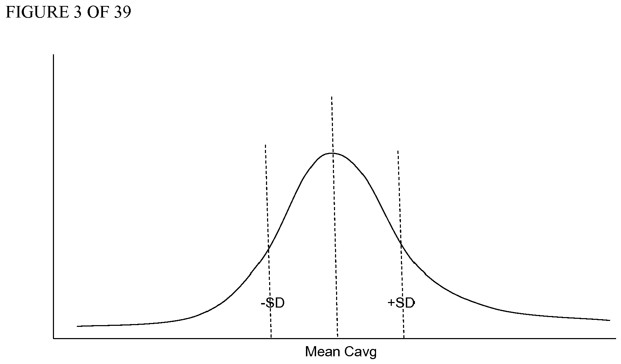

Figure 3 provides a schematic representation of the distribution of serum T C_{avg} values that would be expected if the 200 mg T BID (as TU) was administered to a very large number of subjects. Because of inherent between-patient variability, observed values of C_{avg} in the individual patients would be expected to be distributed over a wide range, even if the dose was accurately and consistently administered. The mean and standard deviation (or CV = SD/Mean) can be used to characterize this distribution.

### Linearity (Time Invariance and Dose Proportionality)

As noted elsewhere, the AM and PM doses of TU were observed to produce similar serum T profiles, have similar Cₘₐₓ values, AUCs and C_{avg} values (Table 2). Consequently, the probability modeling was conducted using the means and standard deviations obtained following the AM doses. This choice is also likely to reflect conditions of actual clinical use since monitoring of T levels in a patient would most likely occur during daylight hours, rather than overnight, or for an entire 24-hour period.

**Table 3. AM dose and PM Doses of TU Showed Similar T Pharmacokinetics**

| | AM | | PM | |
|---|---|---|---|---|
| | 300 mg BID | | 300 mg BID | |
| Cₘₐₓ (ng/mL) | 1410 | ± 771 | 1441 | ± 627 |
| Tₘₐₓ (hr) | 4.5 | ± 2.1 | 17.9 | ± 2.6 |
| Cₘᵢₙ (ng/mL) | 305 | ± 161 | 324 | ± 191 |
| AUC₍₀₋₁₂₎ (ng•hr/mL) | 9179 | ± 3990 | 9830 | ± 3489 |
| C_{avg} (ng/mL) | 765 | ± 332 | 819 | ± 291 |

The 200 mg BID and 300 mg BID doses with TU showed dose-proportionality for T concentrations after correcting for the baseline T concentrations associated with endogenous T production. The mean values for both serum T C_{avg} and Cₘₐₓ at both doses are summarized in Table 3. The ratios of the means were nearly identical to what was expected based on the theoretical difference in doses. Demonstrating dose-proportionality in T response to TU dosing simplified the modeling of alternative dosing regimens with TU, because adjustment to the desired dose could be done simply by direct scaling of the baseline-corrected mean value for the population. The variability about the mean was adjusted by assuming a constant coefficient of variation (i.e., the standard deviation varying in direct proportion to the mean).

**Table 4. Dose-Proportionality in Baseline-Corrected C_{avg} and Cₘₐₓ**

| | 200 mg BID | 300 mg BID | Ratio of means (Theoretical = 1.50) |
|---|---|---|---|
| C_{avg} | 379 ± 255 | 586 ± 330 | 1.55 |
| Cₘₐₓ | 1204 ± 815 | 816 ± 436 | 1.48 |

### Baseline T Concentrations

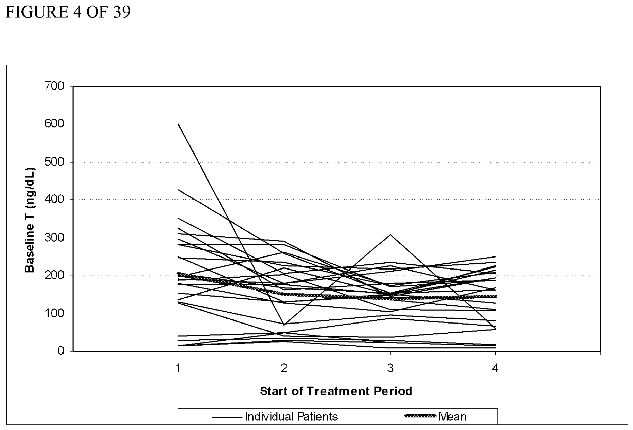

Baseline concentrations of T were determined prior to the start of the study and immediately prior to the start of each treatment cycle (i.e., after each 7 to 14-day washout period). The washout periods were sufficiently long to assure that T concentrations from the previous dosing cycle were no longer detectable. However, it was discovered that the washout periods were apparently not sufficiently long to assure that endogenous T production had recovered from suppression associated with the administration of exogenous T. Baseline T concentrations progressively decreased with each additional dosing period of the study, as shown in Figure 4. Mean baseline concentrations associated with endogenous T production were greatest pre-study (immediately prior to Treatment Period 1) at 206 ng/dL, decreasing progressively with Treatment Periods 2 and 3 (152 ng/dL and 139 ng/dL, respectively), and then remaining nearly unchanged for the start of Treatment Period 4 (145 ng/dL).

While the mean baseline T concentrations progressively decreased towards an asymptotic value, baseline concentrations for the individual patients mostly followed one of two patterns. Patients with pre-study baseline concentrations greater than 100 ng/dL showed the progressive decreases in concentration as just described for the population mean, whereas patients with pre-study concentrations less than 100 ng/dL showed little, if any, additional suppressive effect from the administration of the exogenous testosterone. This result suggests that continuous T treatment may progressively suppress endogenous T concentrations to some asymptotic level (~100 ng/dL) over the initial 1 to 4 weeks. The probability model incorporated this suppression phenomenon by assuming that endogenous T concentrations were at least as low as the lowest baseline T observed in the LOT-A study.

### Relationship between C_{avg} and Cₘₐₓ

Figure 5 displays the observed relationship between serum T C_{avg} and Cₘₐₓ on Day 7 of TU treatment in study LOT-A. The data from Treatment Period 1 (300 mg T as TU, BID) and Treatment Period 3 (200 mg T as TU, BID) are plotted with different symbols, but the relationship is continuous over the combined range associated with the two doses. The pooled dataset showed a high degree of correlation (R² = 0.6934), indicating that approximately 70% of the variability in the observed Cₘₐₓ values was accounted for by the variation in C_{avg}.

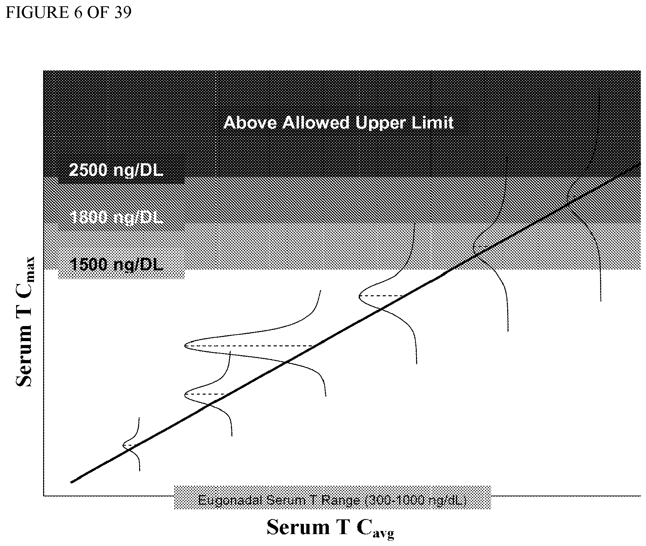

Figure 6 provides a schematic view of the interplay of C_{avg}, Cₘₐₓ and the variability in Cₘₐₓ. In a population of patients treated with the same dose of TU, the distribution of C_{avg} values is uniform along the x-axis, but has a normal or log-normal distribution (e.g., Figure 3) that can be characterized by a mean and standard deviation. Since approximately 2/3 of the patients are expected to have serum T C_{avg} values within one standard deviation of the mean, 2/3 of the resulting Cₘₐₓ values will be in distributed on both sides of the regression line in Figure 6 and mostly in the region within one standard deviation of the mean C_{avg} for the dose of TU. Usually the Cₘₐₓ values will lie in close proximity to the regression line, but some can be expected to be significantly above or below the regression line. Some of the Cₘₐₓ values will fall within the concentration ranges denoted by the orange and red regions in Figure 6, with the probability of doing so being greater in patients with higher C_{avg} values.

At the lower extreme of the regression line the total number of patients in the distribution about the regression line should be a small fraction of the total population, and the variation about the mean Cₘₐₓ in that region relatively limited, e.g., a value of Cₘₐₓ of 250 ng/dL would have a standard deviation of approximately 50 ng/dL, compared to a mean Cₘₐₓ of 1000 ng/dL having a standard deviation of 200 ng/dL. Similarly, the number of patients with Cₘₐₓ values near the upper extreme of the regression line should also be a small fraction of the total population, but, the variability in Cₘₐₓ values would be anticipated to be quite wide, e.g., patients with mean Cₘₐₓ value of 2000 ng/dL might have a standard deviation of 400 ng/dL. This variation in population density is captured in Figure 6 by the higher peak values and greater AUC under the Cₘₐₓ distribution curves near the midpoint of the figure and the lower values at the upper and lower extremes. The increased variation in Cₘₐₓ variability as C_{avg} increases is portrayed by the progressive broadening of the distributions as concentrations increase.

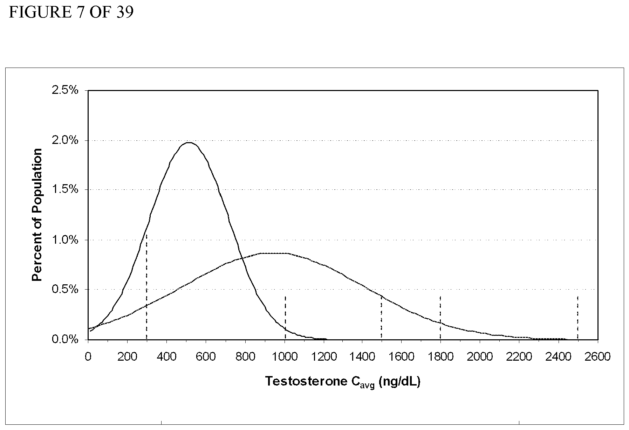

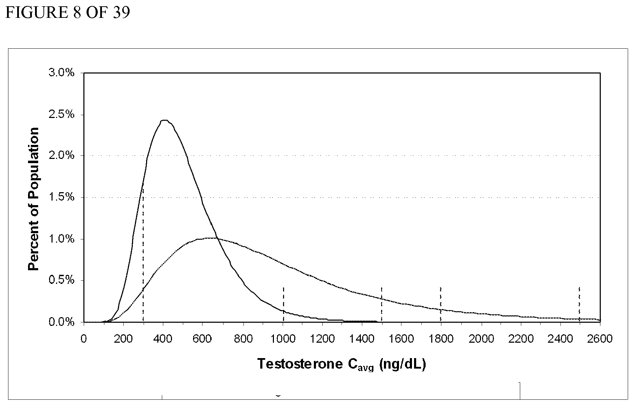

An objective of the modeling process was to determine what fractions of the population would be predicted to have serum T Cₘₐₓ values in the concentration ranges symbolized by the three bands of orange through red colors in Figure 6. The lower edges of these three bands represent the 1500 ng/dL, 1800 ng/dL and 2500 ng/dL cutoff values noted in Table 1. The fractions of the population predicted to have Cₘₐₓ values in each of these bands was calculated by dividing the C_{avg} distribution into a series of 10 ng/dL wide bins, and then determining what fractions of the population within that bin had Cₘₐₓ values inside and outside the various acceptance limits noted in Table 1. The fractions of the patient population in each of these small bins were then summed across the entire range of serum T C_{avg} values, to give the fractions of the total population meeting each of the criteria noted in Table 1. Figure 7 and Figure 8 provide alternative views of these summations - Figure 7 applies if the underlying distribution happens to be normal, and Figure 8 if the underlying distribution happens to be log-normal. In each of the figures, the demarcation concentrations for the limits of various C_{avg} and Cₘₐₓ regions are represented by vertical dashed lines. Tables in the discussion that follow summarize changes in the fraction of the populations predicted to be in the various regions as selected dosing adjustments are modeled or selected assumptions about the distributions are altered.

### Modeling of Proposed Dose Titration Scheme For Phase III

Five parameters, as summarized in Table 4were sufficient to characterize the designated serum T C_{avg} and Cₘₐₓ distributions for T following a dose of 200 mg T BID as TU. The values of two of the parameters, the mean C_{avg} and its CV, depended on whether the frequency distribution for C_{avg} was assumed normal or log-normal.

**Table 4. Nominal Parameters Values for Probability Modeling**

| Parameter | Parameter Value |
|---|---|
| Mean C_{avg} (CV) | 520 (39%) |
| Mean Ln(C_{avg}) (CV) | Ln(473) (6%) |
| Cₘₐₓ (CV) | 2.2 x C_{avg} (21%) |
| Baseline T | 120 ng/dL |

A preliminary review of the data indicated that no patients in study LOT-AA with C_{avg} concentrations of 900 ng/dL or less were observed to have Cₘₐₓ concentrations greater than 2000 ng/dL (Figure 5). Therefore, it was concluded that keeping C_{avg} concentrations at 800 ng/dL or lower would likely result in negligible risk of any patients having Cₘₐₓ values >2500 ng/dL. A titration scheme was developed, as outlined in Table 5, incorporating this titration feature, as well as a mechanism for increasing the TU dose in patients whose T concentrations did not increase sufficiently for a patient's T concentration to get into the normal range at the standard dose (i.e., serum T C_{avg}< 300 ng/dL).

**Table 52. Titration Scheme to Reduce Incidence of Under-Responders and Over-Responders**

| Category | Definition | Dose Adjustment |
|---|---|---|
| Initial Dose | 200 mg T BID (as TU) | (Starting Dose) |
| Under- | C_{avg}< 300 ng/dL | Increase Dose to 300 mg |

| Responder | | BID |
|---|---|---|
| Over Responder | C_{avg}>800 ng/dL | Decrease Dose to 100 mg BID |

Table 6 summarizes the results generated by the probability model when all patients in the distribution were assumed to be receive a standard 200 mg BID dose of T (as TU) (the "Before Titration" column), and the results after patients at the low end of the distribution (Under-Responders) had their T doses increased to 300 mg BID, and patients at the high end of the distribution (Over-Responders) had their T dose decreased to 100 mg BID (the "After Titration" column). Results are provided in the table for both the normal and log-normal based models. The modeling predicts that the minimum efficacy goal (75% of patients with C_{avg} values in the normal range) will be easily met even before a titration decision is made (85% success if normally distributed, 87% success if log-normally distributed), and the predicted success rates will climb another 8% - 12% if the titration step is implemented (to 93% and 97% for normal and log-normal, respectively). However, the models also predict that simply treating all patients with 200 mg BID is likely to result in more than 15% of patients having Cₘₐₓ values greater than 1500 ng/dL. The predicted over-response rate is slightly higher assuming a normal distribution than assuming a log-normal distribution (22% vs. 18%). However, after inclusion of the titration step, the predicted incidence rate for over-responders is reduced to approximately the targeted maximum 15% rate indicated in the guidelines (17% for normal, 12% for log-normal). The predicted rates for extreme over-responders (Cₘₐₓ>2500 ng/dL) is less than 2% at the 200 mg BID dose, but is reduced even further to a predicted rate of 0.1% or less by titrating the over-responders to a 100 mg BID dose.

**Table 6. Predicted Frequency Rates for Selected Population Segments when Dosed at 200 mg BID, before and after a Titration for Under-Responders and Over-Responders**

| Assuming a Normal Distribution | | |
|---|---|---|
| | Before Titration | After Titration |
| | (200 mg T, as TU, BID) | (100, 200 or 300 mg T, as TU, BID) |
| C_{avg} Regions | | |
| C_{avg}< 300 | 14% | 7% |
| 300 ≤ C_{avg} ≤ 1000 | 85% | 93% |
| C_{avg}>1000 | 0.78% | 0.0000% |
| Cₘₐₓ Categories | | |
| Cₘₐₓ ≤ 1500 | 78% | 83% |
| Cₘₐₓ>1500 | 22% | 17% |
| Cₘₐₓ> 1800 | 10% | 5.9% |
| Cₘₐₓ>2500 | 1.0% | 0.11% |
| | | |
| Assuming a Log-Normal Distribution | | |
| C_{avg} Regions | | |
| C_{avg}< 300 | 11% | 1% |
| 300 ≤ C_{avg} ≤ 1000 | 87% | 99% |
| C_{avg}>1000 | 2.0% | 0.0005% |
| Cₘₐₓ Categories | | |
| Cₘₐₓ ≤ 1500 | 82% | 88% |
| Cₘₐₓ>1500 | 18% | 12% |
| Cₘₐₓ> 1800 | 9% | 3.9% |
| Cₘₐₓ>2500 | 1.6% | 0.07% |

| | | |
|---|---|---|
| Predictions are based on observed CVs for C_{avg} and Cₘₐₓ following 200 mg BID dosing (Table 4) Titration to 300 mg if C_{avg}<300 ng/mL, titration to 100 mg if C_{avg}>800 ng/mL | | |

The results summarized in Table 6 demonstrate the interesting finding that assuming a log-normal distribution provides more optimistic results, both before and after titration, than does assuming a simple normal distribution. The predicted efficacy success rates are higher, and the predicted failure rates based on the safety surrogate are lower when the log-normal distribution is assumed. In addition, a greater beneficial impact of the titration step is predicted with the log-normal distribution.

### Robustness of Model to Parameter Choices

The preceding results from the probability model indicate that a proposed initial standard dose of 200 mg BID T (as TU) in a Phase III setting is predicted to meet the criteria for adequate efficacy, whether or not the over-responders and under-responders subsequently have their T doses adjusted. However, the predicted rates for the safety surrogates (i.e., serum T Cₘₐₓ categories) exceeded the target ranges unless a dosage adjustment was incorporated for over-responders and under-responders. After dosage titration, the percentage of patients predicted to have Cₘₐₓ concentrations in excess of 1500 ng/dL or 1800 ng/dL were close to maximum suggested limits proposed in the FDA guidelines.

Consequently, a sensitivity analysis was performed using the model to determine how critical were the assumptions pertaining to (1) the variability about C_{avg} and Cₘₐₓ, the CVs associated with C_{avg} and Cₘₐₓ, (2) the steepness of the C_{avg}/Cₘₐₓ relationship, (3) the standard dose of T administered, and (4) the baseline T associated with endogenous production.

Table 7 and Table 8 summarize the predicted fractions in the critical categories if the CVs describing the variability about the mean values are either decreased or increased by approximately 15-20%, and if the slope describing the relationship between C_{avg} and Cₘₐₓ is increased or decreased by approximately 20%. Table 7 summarizes the results when the modeling assumed the normal distribution, while Table 8 summarizes the results based on a log-normal distribution.

Not surprisingly, decreasing the CVs and the Cₘₐₓ/C_{avg} slope associated with the mean values increased the success rates for keeping C_{avg} within the normal range, and reduced the frequency of patients being in concentration categories used as surrogates for patient safety. Increasing the values of these operating parameters had the opposite effect. While none of the scenarios reduced the efficacy success rate outside the desired range, a population of patients with greater variability than observed in study LOT-AA, and a steeper Cₘₐₓ/C_{avg} relationship might well be left with a greater than desired number of patients with high Cₘₐₓ values, even after dosage adjustment had occurred. The impact of varying the CV and Cₘₐₓ/C_{avg} relationship was more pronounced on the fractions in the targeted Cₘₐₓ categories than on the fraction of patients in the various C_{avg} regions. As noted previously, the modeling predicted more optimistic outcomes when a log-normal distribution was assumed (Table 8) than when a normal distribution was assumed Table 7).

**Table 7. Robustness Investigation: Variation in CV and Cₘₐₓ/C_{avg} Relationship (Normal Distribution)**

| Distribution at Steady-State with Initial 200 mg T, BID, as TU Dose | | | |
|---|---|---|---|
| | Low CVs & Cₘₐₓ/C_{avg} | Mid-range CVs & Cₘₐₓ/C_{avg} | High CVs & Cₘₐₓ/C_{avg} |
| C_{avg} Regions | | | |
| C_{avg}< 300 | 10% | 14% | 17% |
| 300 ≤ C_{avg} ≤ 1000 | 90% | 85% | 81% |
| C_{avg}>1000 | 0.21% | 0.78% | 1.8% |
| Cₘₐₓ Categories | | | |
| Cₘₐₓ ≤ 1500 | 92% | 78% | 62% |
| Cₘₐₓ>1500 | 8% | 22% | 38% |
| Cₘₐₓ>1800 | 2% | 10% | 24% |
| Cₘₐₓ>2500 | 0.076% | 1.0% | 6% |
| Distribution at Steady-State after Dose Titration (100, 200 or 300 mg BID) | | | |
| C_{avg} Regions | | | |
| C_{avg}< 300 | 4% | 7% | 10% |
| 300 ≤ C_{avg} ≤ 1000 | 96% | 93% | 90% |
| C_{avg}>1000 | 0.0000% | 0.0000% | 0.0001% |
| Cₘₐₓ Categories | | | |
| Cₘₐₓ ≤ 1500 | 96% | 83% | 67% |
| Cₘₐₓ>1500 | 3.5% | 17% | 33% |
| Cₘₐₓ>1800 | 0.3% | 5.9% | 18% |
| Cₘₐₓ>2500 | 0.0000% | 0.11% | 2.1% |

| | | | |
|---|---|---|---|
| CVs for C_{avg} = 33%, 39%, 43% CVs for Cₘₐₓ = 17%, 21%, 25% Cₘₐₓ/C_{avg} = 1.8, 2.2, 2.6 | | | |

**Table 3. Robustness Investigation: Variation in CV and Cmax/Cavg Relationship (Log-Normal Distribution)**

| Distribution at Steady-State with Initial 200 mg T, BID, as TU Dose | | | |
|---|---|---|---|
| | Low CVs & Cₘₐₓ/C_{avg} | Mid-range CVs & Cₘₐₓ/C_{avg} | High CVs & Cₘₐₓ/C_{avg} |
| C_{avg} Regions | | | |
| C_{avg}< 300 | 7% | 11% | 14% |
| 300 ≤ C_{avg} ≤ 1000 | 92% | 87% | 82% |
| C_{avg}>1000 | 0.72% | 2.0% | 4% |
| Cₘₐₓ Categories | | | |
| Cₘₐₓ ≤ 1500 | 94% | 82% | 68% |
| Cₘₐₓ>15 00 | 6% | 18% | 32% |
| Cₘₐₓ>1800 | 2% | 9% | 20% |
| Cₘₐₓ>2500 | 0.18% | 1.6% | 7% |
| Distribution at Steady-State after Dose Titration (100, 200 or 300 mg BID) | | | |
| C_{avg} Regions | | | |
| C_{avg}< 300 | 0.82% | 1.4% | 4% |
| 300 ≤ C_{avg} ≤ 1000 | 99% | 99% | 96% |
| C_{avg}>1000 | 0.0009% | 0.0005% | 0.11% |
| Cₘₐₓ Categories | | | |
| Cₘₐₓ ≤ 1500 | 96% | 88% | 72% |
| Cₘₐₓ>15 00 | 4% | 12% | 28% |
| Cₘₐₓ>1800 | 0.81% | 3.9% | 14% |
| Cₘₐₓ>2500 | 0.0037% | 0.073% | 1.8% |

| | | | |
|---|---|---|---|
| CVs for Ln(C_{avg}) = 5%, 6%, 7% CVs for Cₘₐₓ = 17%, 21%, 25% Cₘₐₓ/C_{avg} = 1.8, 2.2, 2.6 | | | |

Table 9 and Table 10 summarize the predicted fractions of subjects in the critical categories if the proposed standard 200 mg BID dose of T (as TU) administered to all patients was either decreased or increased by 25 mg (12.5%). Table 9 summarizes the results when the modeling assumed the normal distribution, while Table 10 summarizes the results based on a log-normal distribution.

Not surprisingly, decreasing the dose reduced the fraction of patients appearing as over-responders, but it also increased the fraction of the population classified as under-responders. The dosage adjustments associated with including a titration step for under-responders and over-responders reduced the fraction of patients with below normal range C_{avg} concentrations by approximately 50%. For the over-responders, dosage adjustment was effective for the low dose regimen (175 mg before titration), right at the borderline for the mid-range dose (200 mg before titration), potentially insufficient for the highest dose group (225 mg before titration). The titration process made substantial corrections to the fractions of the population appearing in the undesirable categories, but the fraction of patients predicted to appear in the 1500-2500 ng/dL category for Cₘₐₓ, was still very near to, or greater, than the upper limit. As noted previously, the modeling predicted more optimistic outcomes when a log-normal distribution (Table 10) was assumed than when a normal distribution was assumed (Table 9).

**Table 9. Robustness Investigation: Effect of Dose (Normal Distribution)**

| Distribution at Steady-state with Initial Dose (Normal) | | | |
|---|---|---|---|
| | -12.5% Decrease | Proposed Dose | +12.5% Increase |
| | (175 mgT, BID as TU) | (200 mgT, BID, as TU) | (225 mg T, BID, as TU) |
| C_{avg} Regions | | | |
| C_{avg}< 300 | 18% | 14% | 11% |
| 300 ≤ C_{avg} ≤ 1000 | 82% | 85% | 86% |
| C_{avg}>1000 | 0.16% | 0.78% | 1.8% |
| Cₘₐₓ | | | |
| Categories | | | |
| Cₘₐₓ ≤ 1500 | 85% | 78% | 62% |
| Cₘₐₓ>15 00 | 15% | 22% | 38% |
| Cₘₐₓ>1800 | 6% | 10% | 24% |
| Cₘₐₓ>2500 | 0.35% | 1.0% | 6% |

| Distribution at Steady-state after Dose Titration (Normal) | | | |
|---|---|---|---|
| | -12.5% Decrease | Proposed Dose | +12.5% Increase |
| | (87.5, 175 or 262.5 mg BID) | (100, 200 or 300 mg BID) | (112.5, 225 or 337.7 mg BID) |
| C_{avg} Regions | | | |
| C_{avg}< 300 | 9% | 7% | 6% |
| 300 ≤ C_{avg} ≤ 1000 | 91% | 93% | 94% |
| C_{avg}>1 000 | 0.0000% | 0.0000% | 0.0000% |
| Cₘₐₓ | | | |
| Categories | | | |
| Cₘₐₓ ≤ 1500 | 87% | 83% | 79% |
| Cₘₐₓ>15 00 | 13% | 17% | 21% |
| Cₘₐₓ> 1800 | 4.1% | 5.9% | 7.4% |
| Cₘₐₓ>2500 | 0.068% | 0.11% | 0.15% |

| | | | |
|---|---|---|---|
| T Doses modeled (initial doses, before titration): 175 mg BID, 200 mg BID, and 225 mg BID (as TU) Titration for under-responders was to a 50% higher dose (262.5 mg, 300 mg or 337.5 mg BID) Titration for over-responders was to a 50% lower dose (87.5 mg, 100 mg or 112.5 mg BID) | | | |

**Table 10. Robustness Investigation: Effect of Dose (Log-Normal Distribution)**

| Distribution at Steady-state with Initial Dose (Log-Normal) | | | |
|---|---|---|---|
| | -12.5% Decrease | Proposed Dose | +12.5% Increase |
| | (175 mgT, BID as TU) | (200 mg T, BID, as TU) | (225 mg T, BID, as TU) |
| C_{avg} Regions | | | |
| C_{avg}< 300 | 16% | 11% | 7% |
| 300 ≤ C_{avg} ≤ 1000 | 83% | 87% | 89% |
| C_{avg}>1000 | 0.96% | 2.0% | 4% |
| Cₘₐₓ | | | |
| Categories | | | |
| Cₘₐₓ ≤ 1500 | 88% | 82% | 76% |
| Cₘₐₓ>15 00 | 12% | 18% | 24% |
| Cₘₐₓ>1800 | 5% | 9% | 13% |
| Cₘₐₓ>2500 | 0.80% | 1.6% | 3% |

| Distribution at Steady-state after Dose Titration (Log-Normal) | | | |
|---|---|---|---|
| | -12.5% Decrease | Proposed Dose | +12.5% Increase |
| | (87.5, 175 or 262.5 mg BID) | (100, 200 or 300 mg BID) | (112.5, 225 or 337.7 mg BID) |
| C_{avg} Regions | | | |
| C_{avg}< 300 | 2% | 1% | 1% |
| 300 ≤ C_{avg} ≤ 1000 | 98% | 99% | 99% |
| C_{avg}>1000 | 0.0001% | 0.0005% | 0.0023% |
| Cₘₐₓ | | | |
| Categories | | | |
| Cₘₐₓ ≤ 1500 | 91% | 88% | 85% |
| Cₘₐₓ>15 00 | 9% | 12% | 15% |
| Cₘₐₓ>1800 | 3% | 3.9% | 5% |
| Cₘₐₓ>2500 | 0.049% | 0.073% | 0.10% |

| | | | |
|---|---|---|---|
| T Doses modeled (before titration): 175 mg BID, 200 mg BID, and 225 mg BID (as TU) Titration for under-responders was to a 50% higher dose (262.5 mg, 300 mg or 337.5 mg BID) Titration for over-responders was to a 50% lower dose (87.5 mg, 100 mg or 112.5 mg BID) | | | |

Table 11 and Table 12 summarize the predicted fractions in the critical categories if the baseline T concentrations associated with production of endogenous T was either reduced (from 120 ng/dL to 75 ng/dL) or increased (from 120 ng/dL to 200 ng/dL). Table 11 summarizes the results when the modeling assumed the normal distribution, while Table 12 summarizes the results based on a log-normal distribution.

This sensitivity analysis was conducted by assuming that pre-titration results were the same for all three cases, but that the endogenous baseline concentrations might have been suppressed to a greater (75 ng/dL) or lesser extent (200 ng/dL) than the modeling originally assumed (120 ng/dL). The results in Table 11 and Table 12 suggest that the outcomes from the model are relatively insensitive to an incorrect estimation of the contribution of endogenous T to the total. Neither the fraction of patients in the various efficacy categories, nor the fractions of patients in the various categories serving as surrogates for safety were greatly altered by alternative estimates of the baseline T concentrations. The results suggest that while the effect of progressive suppression of endogenous T with continuing treatment may be observable, the ultimate impact on the rate of treatment success is minimal. As noted previously, the modeling predicted more optimistic outcomes when a log-normal distribution (Table 12) was assumed than when a normal distribution was assumed (Table 11).

**Table 4. Robustness Investigation: Effect of Endogenous Baseline T**

| (Normal Distribution) | | | |
|---|---|---|---|
| Distribution at Steady-state with Initial Dose of 200 mg T, BID, as TU (Normal) | | | |
| | Baseline T | Baseline T | Baseline T |
| | 75 ng/dL | 120 ng/dL | 200 ng/dL |
| C_{avg} Regions | | | |
| C_{avg}< 300 | 14% | 14% | 14% |
| 300 ≤ C_{avg} ≤ 1000 | 85% | 85% | 85% |
| C_{avg}>1000 | 0.78% | 0.78% | 0.78% |
| Cₘₐₓ Categories | | | |
| Cₘₐₓ ≤ 1500 | 78% | 78% | 78% |
| Cₘₐₓ>15 00 | 22% | 22% | 22% |
| Cₘₐₓ>1800 | 10% | 10% | 10% |
| Cₘₐₓ>2500 | 1.0% | 1.0% | 1.0% |

| Distribution at Steady-state after Dose Titration (100, 200 or 300 mg BID) (Normal) | | | |
|---|---|---|---|
| C_{avg} Regions | | | |
| C_{avg}< 300 | 6% | 7% | 10% |
| 300 ≤ C_{avg} ≤ 1000 | 94% | 93% | 90% |
| C_{avg}>1000 | 0.0000% | 0.0000% | 0.0001% |
| Cₘₐₓ Categories | | | |
| Cₘₐₓ ≤ 1500 | 83% | 83% | 81% |
| Cₘₐₓ>15 00 | 17% | 17% | 19% |
| Cₘₐₓ>1800 | 6% | 6% | 6% |
| Cₘₐₓ>2500 | 0.11% | 0.11% | 0.12% |

| | | | |
|---|---|---|---|
| Baseline corrected T concentrations modeled: 75 mg/dL, 120 mg/dL and 200 mg/dL Titration for under-responders was to 300 mg BID Titration for over-responders was to 100 mg BID | | | |

**Table 12. Robustness Investigation: Effect of Endogenous Baseline T**

| (Log-Normal Distribution) | | | |
|---|---|---|---|
| Distribution at Steady-state with Initial Dose of 200 mg T, BID, as TU (Log-Normal) | | | |
| | Baseline T | Baseline T | Baseline T |
| | 75 ng/dL | 120 ng/dL | 200 ng/dL |
| C_{avg} Regions | | | |
| C_{avg}< 300 | 11% | 11% | 11% |
| 300 ≤ C_{avg} ≤ 1000 | 87% | 87% | 87% |
| C_{avg}>1000 | 2.0% | 2.0% | 2.0% |
| Cₘₐₓ Categories | | | |
| Cₘₐₓ ≤ 1500 | 82% | 82% | 82% |
| Cₘₐₓ>1500 | 18% | 18% | 18% |
| Cₘₐₓ>1800 | 9% | 9% | 9% |
| Cₘₐₓ>2500 | 1.6% | 1.6% | 1.6% |
| Distribution at Steady-state after Dose Titration (100, 200 or 300 mg BID) (Log-Normal) | | | |
| C_{avg} Regions | | | |
| C_{avg}< 300 | 2% | 2% | 3% |
| 300 ≤ C_{avg} ≤ 1000 | 98% | 98% | 97% |
| C_{avg}>1000 | 0.0057% | 0.017% | 0.085% |
| Cₘₐₓ Categories | | | |
| Cₘₐₓ ≤ 1500 | 87% | 87% | 85% |
| Cₘₐₓ>15 00 | 13% | 13% | 15% |
| Cₘₐₓ>1800 | 4% | 4% | 5% |
| Cₘₐₓ>2500 | 0.082% | 0.10% | 0.16% |

| | | | |
|---|---|---|---|
| Baseline corrected T concentrations modeled: 75 mg/dL, 120 mg/dL and 200 mg/dL Titration for under-responders was to 300 mg BID Titration for over-responders was to 100 mg BID | | | |

The results of the sensitivity analysis suggest that the probability model is quite sensitive to the dose of T administered and to the slope of the relationship between Cₘₐₓ and C_{avg}. Steeper slopes than utilized in the model and higher doses than in proposed dosing scheme are predicted to result in higher than desired rates of patients with Cₘₐₓ concentrations above the thresholds of concern. The results suggest that a more aggressive dose adjustment scheme, i.e., greater than a 50% increase or decrease from the "standard" during the titration step, might provide a mechanism for addressing a steeper than anticipated relationship.

The predictions from the probability model were moderately sensitive to the assumptions about the inter-patient variability in C_{avg} and Cₘₐₓ, and to whether the distribution for C_{avg} was assumed normal or log-normal. The assumption that C_{avg} fit a log normal distribution led to more optimistic projections from the model, both in terms of the efficacy rates (C_{avg} being in the normal range), and in terms of Cₘₐₓ not exceeding designated threshold concentrations in unacceptably large fractions of the treated patients.

The predictions from the probability model were relatively insensitive to the assumed baseline T concentrations resulting from continuing, but partially suppressed, endogenous T production. Incorrect estimation of this value in individual patients appears unlikely to have a detectable impact on the fractions of patients that fall into the various designated categories after the titration step.

### Conclusions

The probability model, as constructed, proved helpful in exploring the potential impact of alternative dosing regimens and dose-adjustment algorithms.

The model results suggest that 200 mg BID dosing of T (as TU) is feasible as the initial dose in a Phase III study, is likely to have a high success rate in terms of C_{avg} being in the normal range, and Cₘₐₓ concentrations not being excessively high, at least after dose titration. The model predicts that choosing a significantly higher T dose than 200 mg BID is likely to result in Cₘₐₓ values being outside the guidelines in a higher than desired fraction of the patient population.

The model predicts that essentially all the over-responders, and most of the under-responders can have their serum T C_{avg} concentration brought into the normal range without exceeding the Cₘₐₓ limitations noted in the guidelines

The model provided similar results whether the underlying distribution between the T dose and C_{avg} was assumed normal or log-normal. The log-normal distribution generally resulted in more optimistic projections from the model.

The model predictions were sensitive to the postulated relationship between Cₘₐₓ/C_{avg} , the steeper the slope of that relationship, the more difficult it being to obtain acceptably low rates of excessive Cₘₐₓ values. The model predictions were relatively insensitive to the assumed value of the baseline T concentration (a value related to residual endogenous T production).

### Example - Assessing Optimal Sampling Time

An investigation was conducted to identify a single optimal sampling time for monitoring the responsiveness of hypogonadal male patients subjects to chronic BID dosing of T using a SEDDS formulation of TU. Concentration data and derived pharmacokinetic parameters for 41 subjects from two studies (LOT-AA and LOT-BB) where subjects received 200 mg BID of T, as TU, for at least seven days were used in this investigation. Correlation and contingency table approaches were used in the investigation.

### Methods

Concentration data and pharmacokinetic parameters from hypogonadal male subjects that participated in study LOT-AA and study LOT-BB were combined into a single data set. From study LOT-AA, only the information from Day 7 of Treatment 3 (8 days of treatment with 200 mg BID of T, as TU) was used. Study LOT-AA contributed data from 26 subjects treated for 7 days, and study LOT-BBBB contributed data from 15 subjects treated for 28 days with 200 mg BID of T, as TU. Combining of the data from these two groups of subjects is supported by the finding from study LOT-BBBB that T steady-state is reached in 5-7 days.

The sample collection times for these two studies both spanned a 12-hour window, but not all sample collection times were common to both studies. LOT-AA used sample collection times of 0, 1, 2, 4, 8 and 12 hours; LOT-BBBB used sample collection times of 0, 1.5, 3, 4, 5, 6, 8 and 12 hours. The current analysis used the superset of the combined set of collection times (0, 1, 1.5, 2, 3, 4, 5, 6, 8 and 12 hours). Concentration values that were missing for a subject in either of the data sets at a particular sample collection time were estimated by linear interpolation, using the existing nearest neighbor concentration values for that subject, i.e., interpolation of missing data was subject-specific.

For the correlation approach, linear regression of the concentrations at each of the sample collection times against C_{avg} was performed. The correlation coefficients and the regression parameters (slope and intercept) were determined and tabulated. The sample collection time with the greatest correlation coefficient was identified as having the best predictive capability for C_{avg}.

For the contingency table approach, the number and percentage of subjects that fell into each of the following seven categories were enumerated, for each of the candidate time points, and for each candidate target range for C_{avg}, and for each candidate acceptance range for C(t).
1. Subjects with C_{avg} within range and with C(t) within range
2. Subjects with C_{avg} below range and with C(t) below range
3. Subjects with C_{avg} above range and with C(t) above range
4. Subjects with C_{avg} within range but with C(t) below range
5. Subjects with C_{avg} within range but with C(t) above range
6. Subjects with C_{avg} below range, but with C(t) within range
7. Subjects with C_{avg} above range, but with C(t) within range

If one defines a value as being "within range" as positive, and being "outside of range" as negative, then Group 1 is subjects that are "true positives", Groups 2 and 3 are "true negatives", groups 4 and 5 are "false negatives", and groups 6 and 7 are "false positives". Groups, 1, 2 and 3 represent successful predictions because C(t) is an appropriate surrogate for C_{avg}; Groups 4, 5, 6 and 7 are prediction failures because C(t) is an inaccurate surrogate for C_{avg}.

Correlations and contingency table calculations were performed using built-in functions in the Excel module of Microsoft Office 2003 (Redmond, WA). Graphs were produced using the built-in charting capabilities of Excel.

### Results

The C_{avg} and Cₘₐₓ values, and T concentrations at each sample time for the 41 subjects incorporated into the investigation, both observed values and estimated values, are presented in Table 13. The C(t) values that were estimated by interpolation are identified by a shaded background

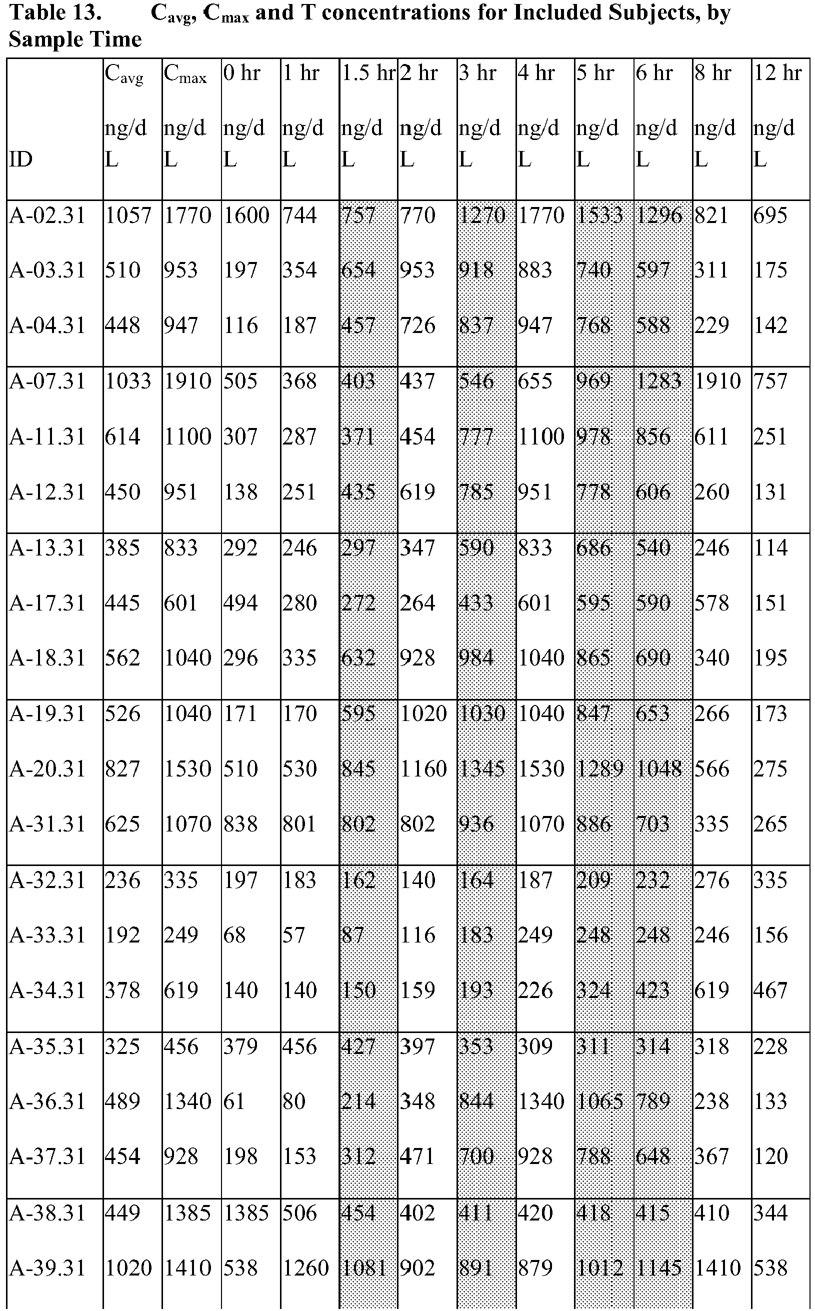

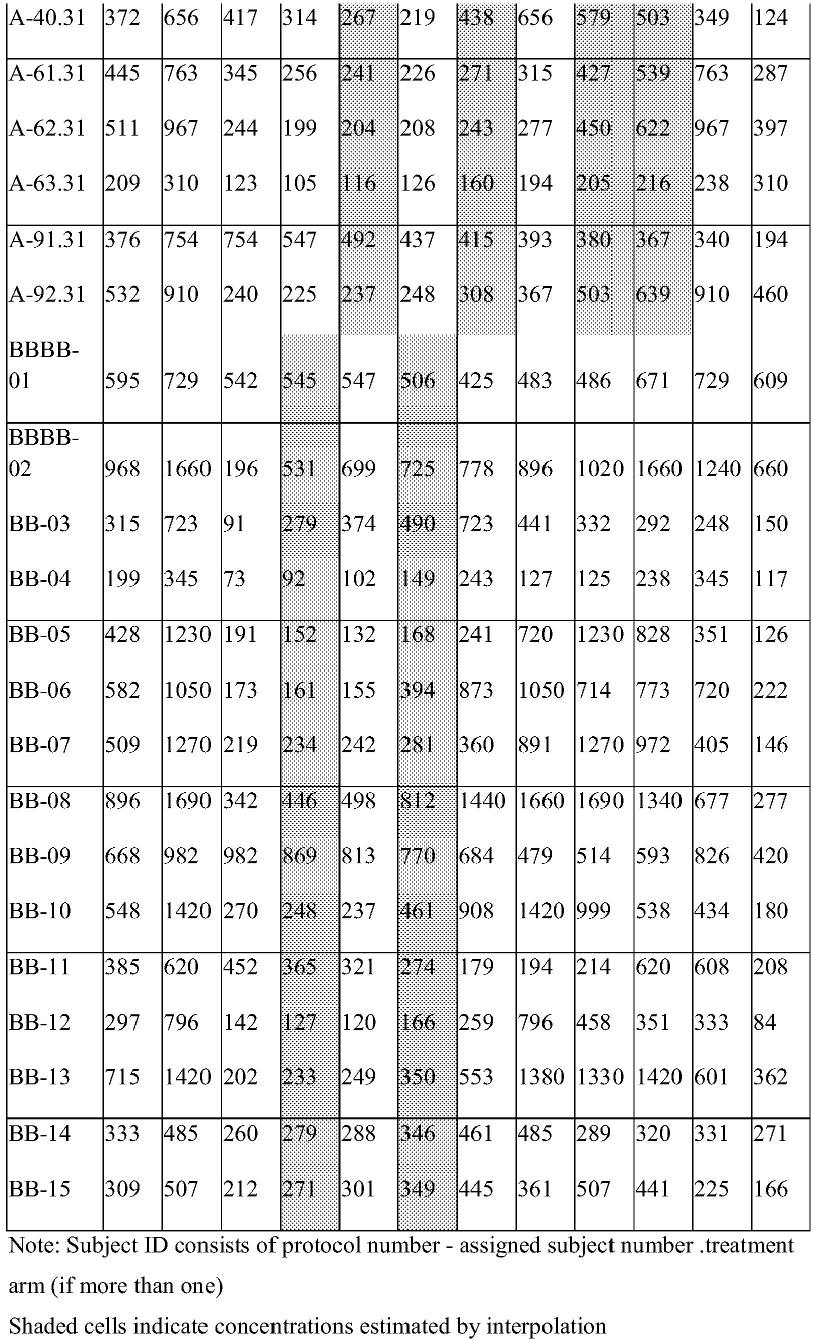

The summary statistics for C_{avg}, Cₘₐₓ and each C(t) are provided in Table 14. In addition, Table 14 provides the correlation coefficient and regression parameters for each candidate C(t) vs. C_{avg} and Cₘₐₓ. The samples collected at 6 hours post dose show the highest degree of correlation against both C_{avg} and Cₘₐₓ, suggesting that C(6) is the best single point estimator for either C_{avg} or Cₘₐₓ. In addition, C(6) has the lowest coefficient of variation among the 10 candidate C(t) data sets, suggesting it may also be among the least susceptible to between-subject variability.

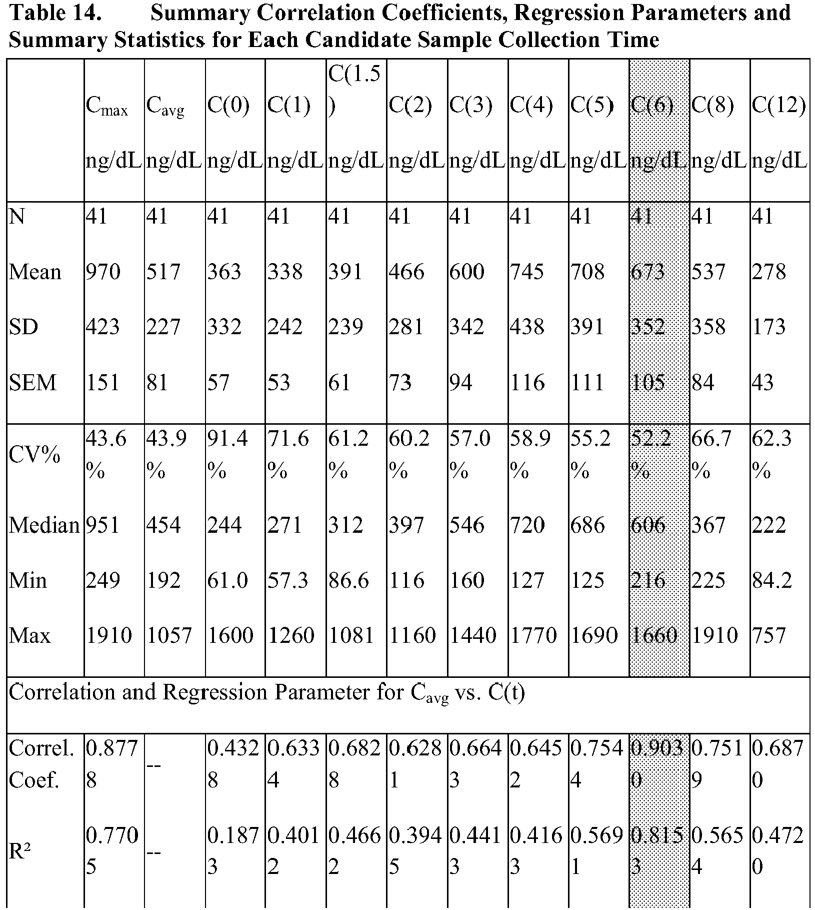

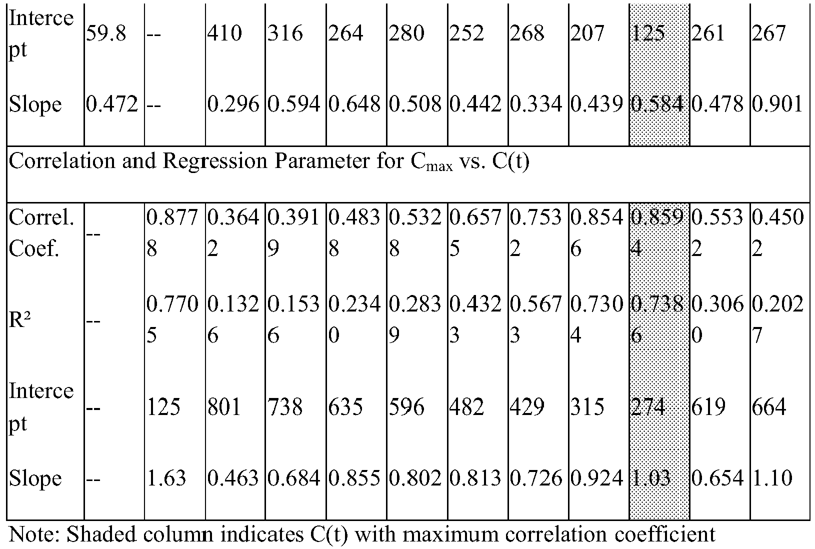

The contingency table results of the search for the optimal range criteria for C(t) are presented in Table 15 and Table 16. The number of subjects in each of the 7 categories, and combinations of categories is summarized in Table 15, while the results are presented as percentages in Table 16. Note that each C(t) has a different upper and lower limit for the acceptance range for C(t), although they share a common target range for C_{avg} (300 ng/dL to 1000 ng/dL).

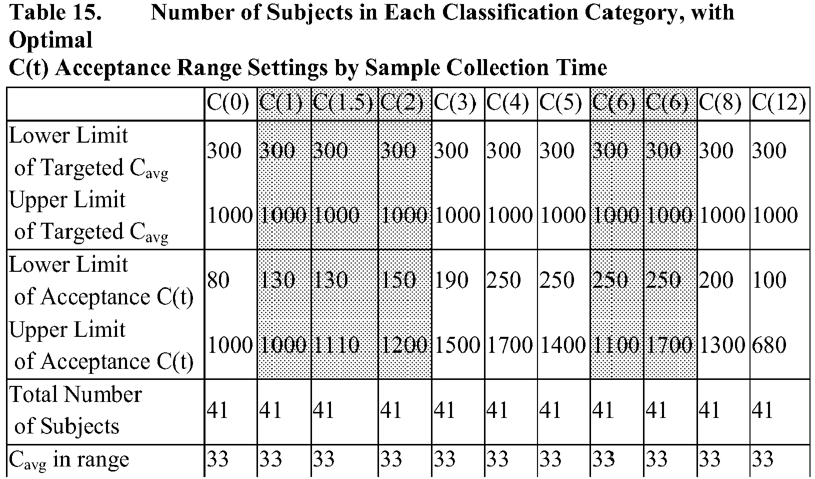

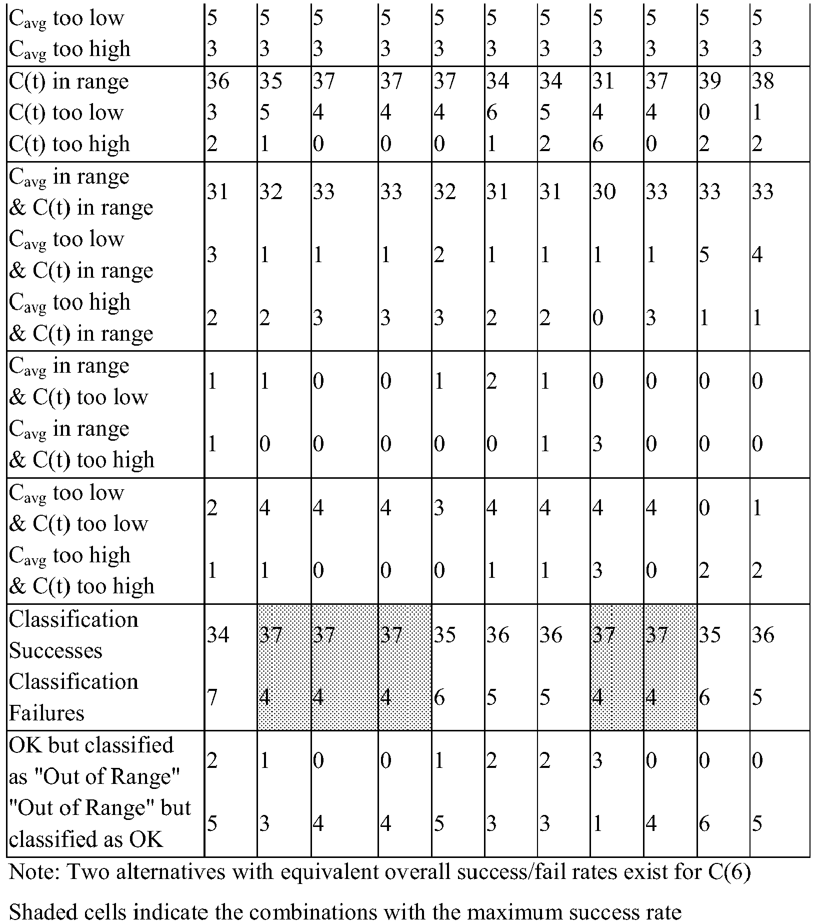

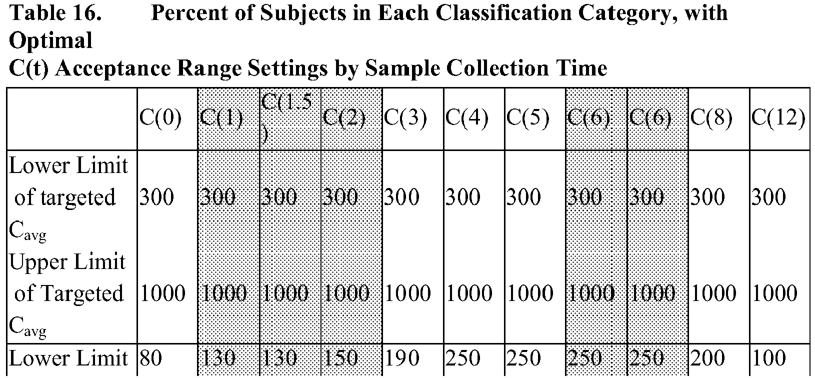

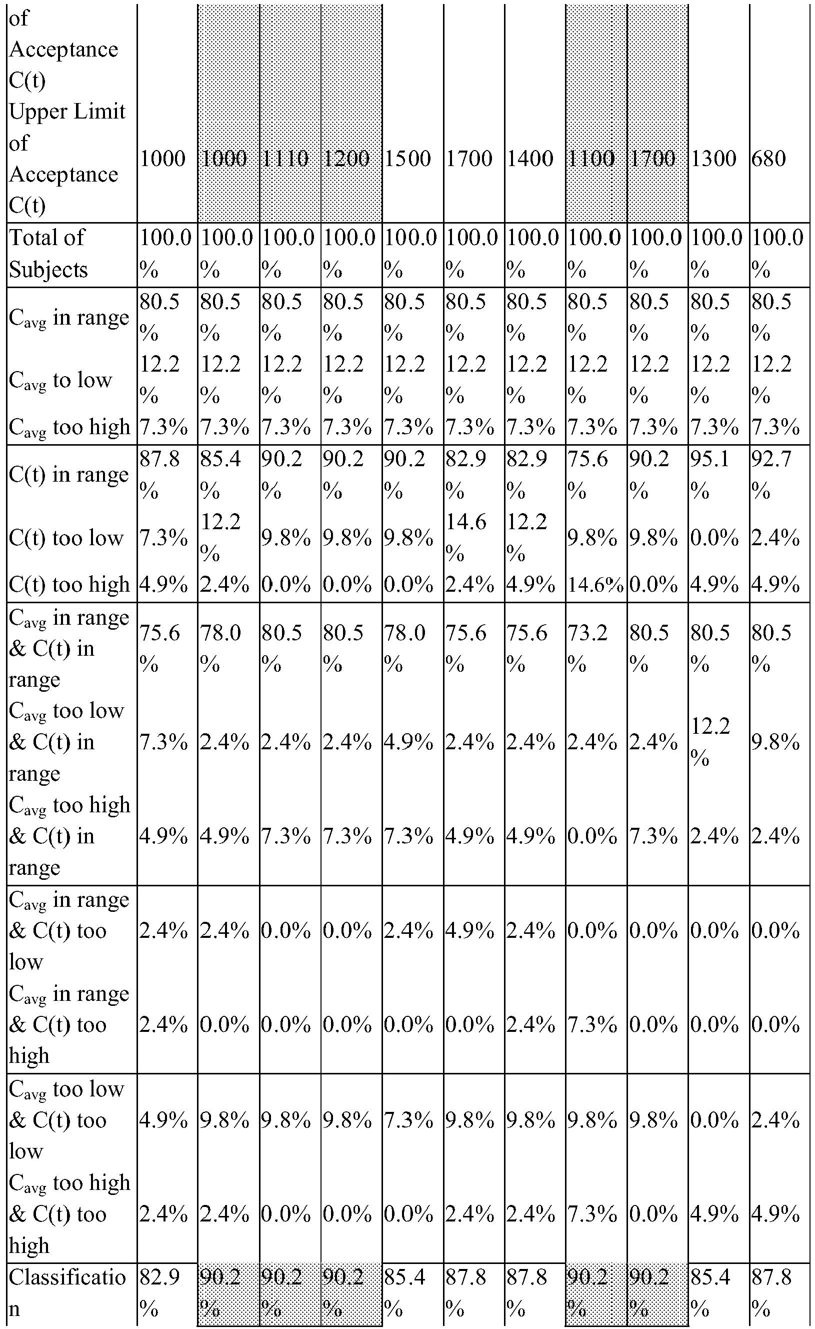

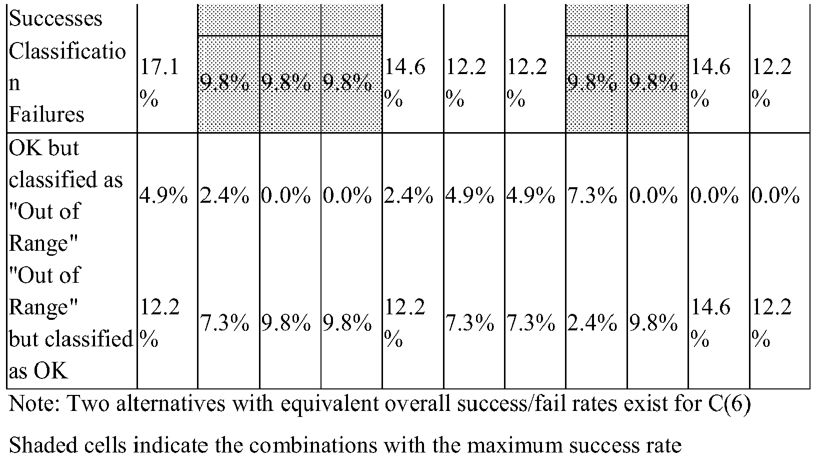

Five combinations of C(t) and designated acceptance ranges for C(t) have been identified that have a 90.2% success rate for predicting C_{avg} values "within range" or "out of range". They are shaded in Table 15 and Table 16 and are C(1), C(1.5), C(2) and C(6). Two candidate acceptance ranges have been identified for use with C(6). C(6) with a designated acceptance range of 250 mg/dL to 1100 ng/dL is felt to be the best choice among these five options because it has the lowest incidence of predicting "false positives", i.e., has the lowest incidence of predicting that a subject has a C_{avg} within the targeted range (300 - 1000 ng/dL), even though the measured C_{avg} was outside the range. It is desirable to minimize the occurrence of this particular outcome since it might result in subjects with higher than desired C_{avg} and Cₘₐₓ values not being recognized.

A set of summary figures are provided end-of-text, which summarize the optimal findings for each of the C(t) and acceptance ranges combinations presented in Table 16. Each tableaux contains a graphical display of the correlation between the C(t) and C_{avg}, along with the regression line, a contingency table summarizing the percentage of subjects within each of the designated categories, and a graph that overlays the selected C_{avg} and C(t) ranges from the contingency analysis on the distribution of concentration data. The data points that fall within the three red rectangles on a graph are "successes" in that the C(t) surrogate has successfully predicted that the subject's C_{avg} is either within the targeted range or outside the targeted range for C_{avg}. Data points outside those red rectangles represent subjects that were not properly categorized, i.e., being either "false negatives" or "false positives".

When reviewing these combination graphs in the end-of-text tableaux it is useful to keep in mind that if a small leftward or rightward movement of one of the vertical red lines will either include or exclude additional data points, then the selection process is very sensitive to that setting and the selection process is not robust to small random variations in concentrations in that concentration range. This particular lack of robustness is evident in the tableaux for C(0), C(1), C(1.5), C(2), C(3), C(4) and C(12). C(6) should be considerably more robust since the same result as is reported in the tables and tableaux holds if the selected lower bound for C(t) takes on any value between 248 and 292 ng/dL (inclusive), and the selected upper bound for C(t) takes on any value between 1048 and 1144 ng/dL (inclusive).

When reviewing the contingency table results or the tableaux, it should also be kept in mind that a different definition of the "target range" for C_{avg} can have a substantial effect on the percentages that are reported into each of the table categories. For example, a decrease in the lower bound for C_{avg} from 300 ng/dL to 295 ng/dL will add an additional subject to the "successful" classifications and increase the success rate from 90.2% to 92.7%. Or an increase in the upper bound of C_{avg} from 1000 ng/dL to 1030 ng/dL will move one subject from a "true failure" designation ("too high" for both C_{avg} and C(6)) to a "false negative" designation ("in range" for C_{avg}, but "too high" according to C(6)).

### Discussion

This analysis was conducted to identify a single sample time that can serve as a surrogate for the time averaged T concentration, C_{avg}. Determination of C_{avg} requires the collection several blood samples over a dosing interval in order to approximate the area under the concentration-time curve (AUC). This need for multiple samples over an extended period is impractical in most clinical settings with outpatients, and so a single sample alternative is desired as a surrogate for C_{avg}.

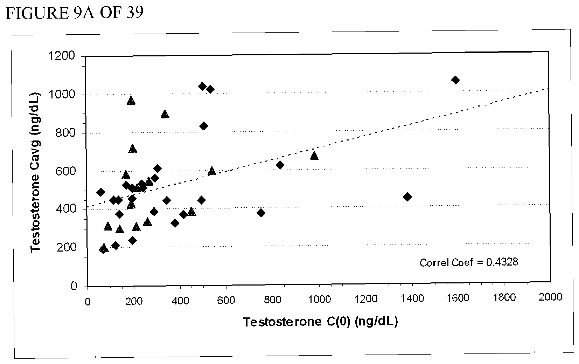

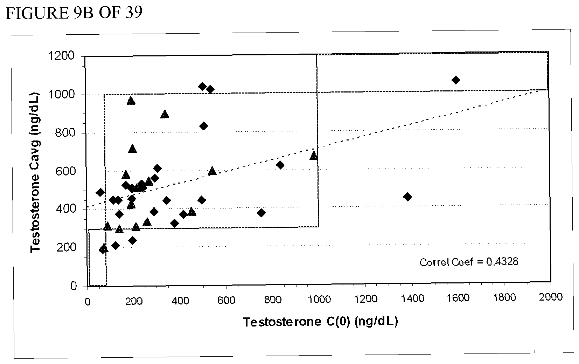

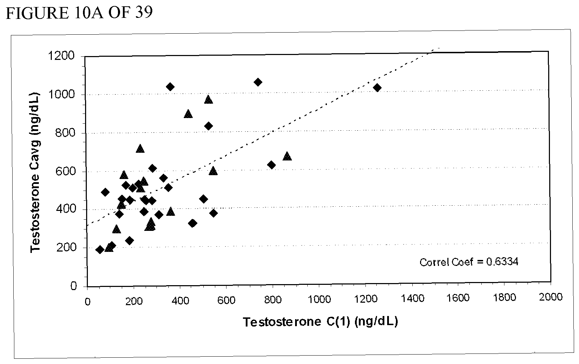

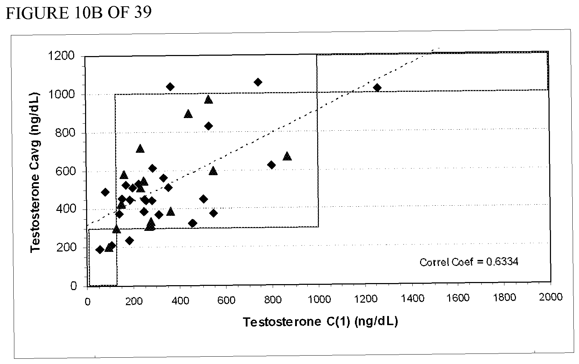

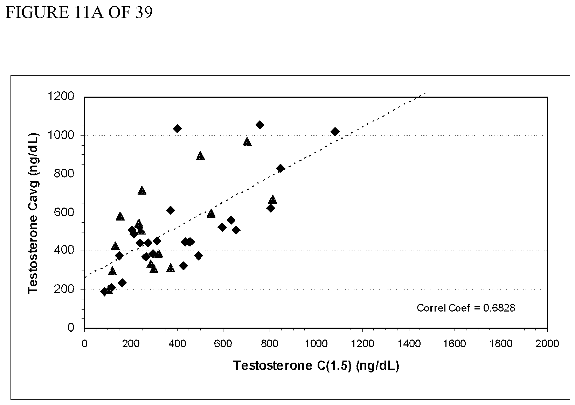

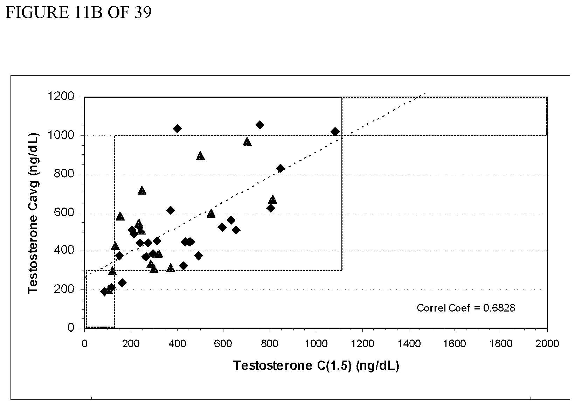

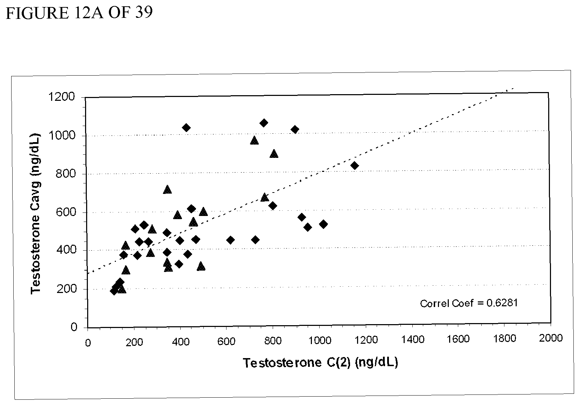

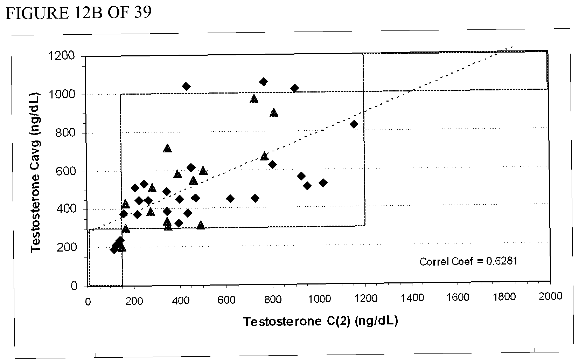

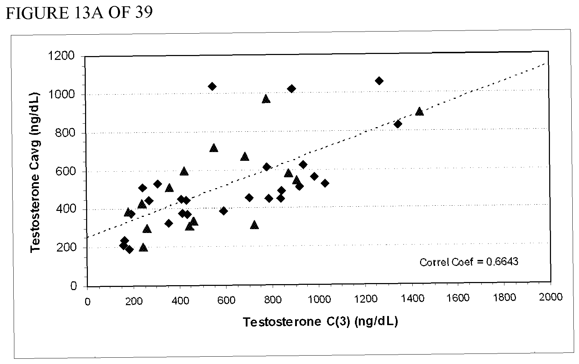

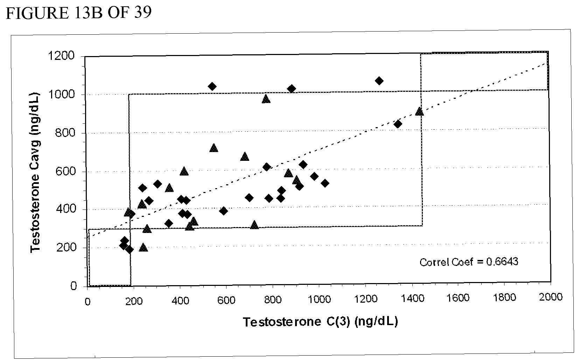

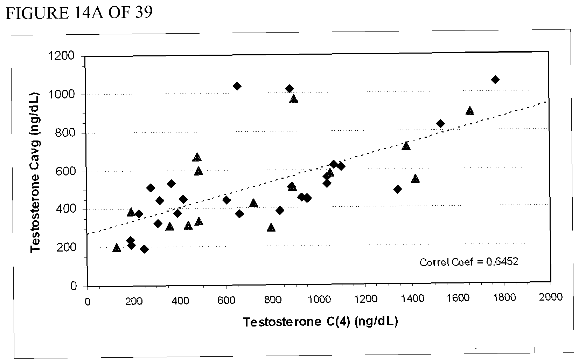

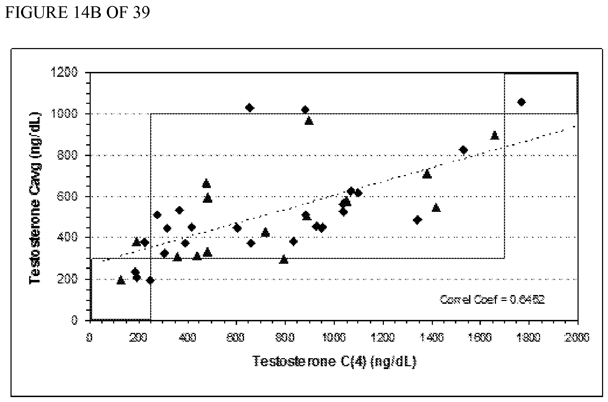

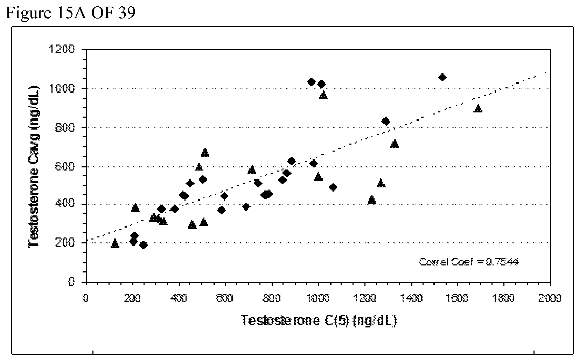

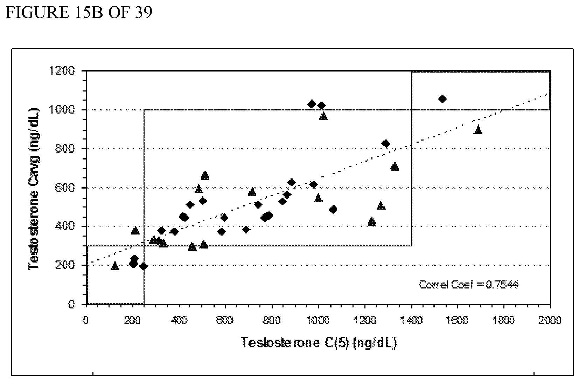

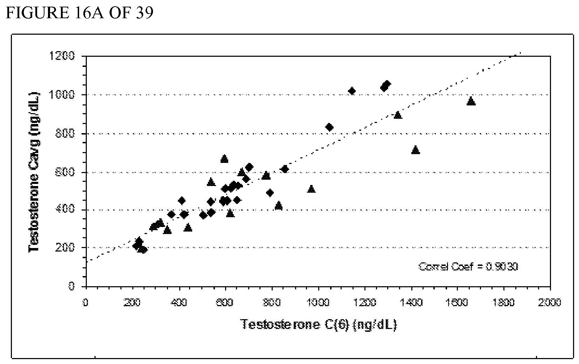

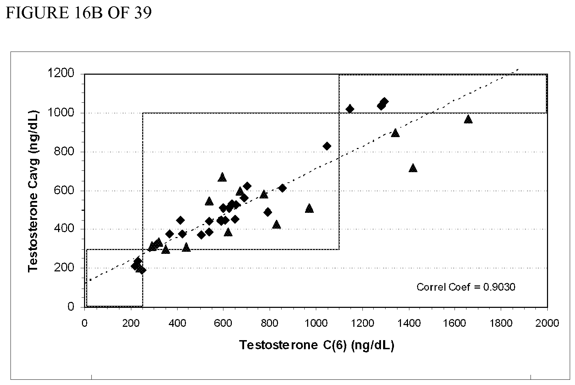

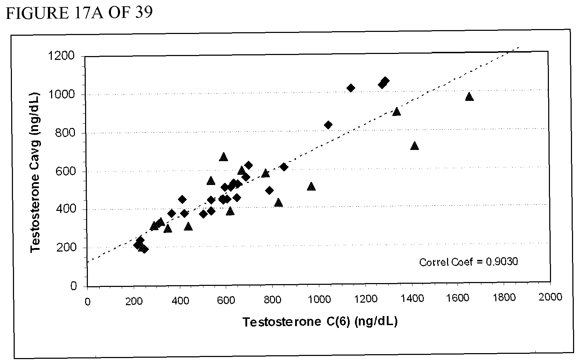

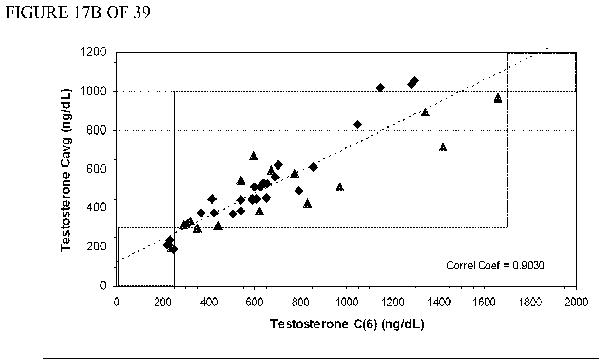

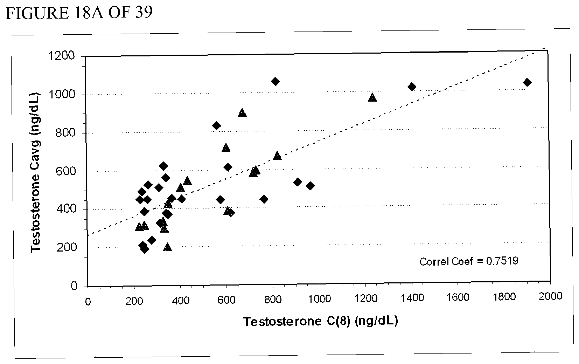

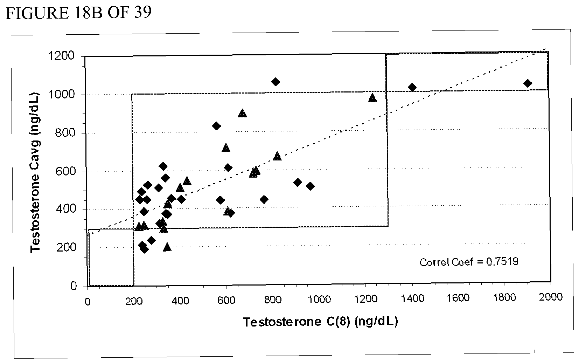

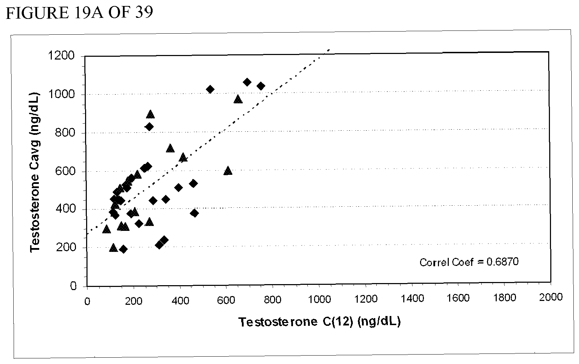

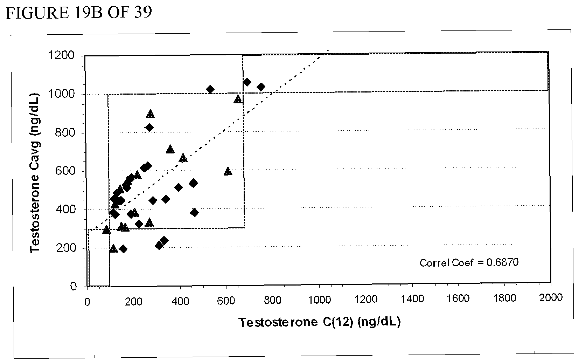

T concentrations from two populations of study subjects were utilized for this analysis. One study provided data from 26 hypogonadal male subjects that received 200 mg BID of T, as TU, for 7 days as the third of four treatments studies in that study protocol. The other study provided data from 15 hypogonadal male subjects that received 200 mg BID of T, as TU, for 28 days. The second study demonstrated that steady-state was reached in 5-7 days, and so participants in both studies were at steady-state at the time blood samples were collected for assaying of T concentrations. A visual review of the clusters of concentration data from the two studies indicates that the results of the two studies were comparable (see Figures 9A - 19B) - the data from both studies show similar clustering and a large degree of overlap in their range of values. However, even if the populations in the two studies could be shown to not be identical, that is not a weakness in the context of this investigation because some heterogeneity in the two studies should result in a more robust finding from the meta-analysis, and the findings developed should be more broadly applicable than if results had been developed from data collected in only one of the studies, or two very similar populations.

The two approaches for identifying a surrogate to C_{avg} - correlation analysis and contingency tables - proved to be complementary. The correlation analysis served to identify the single time point that had concentrations the most tightly correlated to C_{avg} (and to Cₘₐₓ). The results strongly suggest that manipulation of the T dose will alter the C_{avg} value if changing the dose alters the C(6) serum T concentration. An unexpected bonus in this particular investigation is that the C(6) sample time had the lowest coefficient of variation of all the time points examined (although not much lower than the C(3), C(4) or C(5)), suggesting that the decline in T concentrations is less variable in terms of timing than is the rise in concentrations. The contingency analysis identified 5 alternatives for the C_{avg} surrogate, if examined solely based on the success and failure rates. Separation of the failure rate into its contributing factors, the incidence of "false positives" and incidence of "false negatives", resulted in the observation that the C(6) data with the narrower C(t) acceptance range (250-1100 ng/dL) had the lowest rate of "false positives" (2.4% vs. 7.3 to 9.8%). Having false positive, i.e., incorrectly identifying a patient as having a C_{avg} 'in range" when in actuality the C_{avg} is too high, in the therapeutic setting of T replacement can place a subject at risk to maintain higher T concentrations than is recommended to be targeted. Thus, a low "false positive" rate has safety advantages. "False negatives" are unlikely to precipitate a similar problem since they will only lead to an unnecessary dose titration, thereby tending to bring a subject more in line with the population mean, even if it was unnecessary.

Two alternative acceptance ranges for C(6) were identified that provided equivalent success and failure rate (90.2% and 9.8%, respectively). The narrower range (250 - 1100 ng/dL) is believed to be a better choice than 250 - 1700 ng/dL for two reasons. First, the narrower range option results in a lower rate of "false positives", as noted previously, but it also is an acceptance range that is reminiscent of the targeted C_{avg} range for which it is anticipated to serve as a surrogate. Because the proposed surrogate is nearly identical in terms of its upper and lower limits as the targeted C_{avg} range (or the "normal" range for T), the internal consistency and implied logical connection should result in easier acceptance and more consistent implementation by the physician community in their monitoring role.

The search for the optimal acceptance limits for various C(t) candidates demonstrated the critical nature played by the density of data points in the region of a proposed acceptance limit. When the data points are densely packed, as in the cases for the lower limits with C(0), C(1), C(1.5), C(2), C(3), C(4) and C(12), varying the criteria by just 10 ng/dL one way or the other can lead to a detectable change in the predicted success rate. This result suggests that relatively small random variation in assay results under monitoring conditions might result in the wrong choice as to whether to increase a patient's dose. This increased sensitivity to the choice of limit was most apparent for the lower limit, and most frequently encountered when T concentrations tended to be near their trough values. The choice of C(6) as the surrogate of choice helps minimize this particular complication.

Projections were made as to the success rate associated with adjusting the TU dose in subjects identified with C(6) either above or below the acceptance range for C(6). Serum T concentrations from SEDDS TU have been shown to be dose proportional, after correction for the endogenous T concentration (LOT-A), and the endogenous serum T baseline concentrations has been observed to be between 38 and 126 ng/dL (LOT-BB), with a mean value of 108 ng/dL. Of the 6 subjects identified as having C(6) greater than 1100 ng/dL all would be expected to have C(6) and C_{avg} between 400 ng/dL and 900 ng/dL) after dose titration from 200 mg BID of T, as TU, to 100 mg BID of T, as TU. Of the 4 subjects identified as having C(6) less than 250 ng/dL, all four are predicted to have C(6) concentrations above the lower acceptance threshold following a 50% increase in dose (to 300 mg BID T, as TU), but only two or fewer of them are predicted to have a C_{avg} that would actually be above 300 ng/dL, assuming a full pharmacokinetic profile was available to determine the C_{avg}. Therefore, downward titration of the SEDDS TU dose is projected to be successful in a larger portion of the patients needing titration than is upward titration.

It has proven unnecessary to go to similar lengths to identify whether a similar set of correlations and contingency tables can be developed to control Cₘₐₓ. As noted in Table 14, the correlation between Cₘₐₓ and C_{avg} is similar to the correlation between C(6) and Cₘₐₓ, and between C(6) and C_{avg}. Thus controlling C(6) is equivalent to controlling both serum T C_{avg} and Cₘₐₓ. Of the 5 subjects between the two studies that had Cₘₐₓ concentrations greater than 1500 ng/dL (see Table 13), 4 of the 5 were properly detected by the C(6) surrogate as subjects needing a reduction in dosing. Assuming dose proportionality holds for each of those subjects (as demonstrated in study LOT-A), and assuming that the suppressed endogenous T baseline concentrations is approximately 100 ng/dL (as demonstrated in study LOT-BB), all 5 of the subjects would be predicted to respond to a dose reduction of 50% (from 200 mg BID to 100 mg BID) by reductions in their C_{avg} to between 300 and 1000 ng/dL and a reduction in Cₘₐₓ to below 1500 ng/dL. The single subject (subject A-20.31) that had a Cₘₐₓ value greater than 1500 ng/dL, but not a C(6) greater than 1100 ng/dL and was therefore not detected by the C(6) surrogate therefore would not be titrated. His Cₘₐₓ would remain approximately 1530 ng/dL, and he would probably be amongst the small number of subjects permitted to have a Cₘₐₓ value greater than 1500 ng/dL (maximum of 15% of the population). Of importance, there were no cases in this collection of 41 subjects that would be anticipated to have Cₘₐₓ values above 1800 ng/dL or 2500 ng/dL after dose titration, in keeping with using C(6) as a surrogate for C_{avg}.

### Conclusions

Serum T concentration in a blood sample collected 6 hours post-dose, under steady-state dosing conditions (7 or more days after starting treatment) is the suggested surrogate for estimating C_{avg} in the Phase III evaluation of the oral TU product

Setting the acceptance criteria for C(6) as between 250 ng/dL and 1100 ng/dL is projected to result in a 90% success rate in properly categorizing the C_{avg} value as between 300 and 1000 ng/dL, or outside that range.

Serum T C(6) has the highest correlation, of the tested sample collection times, with both C_{avg} and Cₘₐₓ, and controlling C(6) is anticipated to control both serum T C_{avg} and Cₘₐₓ.

Serum T C(6) with a 250-1100 ng/dL acceptance range has the lowest projected rate of false positives, suggesting the choice minimizes the possibility of undetected and uncorrected high T concentrations.

Serum T C(6) as the surrogate for C_{avg} resulted in a substantial projected reduction in the incidence of Cₘₐₓ values greater than 1500 ng/dL. Whereas the pre-titration incidence was observed to be approximately 12%, the post-titration rate, if titration is based on C(6), is projected to be less than 5%.

Based on these analyses, very few subjects dosed at 200 mg T (as TU), BID in a Phase III study are likely to achieve serum T Cmax concentrations > 1500 ng/dL (presuming the hypogonadal men studied in the Phase II study are reflective of likely Phase III study subjects).

It is projected that all subjects with serum T concentrations above the acceptance range for C(6) will respond with C(6) and C_{avg} values to be within the targeted ranges after a 50% reduction in TU dose.

It is projected that all subjects with serum T concentrations below the acceptance range for C(6) (projected to be approximately 10% of the population) will respond with C(6) values to be within the targeted range after a 50% increase in TU dose, however, possibly half or fewer of them will actually have C_{avg} concentrations be above 300 ng/dL.

### Optimal Contingency Table between C_{avg} & C(0)

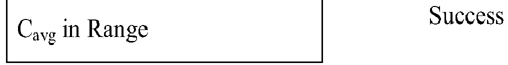

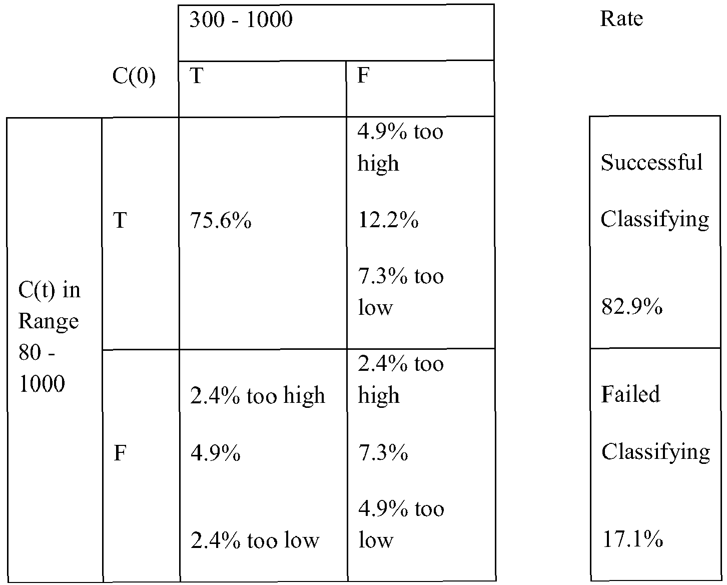

### Optimal Contingency Table between C_{avg} & C(1)

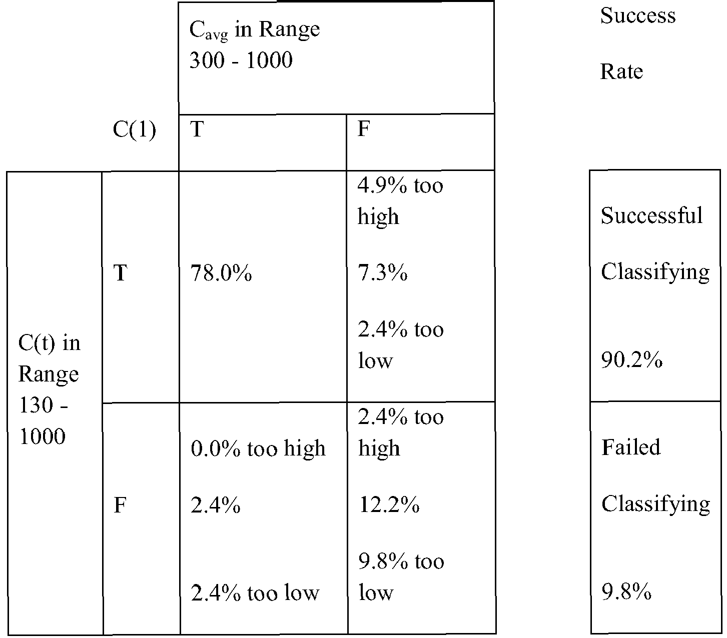

### Optimal Contingency Table between C_{avg} & C(1.5)

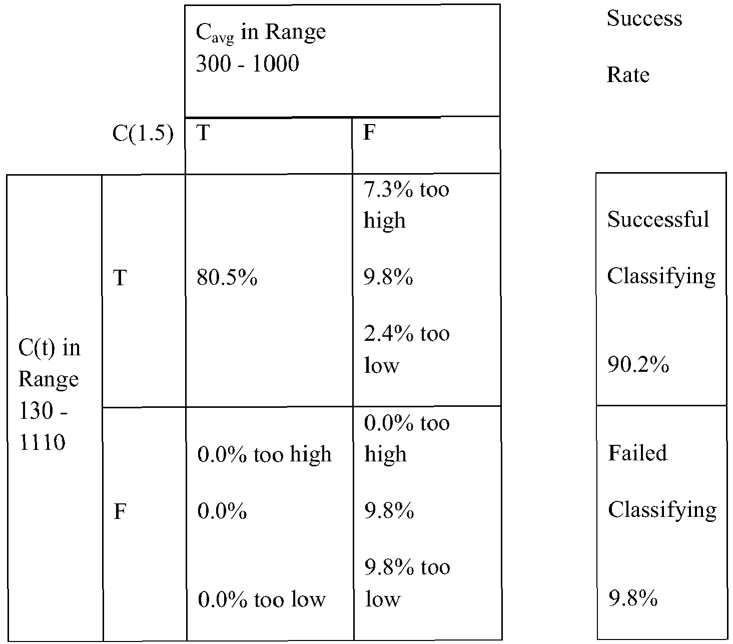

### Optimal Contingency Table between C_{avg} & C(2)

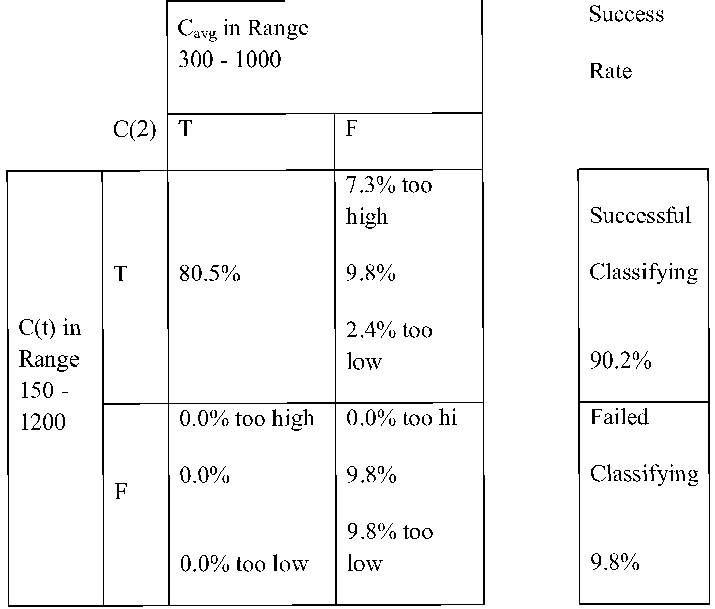

### Optimal Contingency Table between C_{avg} & C(3)

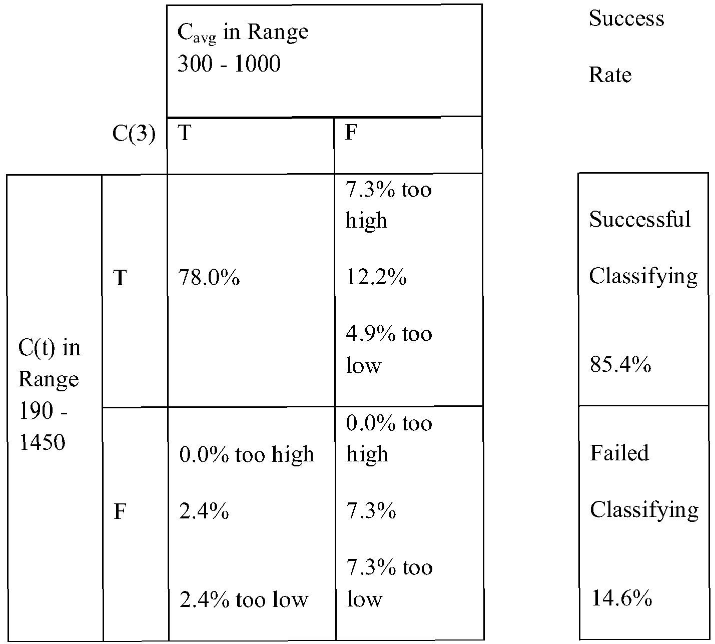

### Optimal Contingency Table between C_{avg} & C(4)

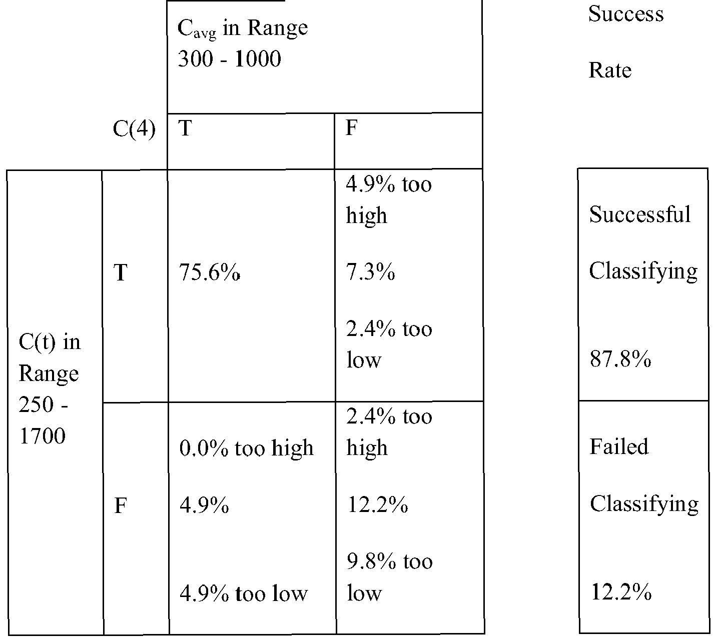

### Optimal Contingency Table between C_{avg} & C(5)

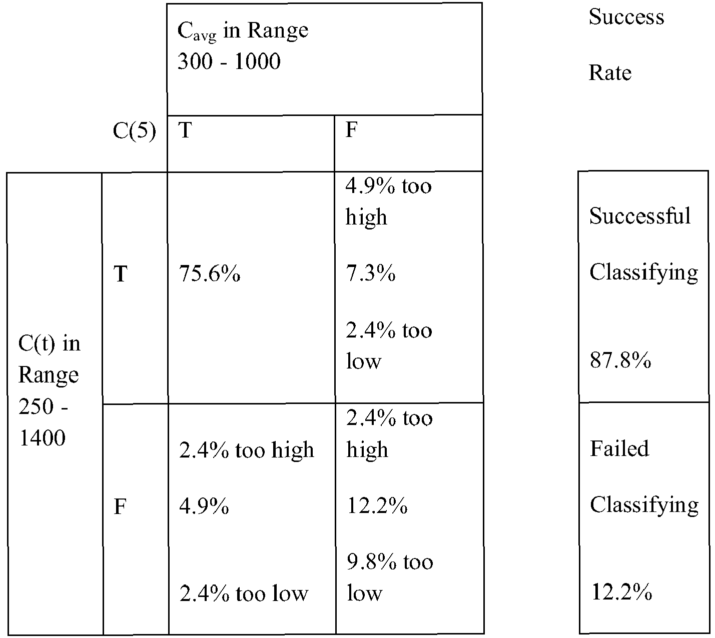

### Optimal Contingency Table between C_{avg} & C(6)

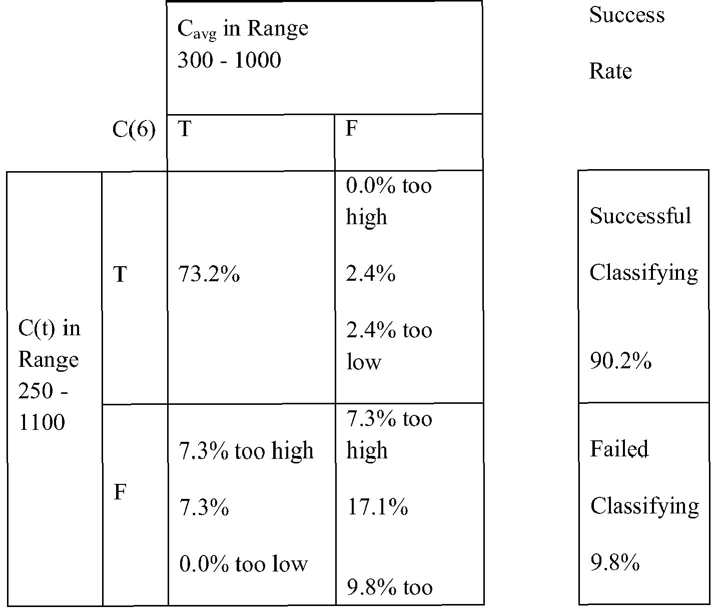

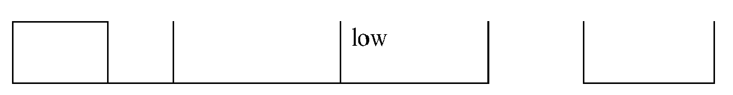

### Alternate Optimal Contingency Table between C_{avg} & C(6)

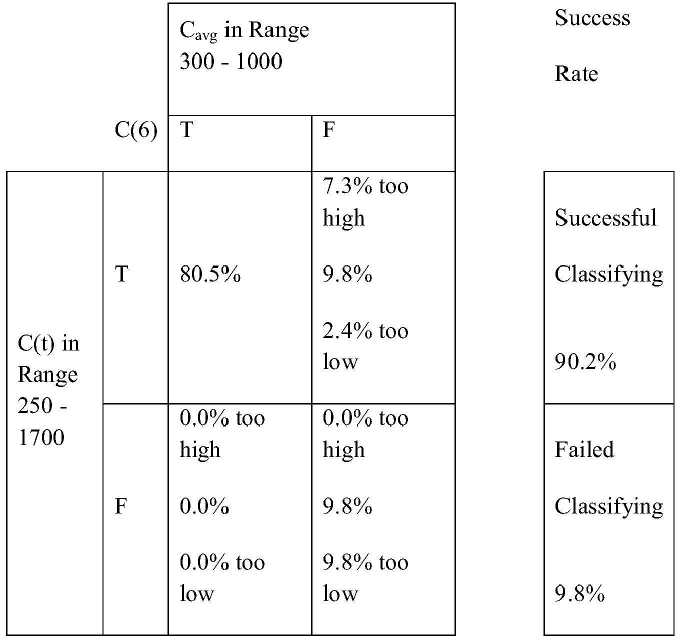

### Optimal Contingency Table between C_{avg} & C(8)

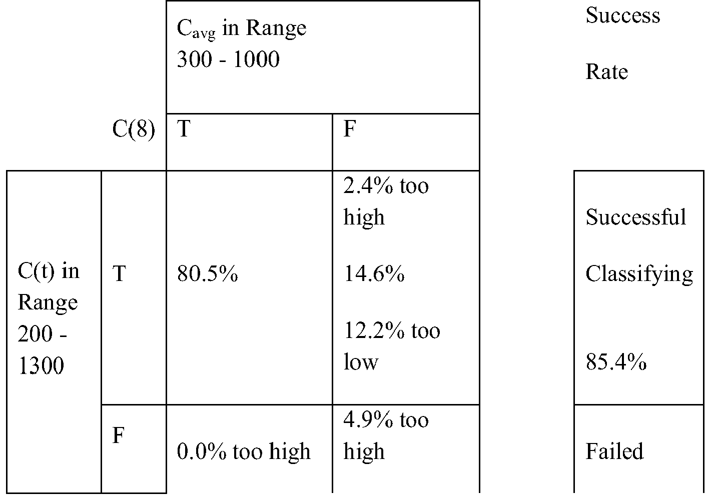

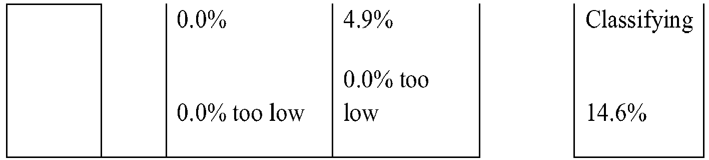

### Optimal Contingency Table between C_{avg} & C(12)

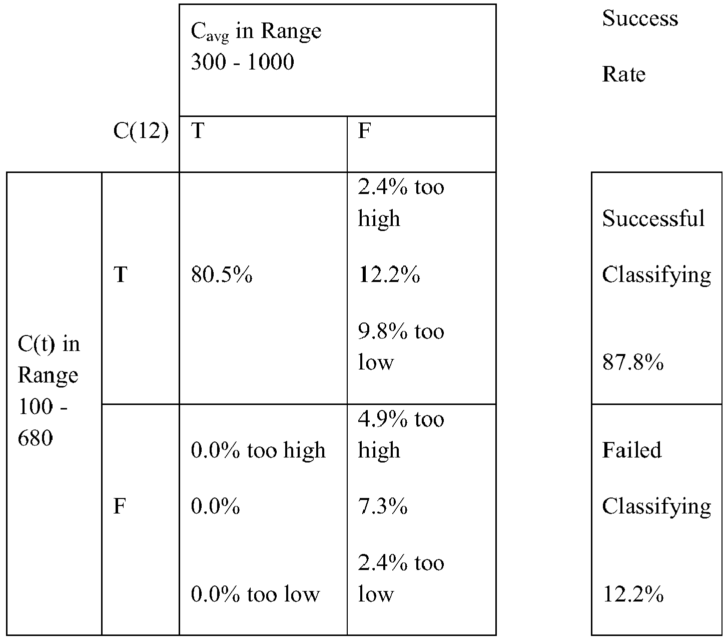

### Examples - LOTUS 1

Table 1 provides composition details of various formulations of testosterone (T) or testosterone-esters (T-esters), in accordance with the teachings of the instant invention. For calculation purposes, 1 mg of T is equivalent to: 1.39 mg T-enanthate; 1.58 mg T-undecanoate; 1.43 mg T-cypionate, and 1.83 mg T-palmitate. TP is a preferred T-ester in some of the formulations listed below. The compositions details of Table 1 (mg/capsule and wt. percentage) are based on 800 mg fill weight per '00' hard gelatin capsule. However, at testosterone-ester amounts less than about 100 mg/capsule, the formulations may be proportionally adjusted for smaller total fill weights that would permit use of smaller hard gelatin capsules (e.g., '0' size).

As well, it should be apparent to one of ordinary skill in the art that many, if not all, of the surfactants within a category (e.g., lipophilic, hydrophilic, etc.) may be exchanged with another surfactant from the same category. Thus, while Table 1 lists formulations comprising Labrafil M1944CS (HLB = 3) and Precirol ATO5 (HLB = 2), one of ordinary skill in the art should recognize other lipophilic surfactants (e.g., those listed above) may be suitable as well. Similarly, while Table 15 lists formulations comprising polyoxyethyelene (40) hydrogenated castor oil (HLB = 13) and Labrasol (HLB = 14), one of ordinary skill in the art should recognize other hydrophilic surfactants (e.g., those listed above) may be suitable.

**Table 15**

| ID | T or T-ester | Labrafil M1944CS | Precirol AT05 | Cremophor RH40 | Labrasol |
|---|---|---|---|---|---|
| A | 400 | 109.68 | 66.49 | 223.83 | - |
| | 50.00% | 13.71% | 8.31% | 27.98% | - |
| B | 360 | 120.64 | 73.14 | 246.21 | - |
| | 45.00% | 15.08% | 9.14% | 30.78% | - |
| C | 320 | 131.61 | 79.79 | 268.60 | - |
| | 40.00% | 16.45% | 9.97% | 33.57% | - |
| D | 280 | 142.58 | 86.44 | 290.98 | - |
| | 35.00% | 17.82% | 10.80% | 36.37% | - |
| E | 240 | 153.55 | 93.09 | 313.36 | - |
| | 30.00% | 19.19% | 11.64% | 39.17% | - |
| F | 228.32 | 156.75 | 95.03 | 319.9 | - |
| | 28.54% | 19.59% | 11.88% | 39.99% | - |
| G | 200 | 164.52 | 99.74 | 335.75 | - |
| | 25.00% | 20.56% | 12.47% | 41.97% | - |
| H | 160 | 175.48 | 106.39 | 358.13 | - |
| | 20.00% | 21.94% | 13.30% | 44.77% | - |
| I | 120 | 186.45 | 113.04 | 380.51 | - |
| | 15.00% | 23.31% | 14.13% | 47.56% | - |
| J | 80 | 197.42 | 119.69 | 402.90 | - |
| | 10.00% | 24.68% | 14.96% | 50.36% | - |
| K | 40 | 208.39 | 126.33 | 425.28 | - |
| | 5.00% | 26.05% | 15.79% | 53.16% | - |
| L | 20 | 213.87 | 129.66 | 436.47 | - |
| | 2.50% | 26.73% | 16.21% | 54.56% | - |
| M | 400 | 199.97 | 66.62 | 133.40 | - |
| | 50.00% | 25.00% | 8.33% | 16.68% | - |
| N | 360 | 219.97 | 73.29 | 146.74 | - |
| | 45.00% | 27.50% | 9.16% | 18.34% | - |
| O | 320 | 239.97 | 79.95 | 160.08 | - |
| | 40.00% | 30.00% | 9.99% | 20.01% | - |
| P | 280 | 259.96 | 86.61 | 173.42 | - |
| | 35.00% | 32.50% | 10.83% | 21.68% | - |
| Q | 240 | 279.96 | 93.27 | 186.76 | - |
| | 30.00% | 35.00% | 11.66% | 23.35% | - |
| R | 228.32 | 285.8 | 95.22 | 190.66 | - |
| | 28.54% | 35.73% | 11.90% | 23.83% | - |
| S | 200 | 299.96 | 99.94 | 200.10 | - |
| | 25.00% | 37.49% | 12.49% | 25.01% | - |
| T | 160 | 319.96 | 106.60 | 213.45 | - |
| | 20.00% | 39.99% | 13.32% | 26.68% | - |
| U | 120 | 339.95 | 113.26 | 226.79 | - |
| | 15.00% | 42.49% | 14.16% | 28.35% | - |
| V | 80 | 359.95 | 119.92 | 240.13 | - |
| | 10.00% | 44.99% | 14.99% | 30.02% | - |
| W | 40 | 379.95 | 126.59 | 253.47 | - |
| | 5.00% | 47.49% | 15.82% | 31.68% | - |
| X | 20 | 389.95 | 129.92 | 260.14 | - |
| | 2.50% | 48.74% | 16.24% | 32.52% | - |
| AA | 400 | 109.79 | 66.55 | 149.72 | 73.94 |
| | 50.00% | 13.72% | 8.32% | 18.72% | 9.24% |
| BB | 360 | 120.77 | 73.21 | 164.69 | 81.33 |
| | 45.00% | 15.10% | 9.15% | 20.59% | 10.17% |
| CC | 320 | 131.75 | 79.87 | 179.66 | 88.72 |
| | 40.00% | 16.47% | 9.98% | 22.46% | 11.09% |
| DD | 280 | 142.73 | 86.52 | 194.64 | 96.12 |
| | 35.00% | 17.84% | 10.82% | 24.33% | 12.01% |
| EE | 240 | 153.70 | 93.18 | 209.61 | 103.51 |
| | 30.00% | 19.21% | 11.65% | 26.20% | 12.94% |
| FF | 228.32 | 156.91 | 95.12 | 213.98 | 105.67 |
| | 28.54% | 19.61% | 11.89% | 26.75% | 13.21% |
| GG | 200 | 164.68 | 99.83 | 224.58 | 110.90 |
| | 25.00% | 20.59% | 12.48% | 28.07% | 13.86% |
| HH | 160 | 175.66 | 106.49 | 239.55 | 118.30 |
| | 20.00% | 21.96% | 13.31% | 29.94% | 14.79% |
| II | 120 | 186.64 | 113.14 | 254.52 | 125.69 |
| | 15.00% | 23.33% | 14.14% | 31.82% | 15.71% |
| JJ | 80 | 197.62 | 119.80 | 269.50 | 133.09 |
| | 10.00% | 24.70% | 14.97% | 33.69% | 16.64% |
| KK | 40 | 208.60 | 126.45 | 284.47 | 140.48 |
| | 5.00% | 26.07% | 15.81% | 35.56% | 17.56% |
| LL | 20 | 214.09 | 129.78 | 291.95 | 144.18 |
| | 2.50% | 26.76% | 16.22% | 36.49% | 18.02% |
| MM | 400 | 81.62 | 94.47 | 223.91 | - |
| | 50.00% | 10.20% | 11.81% | 27.99% | - |
| NN | 360 | 89.78 | 103.92 | 246.30 | - |
| | 45.00% | 11.22% | 12.99% | 30.79% | - |
| OO | 320 | 97.94 | 113.37 | 268.69 | - |
| | 40.00% | 12.24% | 14.17% | 33.59% | - |
| PP | 280 | 106.10 | 122.81 | 291.08 | - |
| | 35.00% | 13.26% | 15.35% | 36.39% | - |
| QQ | 240 | 114.27 | 132.26 | 313.47 | - |
| | 30.00% | 14.28% | 16.53% | 39.18% | - |
| RR | 228.32 | 116.65 | 135.02 | 320.01 | - |
| | 28.54% | 14.58% | 16.88% | 40.00% | - |
| SS | 200 | 122.43 | 141.71 | 335.86 | - |
| | 25.00% | 15.30% | 17.71% | 41.98% | - |
| TT | 160 | 130.59 | 151.16 | 358.25 | - |
| | 20.00% | 16.32% | 18.89% | 44.78% | - |
| UU | 120 | 138.75 | 160.60 | 380.64 | - |
| | 15.00% | 17.34% | 20.08% | 47.58% | - |
| VV | 80 | 146.91 | 170.05 | 403.04 | - |
| | 10.00% | 18.36% | 21.26% | 50.38% | - |
| WW | 40 | 155.08 | 179.50 | 425.43 | - |
| | 5.00% | 19.38% | 22.44% | 53.18% | - |
| XX | 20 | 159.16 | 184.22 | 436.62 | - |
| | 2.50% | 19.89% | 23.03% | 54.58% | - |

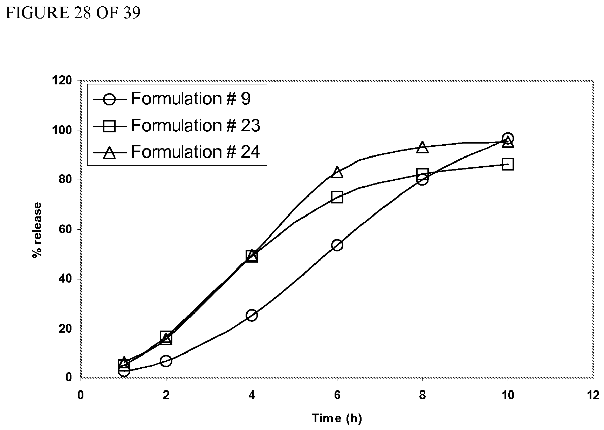

Table 16 provides composition details of various TP formulations in accordance with the teachings of the instant invention and Figure 28provides in vitro dissolution of select formulations therein. TP may be synthesized through esterification of testosterone with palmitoyl chloride in an acetone/pyridine mixture. Testosterone palmitate crude is purified by filtration, crystallized from a methanol/methylene chloride mixture and washed with methanol. When necessary, recrystallization can be done from heptane, followed by washing with methanol.

**Table 16**

| F. No. | Composition details (mg/capsule and wt. percentage)* I | | | | | | | | | | Fill wt (mg)* * |
|---|---|---|---|---|---|---|---|---|---|---|---|
| | TP | LBR | PRC5 | OA | Peceol | TPGS | SO | CRH4 0 | L'sol | M'tol | |
| 1 | 228. 32 (40.0 ) | 285.8 4 (50.0) | 57 (10.0) | | | | | | | | 570 |
| 2 | 228. 32 (40.0 ) | | 57 (10.0) | 228 (40.0) | 57 (10.0) | | | | | | 570 |
| 3 | 228. 32 (40.0 ) | 171 (30.0) | | 114 (20.0) | 57 (10.0) | | | | | | 570 |
| 4 | 228. 32 (40.0 ) | 171 (30.0) | | 114 (20.0) | | 57 (10.0) | | | | | 570 |
| 5 | 228. 32 (40.0 ) | | | 114 (20.0) | | 57 (10.0) | 171 (30.0) | | | | 570 |
| 6 | 228. 32 (28.5 ) | 476 (59.5) | 95.2 (11.9) | | | | | | | | 800 |
| 7 | 228. 32 (28.5 ) | | 95.2 (11.9) | 380.8 (47.6) | 95.2 (11.9) | | | | | | 800 |
| 8 | 228. 32 (28.5 ) | | | 190.4 (23.8) | | 95.2 (11.9) | 285.6 (35.7) | | | | 800 |
| 9 | 228. 32 (28.5 ) | 285.8 4 (35.7) | 95.2 (11.9) | | | | | 190.5 6 (23.8) | | | 800 |
| 10 | 228. 32 (28.5 ) | | | 190.5 6 (23.8) | | 190.56 (23.8) | 190.56 (23.8) | | | | 800 |
| 11 | 228. 32 (28.5 ) | 190.5 6 (23.8) | 95.2 (11.9) | 190.5 6 (23.8) | | | | 95.2 (11.9) | | | 800 |
| 12 | 228. 32 (28.5 ) | 190.5 6 (23.8) | | 190.5 6 (23.8) | 95.2 (11.9) | | | | 95.2 (11.9) | | 800 |
| 13 | 228. 32 (28.5 ) | 190.5 6 (23.8) | | | | | 190.56 (23.8) | 95.2 (11.9) | 95.2 (11.9) | | 800 |
| 14 | 228. 32 (28.5 ) | | | 285 (35.7) | 95.2 (11.9) | 95.2 (11.9) | | | 95.2 (11.9) | | 800 |
| 15 | 228. 32 (28.5 ) | 285.8 4 (35.7) | 20.0 (2.50) | | | | | 265.6 (33.2) | | | 800 |
| 16 | 228. 32 (28.5 ) | 285.8 4 (35.7) | 20.0 (2.50) | 40.0 (5.00) | | | | 225.6 (28.2) | | | 800 |
| 17 | 228. 32 (28.5 ) | 285.8 4 (35.7) | | 80.0 (10.0) | | | | 205.6 (25.7) | | | 800 |
| 18 | 228. 32 (28.5 ) | 95.20 (11.9) | | 190.5 6 (23.8) | | | | 285.6 (35.7) | | | 800 |
| 19 | 228. 32 (50.7 3) | 133.0 8 (29.5 7) | | | | | | 88.67 2 (19.7) | | | 450 |
| 20 | 228. 32 (28.5 ) | 285.8 4 (35.7) | | | | | | 200.2 8 (25.0) | 85.72 (10.7) | | 800 |
| 21 | 228. 32 (28.5 ) | 285.8 4 (35.7) | 95.2 (11.9) | | | | | | | 190.6 7 (23.8 ) | 800 |
| 22 | 228. 32 (28.5 ) | 240.3 3 (30.0) | 65.7 (8.2) | | | | | 160.2 2 (20.0) | | 105.7 4 (13.2 ) | 800 |
| 23 | 228. 32 (28.5 ) | 157.0 2 (19.6) | 95.2 (11.9) | | | | | 320.4 5 (40.0) | | | 800 |
| 24 | 228. 32 (28.5 ) | 157.0 2 (19.6) | 95.2 (11.9) | | | | | 214.4 (26.8) | 105.7 4 (13.2) | | 800 |
| 25 | 228. 32 (28.5 ) | 157.0 2 (19.6) | 65.6 (8.2) | | | | | 349.6 (43.7) | | | 800 |
| 26 | 228. 32 (28.5 ) | 157.0 2 (19.6) | 40.0 (5.0) | | | | | 375.2 (46.9) | | | 800 |
| 57 | 182. 65 (22.8 3) | 229.3 5 (28.7) | 20.0 (2.5) | | | | | 368.0 (46.0) | | | 800 |
| 58 | 120. 0 (15.0 ) | 520.0 (65.0) | 20.0 (2.5) | | | | | 140.0 (17.5) | | | 800 |

| | | | | | | | | | | | |
|---|---|---|---|---|---|---|---|---|---|---|---|
| * TP: Testosterone palmitate; LBR: Labrafil M1944CS; PRC5: PrecirolATO5; OA: Refined Oleic acid; SO: Refined Soybean oil; TPGS: D-α-tocopheryl PEG1000 succinate; CRH 40: polyoxyethyelene (40) hydrogenated castor oil; L'sol: Labrasol; M'tol: Mannitol ** Filled into size"0" capsule (570 mg) or "00"capsule (800mg) | | | | | | | | | | | |

A preferred formulation of TP in accordance with the present invention is:

| Component | mg/capsule | %, w/w |
|---|---|---|
| Testosterone palmitate | 228.32 | 28.5 |
| Cremophor^{®} RH40 | 320.45 | 40.0 |
| Labrafil^{®} M 1944 CS | 157.02 | 19.6 |
| Precirol^{®} ATO 5 | 95.20 | 11.9 |
| Total: | 800 | 100.0 |

In some embodiments, it may be desirable to reduce the absolute concentration of testosterone and/or an ester thereof in order to promote a relatively faster release of the testosterone and/or ester from within the lipid vehicle. That is, it has been found, surprisingly, that reducing the concentration of TP, may in some cases, confer quicker release kinetics. For example, for significant release of TP within about a two hour period, a concentration of TP of less than about 23 percent by weight. In some embodiment, a weight percentage of less than about 20 is preferred, more preferably a weight percentage of less than about 18, and most preferably a weight percentage of less than about 15. Without being bound by or limited to theory, it is believed that TP at levels greater than about 23 weight percent may, in fact, retard its own release. For example, formulations according to the instant invention comprising less than about 23 weight percent TP can release 50 -70 % of the drug at 1 hour and 80 to near 100% at 2 hours. On the other hand, formulations according to the instant invention comprising greater than about 23 weight percent TP release less than 5 % of the drug at 1 hr and less than 70% at 6 hours.

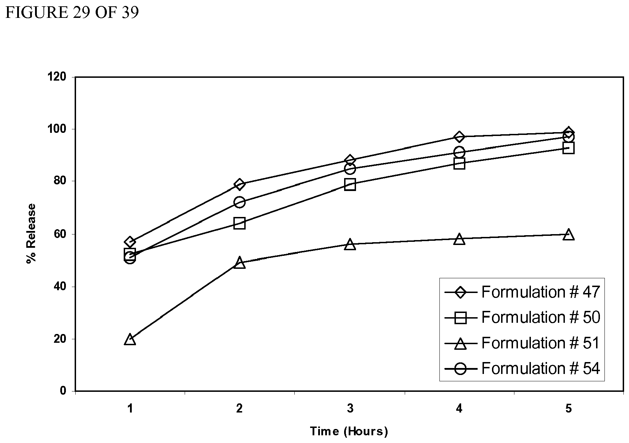

Table 17 provides composition details of various TP formulations that in some cases, are at TP concentrations lower than those in Table 2 and in accordance with the teachings of the instant invention. Figure 29 provides in vitro dissolution of select Table 3 formulations.

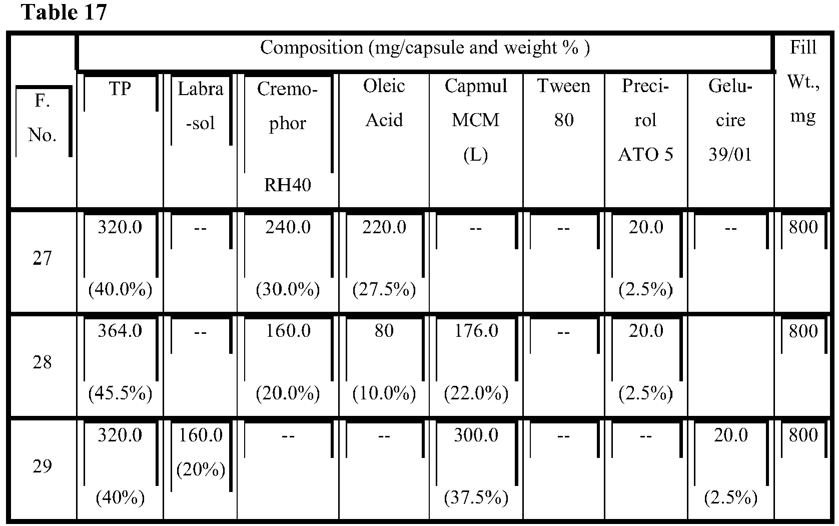

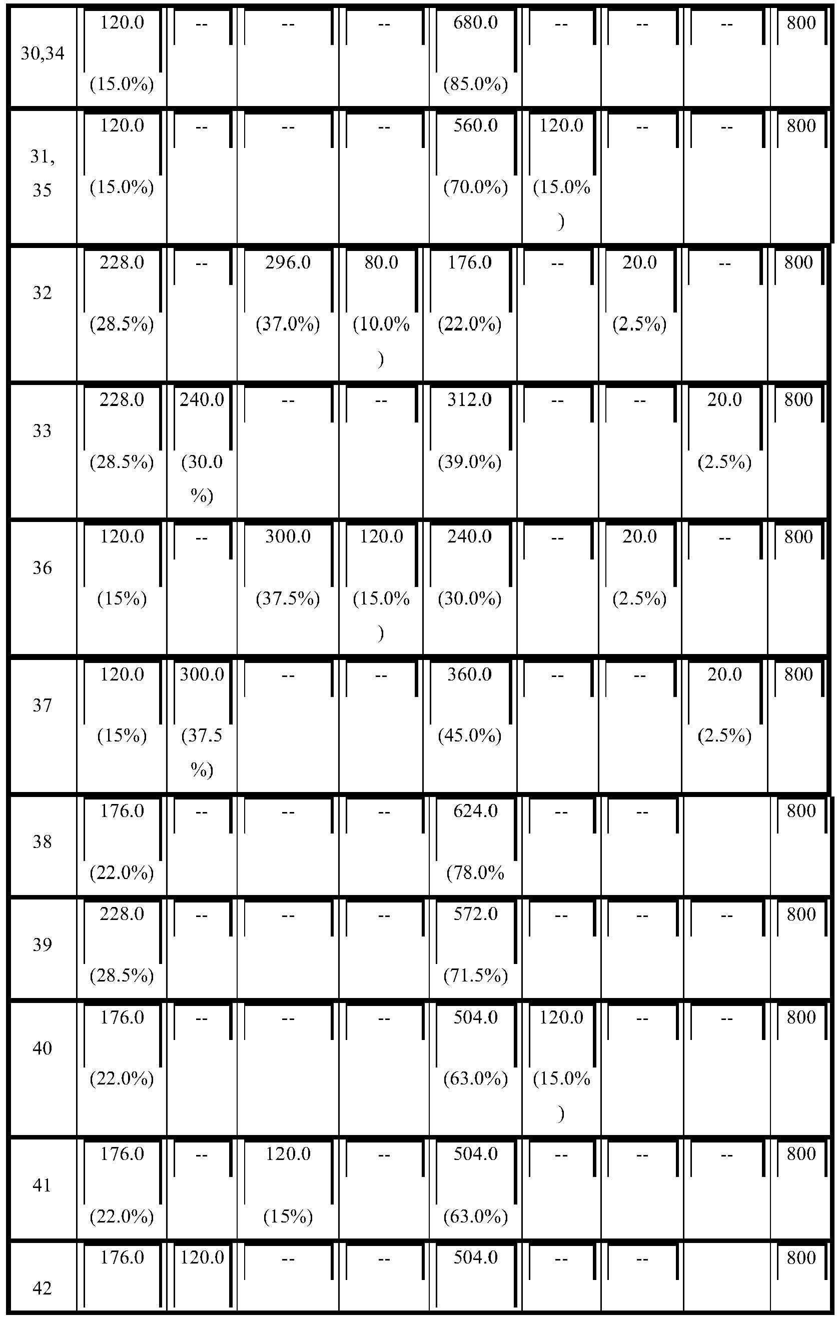

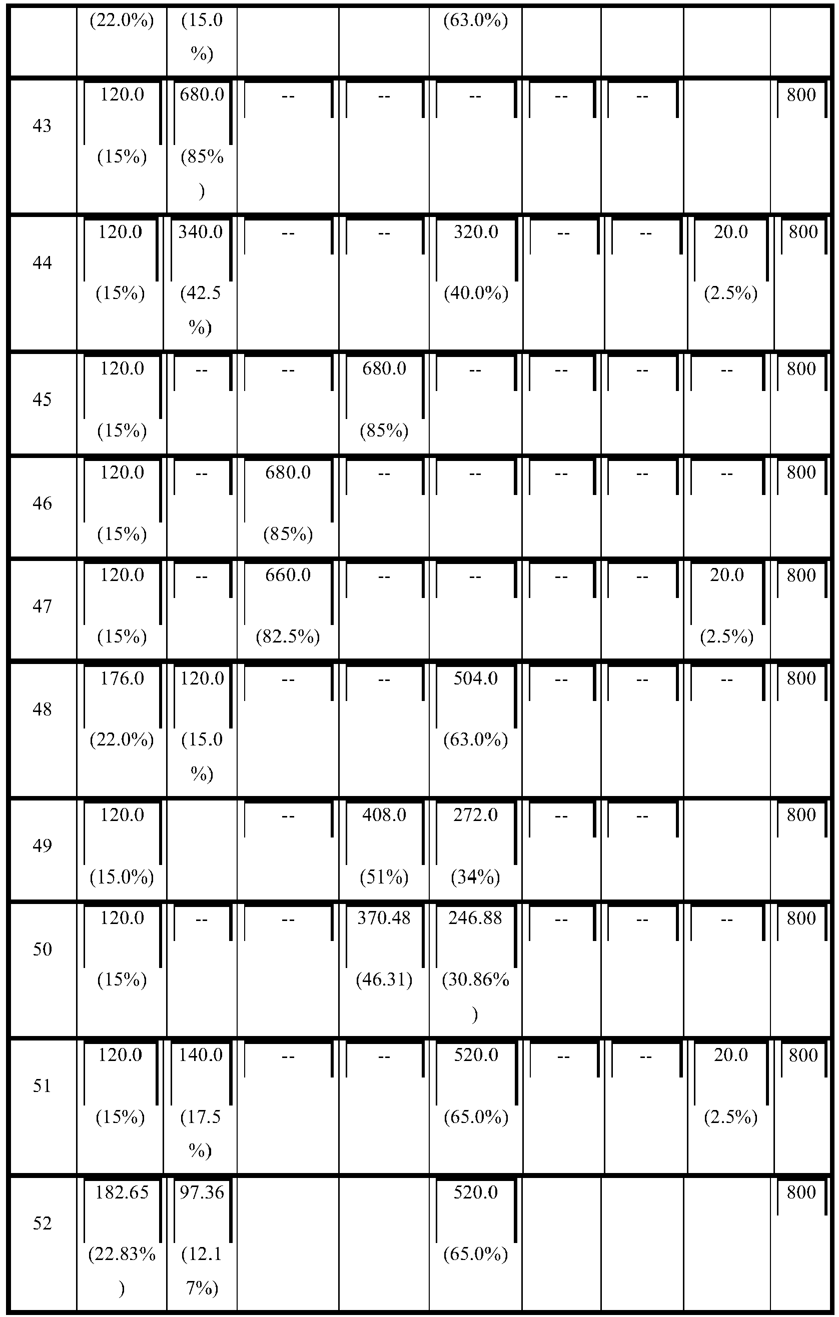

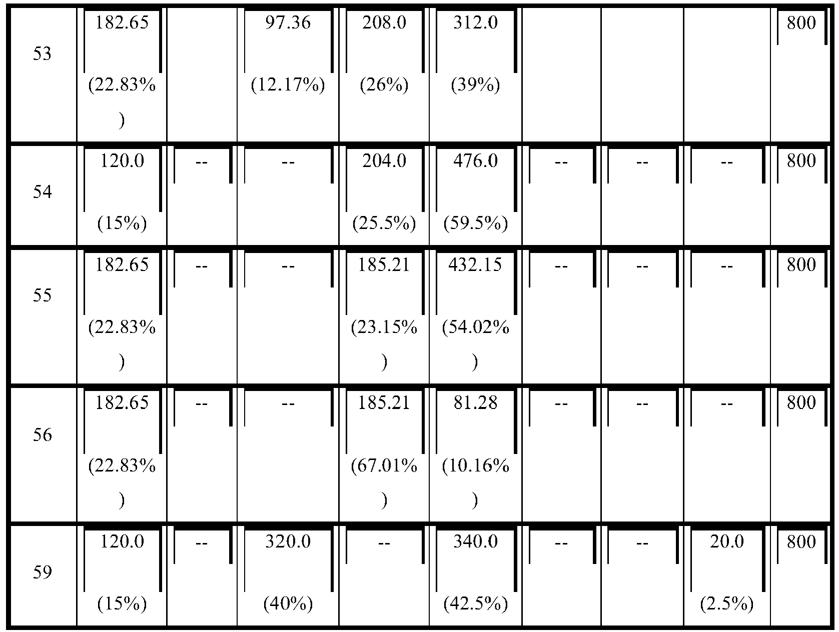

Formulation numbers 50, 51 and 54 are preferred embodiments. As well, while a variety of solvents may be useful in the formulations presented in Table 3, preferred solvents may have the following characteristics: C₄-C₂₄ fatty acids and/or their glycerol-, propylene glycol-, polyethylene glycol, sorbitan- mono-/diesters alone and in mixtures. Preferred fatty acids and esters are C₈-C₁₈, saturated and unsaturated. In addition, the solvents include, fatty acid esters with lower alcohols, such as ethyl oleate, ethyl linoleate, isopropyl myristate, isopropylpalmitate, isopropyloleate and isopropyllinoleate.

### Example

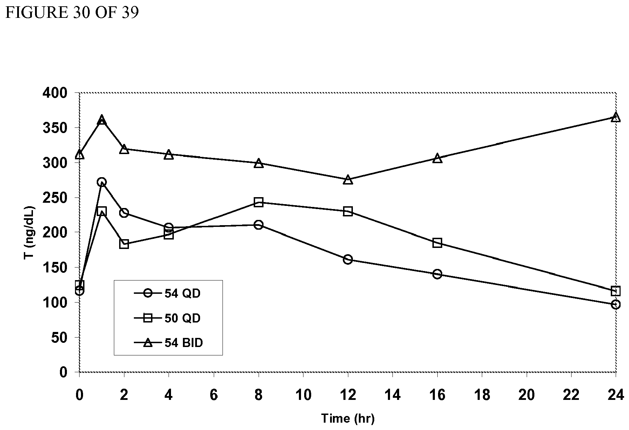

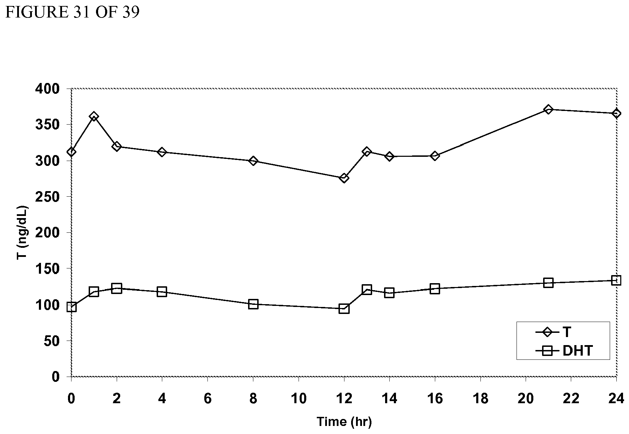

Formulations 50 and 54 were administered to 6 patients; number 50 was administered once-daily ("QD") in the form of two capsules per dose (100 mg T equivalents/capsule) and number 54 was administered once- and twice-daily ("BID") in the form of three capsules per dose (66 mg T equivalents/capsule). The mean steady-state profiles after 7 days of treatment with one of the three, respective, regimens are shown in Figure 30. The pharmacokinetic profile for formulation 54 BID was relatively uniform over the entire 24 hr period and had a trough of the mean profile about 70% of the peak of the mean profile. Additional data from formulation 54 include:
- Average serum T increase from baseline of 275 ng/dL
- Mean serum T levels at lower end of normal range, i.e., about 325 ng/dL.
- Relatively fast release (Tₘₐₓ of about 1 hour)
- Estimated terminal half-life of T at steady-state of approximately 8-9 hours
- Consistent dose-related elevation in serum T baseline levels over the 7-day treatment period
- Average steady-state serum DHT level of 114 ng/dL (Figure 31)

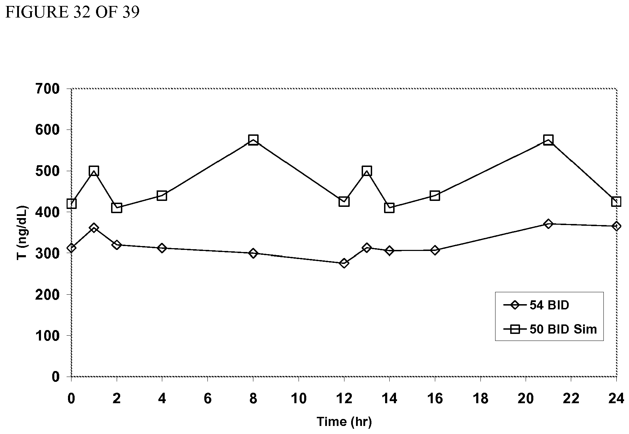

A simulation of the pharmacokinetic profile of formulation 50 administered BID was performed and compared to the observed profile for formulation 54 administered BID. The simulation predicts about a 384 ng/dL increase in C_{avg} over the 24-hour period for formulation 50 over formulation 54 (Figure 32).

In other embodiments of the present invention, methods and compositions for modulating (i.e., sustaining) the rate of available serum testosterone by incorporating component(s) that may biochemically modulate (1) TP absorption, (2) TP metabolism to T, and/or (3) metabolism of T to DHT. For example, the inclusion of medium to long chain fatty acid esters can enhance TP absorption. Without being held to or bound by theory, the present inventors believe that the use of effective amounts fatty acid esters, particularly palmitate esters such as ascorbyl-palmitate, retinyl-palmitate, sorbitan-palmitate and blends thereof may establish competition between said ester and TP for endogenous esterase activity. Indeed, it is believed that testosterone ester metabolism, generally, may be retarded with the administration of an effective amount of an ester of a medium or long chain fatty acid (e.g., esters of oleic acid, linoleic acid, linolenic acid, stearic acid, myristic acid, lauric acid, palmitic acid, capric or decanoic acid octanoic or caprylic acid, pelargonic acid, undecanoic acid, tridecanoic acid, pentadecanoic acid, and the branched chain, cyclic analogues of these acids). In this way, more TP may stave off hydrolysis in the gut and enter the blood stream. In other words, the fatty acid ester may competitively inhibit esterases that would otherwise metabolize TP. Table 4 provides effective amounts of inhibitors of testosterone ester metabolism. Examples of other esters or combinations thereof include botanical extracts or benign esters used as food additives (e.g., propylparben, octylacetate, and ethylacetate).

Other components that can modulate TP absorption include "natural" and synthetic inhibitors of 5α-reductase, which is present in enterocytes and catalyze the conversion of T to DHT. Complete or partial inhibition of this conversion may both increase and sustain increases serum levels of T after oral dosing with TP while concomitantly reducing serum DHT levels. Borage oil, which contains a significant amount of the 5α-reductase inhibitor gamma-linoleic acid (GLA), is an example of a "natural" modulator of TP metabolism. Other than within borage oil, of course, GLA could be directly added as a separate component of TP formulations described herein. Many natural inhibitors of 5α-reductase are known in the art (e.g., epigallocatechin gallate, a catechin derived primarily from green tea and saw palmetto extract from berries of the Serenoa repens species), all of which may be suitable in the present invention. Non-limiting examples of synthetic 5α-reductase inhibitors suitable in the present invention include finasteride and dutasteride.

In addition to 5α-reductase inhibitors, the present invention contemplates the use of inhibitors of T metabolism via other mechanisms. One such point of inhibition may be the cytochrome P450 isozyme CYP3A4 that is present in enterocytes and in liver cells and thus capable of metabolizing testosterone. Accordingly, formulations of the present invention, in some embodiments, include peppermint oil, which is known to contain factors capable of inhibiting CYP3A4.

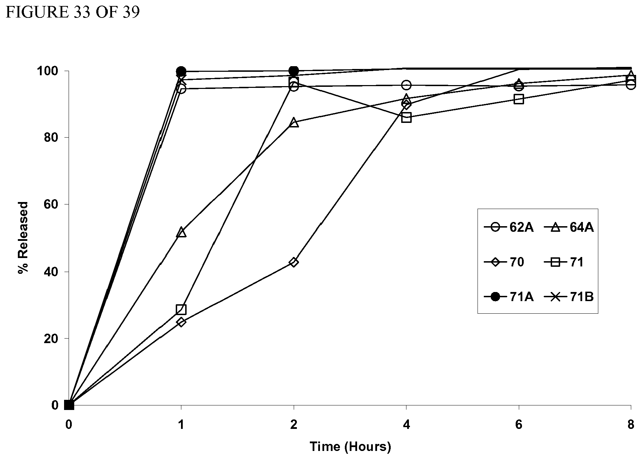

Table 18 provides composition details of various TP formulations comprising ingredients to modulate TP absorption (i.e., ascorbyl-palmitate, borage oil and peppermint oil). Figures 32and 33show representative in vitro dissolution profiles for select TP formulations therein in either phosphate buffer (PBS) or fed-state simulated intestinal fluid (FeSSIF), respectively.

**Table 18**

| F. No. | Composition % w/w (mg/ "00" capsule)¹ | | | | | | | | Fill Wt. (mg)² |
|---|---|---|---|---|---|---|---|---|---|
| | TP | Ascorbyl-Palmitate | Cremophor RH40 | Cremophor EL | Oleic Acid | Peceol | Borage Oil | Peppermint Oil | |
| 62 | 30.0 (240) | 2.5 (20) | - | - | 67.5 (540) | - | - | - | 800 |
| 62A | 15.0 (120) | 2.5 (20) | - | - | 82.5 (660) | - | - | - | 800 |
| 63 | 30.0 (240) | 5.0 (40) | - | - | 65.0 (520) | - | - | - | 800 |
| 63A | 22.9 (183) | 5.0 (40) | 12.2 (97) | - | 60.0 (480) | - | - | - | 800 |
| 64 | 15.0 (120) | 15.0 (120) | - | - | 70.0 (560) | - | - | - | 800 |
| 64A | 15.0 (120) | 10.0 (80) | 25.0 (200) | - | 50.0 (400) | - | - | - | 800 |
| 65 | 22.9 (183) | - | 25.0 (200) | - | 52.0 (417) | - | - | - | 800 |
| 66 | 15.0 (120) | - | 42.5 (340) | - | - | 42.5 (340) | - | - | 800 |
| 67 | 15.0 (120) | - | 30.0 (240) | - | - | 55.0 (440) | - | - | 800 |
| 68 | 22.9 (183) | - | 20.0 (160) | - | 45.0 (360) | 12.0 (96) | - | - | 800 |
| 69 | 22.9 (183) | - | - | - | 53.0 (424) | 19.0 (152) | - | - | 800 |
| 70 | 22.9 (183) | 10.0 (80) | 25.0 (200) | - | 22.1 (177) | - | 10.0 (80) | 10.0 (80) | 800 |
| 70B | 22.9 (183) | 2.5 (20) | 20.0 (160) | - | 39.7 (318) | - | 10.0 (80) | 5.0 (40) | 800 |
| 71 | 15.0 (120) | 10.0 (80) | 25.0 (200) | - | 30.0 (240) | - | 10.0 (80) | 10.0 (80) | 800 |
| 71A | 10.0 (80) | 2.5 (20) | 20.0 (160) | - | 52.5 (420) | - | 10.0 (80) | 5.0 (40) | 800 |
| 71B | 15.0 (120) | 2.5 (20) | 20.0 (160) | - | 47.5 (380) | - | 10.0 (80) | 5.0 (40) | 800 |
| 72 | 15.0 (120) | - | 60.0 (480) | - | 25.0 (200) | - | - | - | 800 |
| 73 | 15.0 (120) | - | - | 60.0 (480) | 25.0 (200) | - | - | - | 800 |

| | | | | | | | | | |
|---|---|---|---|---|---|---|---|---|---|
| ¹ Milligram weights rounded to nearest whole number ² ± 1 mg | | | | | | | | | |

In yet another embodiment of the present invention, drug delivery systems disclosed herein may also be suitable for ameliorating some of the side-effects of certain strategies for male contraception. For example, progestin-based male contraception substantially suppresses luteinizing hormone (LH) and follicle-stimulating hormone (FSH), and thereby suppresses spermatogenesis, resulting in clinical azoospermia (defined as less than about 1 million sperm/ml semen for 2 consecutive months). However, administration of progestins also has the undesirable side-effect of significantly reducing steady-state serum testosterone levels.

In such situations, for example, it may be preferable to provide preparations of progestin concomitantly with testosterone or a testosterone derivative (e.g., TP). More preferably, a pharmaceutical preparation according to the invention is provided, comprising progestin-in an amount sufficient to suppress LH and FSH production-in combination with testosterone. In some embodiments, the pharmaceutical preparation is for once-daily, oral delivery.

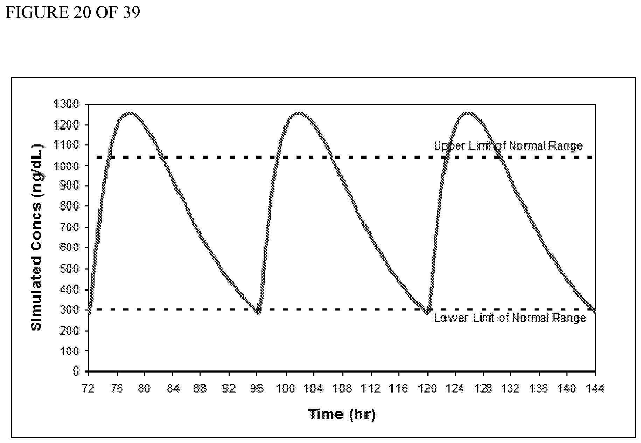

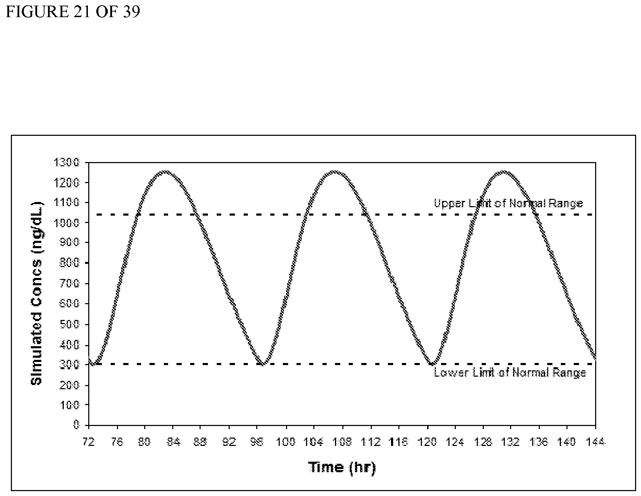

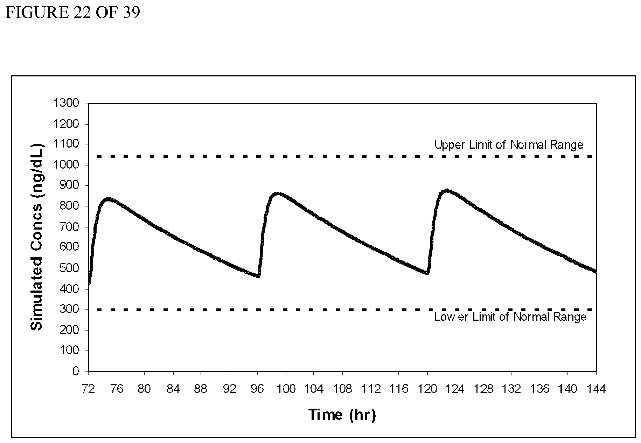

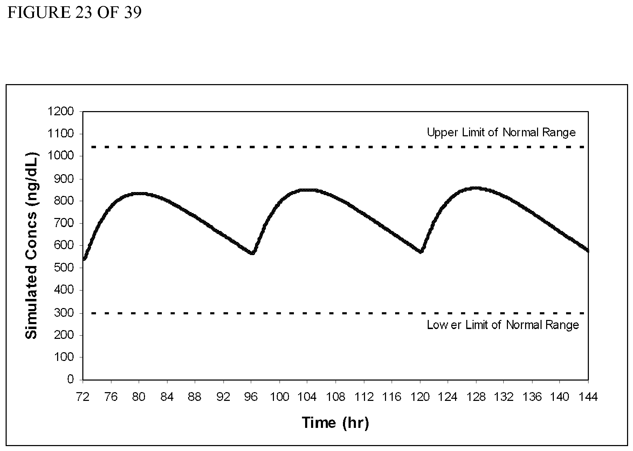

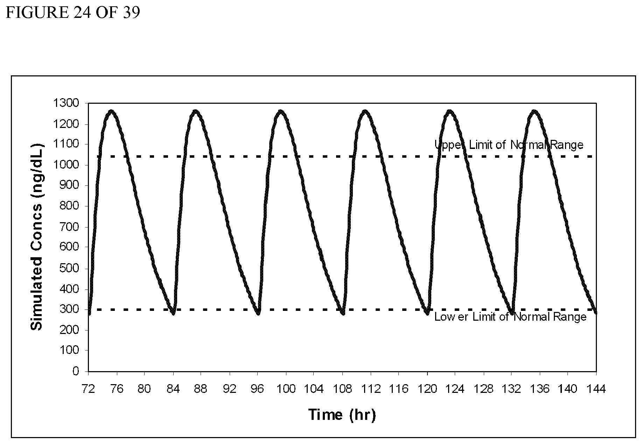

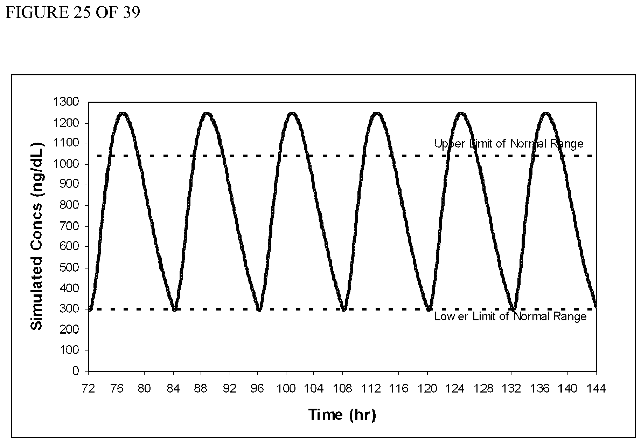

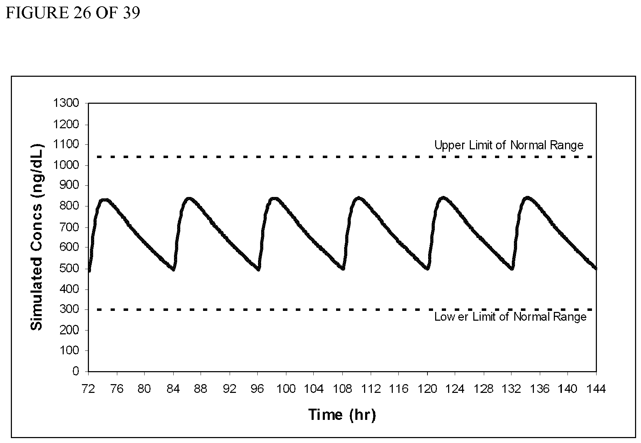

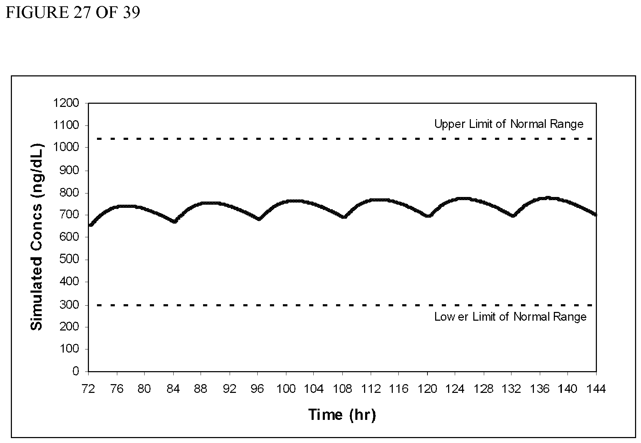

Drug delivery systems, in one aspect of the present invention, afford the flexibility to achieve desirable pharmacokinetic profiles. Specifically, the formulations can be tailored to deliver medicament in a relatively early peak serum concentration (Tₘₐₓ) or one that appears later. See Figures 20, 22, 24and 26versus Figures 21, 23, 25and 27, respectively. Similarly, the formulations may be tailored to have a relative steep or wide drop in drug serum concentration upon obtaining Tₘₐₓ. See Figures 20, 22, 24and 26versus Figures 21, 23, 25and 27, respectively. Accordingly, pharmaceutical preparations of the instant invention may be administered once-daily, twice-daily, or in multiple doses per day, depending on, for example, patient preference and convenience.

One way in which the formulations may be modified to affect these changes is to calibrate the ratio of lipophilic surfactants. The magnitude and timing of the Tₘₐₓ, for example, can be affected by not only the type of lipids used, but also the ratios thereof. For example, to obtain a relatively early Tₘₐₓ, or fast release of the medicament from the delivery system, the concentration of the "controlled-release" lipophilic surfactant (e.g., Precirol) may be reduced relative to the concentration of the other lipophilic solvents (e.g., Labrafil M1944CS). On the other hand, to achieve a delayed Tₘₐₓ, the percentage of "controlled-release" lipophilic surfactant in composition can be increased. Figures 29and 30show in vitro dissolution curves of TP from three formulations, respectively, in a phosphate buffered dissolution medium incorporatingTritonX-100 as a surfactant in accordance with the present invention.

Without being bound by or limited to theory, it is believed that the inventive formulations described herein, in one aspect, enhance absorption of a medicament therein by the intestinal lymphatic system. In this way, drug delivery systems of the present invention can provide extended release formulations that can deliver testosterone into the serum over several hours. The serum half-life of testosterone in men is considered to be in the range of 10 to 100 minutes, with the upper range for testosterone administered in a form (i.e., TU) that favors lymphatic absorption. However, oral dosages of the present invention can be taken by a patient in need of testosterone therapy once every about twelve hours to maintain desirable levels of serum testosterone. In a more preferred embodiment, oral dosages are taken by a patient in need of testosterone therapy once every about twenty-four hours. In general, "desirable" testosterone levels are those levels found in a human subject characterized as not having testosterone deficiency.

### Examples - LOTUS 2

Specific embodiments of the instant invention will now be described in non-limiting examples. Table 2 provides composition details of various formulations of TU, in accordance with the teachings of the instant invention. For calculation purposes, 1 mg of T is equivalent to 1.58 mg T-undecanoate.

The compositions details of Table 19 (mg/capsule and wt. percentage) are based on an approximate fill weight of 800 mg fill weight per '00' hard gelatin capsule. However, at testosterone-ester amounts less than about 100 mg/capsule, the formulations may be proportionally adjusted for smaller total fill weights that would permit use of smaller hard gelatin capsules (e.g., size '0' or smaller size if needed).

As well, it should be apparent to one of ordinary skill in the art that many, if not all, of the surfactants within a category (e.g., lipophilic, hydrophilic, etc.) may be exchanged with another surfactant from the same category. Thus, while Table 1 lists formulations comprising oleic acid, one of ordinary skill in the art should recognize other lipophilic surfactants (e.g., those listed above) may be suitable as well. Similarly, while Table 1 lists formulations comprising Cremophor RH40 (HLB = 13), one of ordinary skill in the art should recognize other hydrophilic surfactants (e.g., those listed above) may be suitable. Borage oil, peppermint oil, BHT, and ascorbyl palmitate may be substituted for chemically similar substances or eliminated.

**Table 19**

| F. | Composition % w/w (mg/ "00" capsule)¹ | | | | | | | Fill Wt. (mg) ² |
|---|---|---|---|---|---|---|---|---|
| | TU | Oleic Acid | Cremophor RH40 | Borage Oil | Peppermint Oil | BHT | Ascorbyl Palmitate | |
| 1 | 20 (158) | 51.5 (413) | 16 (128.5) | 10 (80) | 2.5 (20) | 0.06 (0.5) | - | 800 |
| 2 | 15 (120) | 54.5 (436) | 18 (144) | 10 (80) | 2.5 (20) | 0.02 (0.2) | 0.8 (6.4) | 806. 6 |
| 3 | 17 (136) | 52.5 (420) | 18 (144) | 10 (80) | 2.5 (20) | 0.02 (0.2) | 0.8 (6.4) | 806. 6 |
| 4 | 19 (152) | 50.5 (404) | 18 (144) | 10 (80) | 2.5 (20) | 0.02 (0.2) | 0.8 (6.4) | 806. 6 |
| 5 | 21 (168) | 50 (400) | 16.5 (132) | 10 (80) | 2.5 (20) | 0.02 (0.2) | 0.8 (6.4) | 806. 6 |
| 6 | 23 (184) | 50 (400) | 14.5 (116) | 10 (80) | 2.5 (20) | 0.02 (0.2) | 0.8 (6.4) | 806. 6 |
| 7 | 25 (200) | 50 (400) | 12.5 (100) | 10 (80) | 2.5 (20) | 0.02 (0.2) | 0.8 (6.4) | 806. 6 |
| 8 | 16 (128) | 53.5 (428) | 18 (144) | 10 (80) | 2.5 (20) | 0.02 (0.2) | 0.8 (6.4) | 806. 6 |
| 9 | 18 (144) | 51.5 (413) | 18 (144) | 10 (80) | 2.5 (20) | 0.02 (0.2) | 0.8 (6.4) | 806. 6 |
| 10 | 22 (176) | 50 (400) | 15.5 (124 | 10 (80) | 2.5 (20) | 0.02 (0.2) | 0.8 (6.4) | 806. 6 |
| 11 | 24 (192) | 50 (400) | 13.5 (108) | 10 (80) | 2.5 (20) | 0.02 (0.2) | 0.8 (6.4) | 806. 6 |
| 12 | 15 (120) | 55.5 (444) | 17 (136) | 10 (80) | 2.5 (20) | 0.02 (0.2) | 0.8 (6.4) | 806. 6 |
| 13 | 17 (136) | 53.5 (428) | 17 (136) | 10 (80) | 2.5 (20) | 0.02 (0.2) | 0.8 (6.4) | 806. 6 |
| 14 | 19 (152) | 51.5 (412) | 17 (136) | 10 (80) | 2.5 (20) | 0.02 (0.2) | 0.8 (6.4) | 806. 6 |
| 15 | 15 (120) | 56.5 (452) | 16 (128) | 10 (80) | 2.5 (20) | 0.02 (0.2) | 0.8 (6.4) | 806. 6 |
| 16 | 17 (136) | 54.5 (436) | 16 (128) | 10 (80) | 2.5 (20) | 0.02 (0.2) | 0.8 (6.4) | 806. 6 |
| 17 | 19 (152) | 52.5 (420) | 16 (128) | 10 (80) | 2.5 (20) | 0.02 (0.2) | 0.8 (6.4) | 806. 6 |
| 18 | 21 (168) | 50.5 (404) | 16 (128) | 10 (80) | 2.5 (20) | 0.02 (0.2) | 0.8 (6.4) | 806. 6 |
| 19 | 20 (160) | 50.5 (404) | 17 (136) | 10 (80) | 2.5 (20) | 0.02 (0.2) | 0.8 (6.4) | 806. 6 |
| 20 | 20 (160) | 51.5 (412) | 16 (128) | 10 (80) | 2.5 (20) | 0.02 (0.2) | 0.8 (6.4) | 806. 6 |
| 21 | 15 (120) | 57.5 (460) | 15 (120) | 10 (80) | 2.5 (20) | 0.02 (0.2) | 0.8 (6.4) | 806. 6 |
| 22 | 16 (128) | 56.5 (452) | 15 (120) | 10 (80) | 2.5 (20) | 0.02 (0.2) | 0.8 (6.4) | 806. 6 |
| 23 | 17 (136) | 55.5 (444) | 15 (120) | 10 (80) | 2.5 (20) | 0.02 (0.2) | 0.8 (6.4) | 806. 6 |
| 24 | 18 (144) | (54.5 (436) | 15 (120) | 10 (80) | 2.5 (20) | 0.02 (0.2) | 0.8 (6.4) | 806. 6 |
| 25 | 19 (152) | 53.5 (428) | 15 (120) | 10 (80) | 2.5 (20) | 0.02 (0.2) | 0.8 (6.4) | 806. 6 |
| 26 | 20 (158) | 51.5 (413) | 16 (128.5) | 9.4 (75) | 3.1 (25) | 0.06 (0.5) | -- | 800 |
| | | | | | | | -- | |
| 27 | 20 (158) | 51.5 (413) | 16 (128.5) | 10.6 (85) | 1.9 (15) | 0.06 (0.5) | -- | 800 |
| | | | | | | | -- | |
| 28 | 20 (158) | 51.5 (413) | 16 (128.5) | 11.2 (90) | 1.2 (10) | 0.02 (0.2) | 0.8 (6.4) | 806. 1 |
| 29 | 20 (158) | 51.5 (413) | 16 (128.5 | 11.8 (95) | 0.6 (5) | 0.02 (0.2) | 0.8 (6.4) | 806. 1 |
| 30 | 25 (200) | 50 (400) | 12.5 (100) | 10.6 (85) | 1.9 (15) | 0.06 (0.5) | -- | 800. 5 |
| | | | | | | | -- | |

| | | | | | | | | |
|---|---|---|---|---|---|---|---|---|
| ¹ Milligram weights rounded to nearest whole number; 800 (±10%) ² ± 8 mg | | | | | | | | |

Preferred formulations of TU filled into size "00" capsules in accordance with the present invention are:
Formulation A

| Ingredients | mg/capsule | %, w/w |
|---|---|---|
| Testosterone Undecanoate | 158.3 | 19.8 |
| Oleic Acid | 413.1 | 51.6 |
| Cremophor RH 40 | 128.4 | 16.1 |
| Borage Seed Oil | 80.0 | 10 |
| Peppermint Oil | 20.0 | 2.5 |
| BHT | 0.2 | 0.03 |
| Total | 800 | 100 |

Formulation B

| Ingredients | mg/capsule | %, w/w |
|---|---|---|
| Testosterone Undecanoate | 158.3 | 19.8 |
| Oleic Acid | 412.5 | 51.6 |
| Cremophor RH 40 | 128.4 | 16.0 |
| Peppermint Oil | 20.0 | 2.5 |
| Borage Seed Oil + 0.03% BHT | 80.0 | 10 |
| Ascorbyl Palmitate | 0.8 | 0.1 |
| Total | 800 | 100 |

Formulation C

| **Ingredients** | **mg/capsule** | **%, w/w** |
|---|---|---|
| Testosterone Undecanoate | 120 | 15 |
| Cremophor RH 40 | 128 | 16 |
| Maisine 35-1 | 504 | 63 |
| Polyethylene Glycol 8000 | 48 | 6 |
| TOTAL | 800 | 100 |

In vivo and in vitro performance data of the formulations in keeping with the invention will next be described. However, the scope of the invention should not be limited to the following examples nor the specific formulations studied in the examples.

### Example 1 - Single-Day study

Formulation B was studied for its single-day pharmacokinetic profile upon once- or twice-daily administration to hypogonadal men. The study was designed as an open-label, single-day dosing, sequential, cross-over, pharmacokinetic study. Twelve (12) hypogonadal men were enrolled after giving written informed consent, and all 12 subjects completed the study. Each subject received a daily dose of Formulation B as follows:
1. 200 mg T (as TU) QD, i.e., 2 capsules/dose
2. 200 mg T (as TU) BID (100 mg/dose), i.e., 1 capsule/dose
3. 400 mg T (as TU) BID (200 mg/dose)

The doses were administered as capsules to subjects five minutes after a meal (breakfast for QD, and breakfast and dinner for BID).

**Table 20 provides the relevant PK parameters from the study:**

| Table 20. Single-Day Pharmacokinetic Parameters for T, DHT, and DHT:T Ratio | | | |
|---|---|---|---|
| | Means (Standard Deviations) of Pharmacokinetic Parameters^{a} | | |
| Pharmacokinet ic Parameter (unit) | Regimen 1 (TU QD 200 mg^{b}) | Regimen 2 (TU BID 100 mg^{b}) | Regimen 3 (TU BID 200 mg^{b}) |
| T | | | |
| AUC₂₄ | 5907 | 6751 | 9252 |
| (ng•hr/dL) | (1840) | (2145) | (3173) |
| C_{avg} (ng/dL) | 246 (77) | 281 (89) | 385 (132) |
| T_{1/2} (hr)^{a} | 15.5 (7.0-24.0) | 15.1 (4.5-43.4) | 8.0 (4.2-16.3) |
| Cₘₐₓ (ng/dL) | 0-24 hrs: 557 (252) | 0-12 hrs: 470 (247) | 0-12 hrs: 626 (267) |
| | | 12-24 hrs: 466 (160) | 12-24 hrs: 718 (333) |
| Tₘₐₓ (hr)^{a} | 0-24 hrs: 4.0 (2.0-8.0) | 0-12 hrs: 4.0 (2.0-12.0) | 0-12 hrs: 4.0 (2.0-12.0) |
| | | 12-24 hrs: 16.0 (14.0-20.0) | 12-24 hrs: 16.0 (14.0-20.0) |

| DHT | | | |
|---|---|---|---|
| AUC₂₄ (ng•hr/dL) | 1097 (387) | 1400 (758) | 1732 (859) |
| C_{avg} (ng/dL) | 45.7 (16.1) | 58.3 (31.6) | 72.2 (35.8) |
| Cₘₐₓ (ng/dL) | 0-24 hrs: 122 (66) | 0-12 hrs: 81.3 (40.3) | 0-12 hrs: 108 (59) |
| | | 12-24 hrs: 97.9 (51.2) | 12-24 hrs: 114 (58) |
| Tₘₐₓ (hr)^{a} | 0-24 hrs: 4.0 (1.0-8.0) | 0-12 hrs: 4.0 (1.0-12.0) | 0-12 hrs: 4.0 (1.0-12.0) |
| | | 12-24 hrs: 16.0 (13.0-20.0) | 12-24 hrs: 16.0 (14.0-20.0) |

| DHT:T Ratio | | | |
|---|---|---|---|
| R_{avg} (ng/dL) | 0.189 (0.070) | 0.233 (0.137) | 0.198 (0.041) |

| | | | |
|---|---|---|---|
| ^{a} Values shown for half-life and time to maximum concentration are median and the range. ^{b} Doses indicated are in T equivalents. Each TU capsule contained 158.3 mg TU, which corresponds to 100 mg T equivalents. | | | |

Mean serum T concentration during the 24-hour period post-dose (C_{avg}) indicated positive increases in serum T levels for all regimens studied, with the best response obtained in Regimen 3 (C_{avg} 385 ng/dL). Mean peak serum T concentration observed in response to the oral T-ester preparations evaluated in this study never exceeded the upper limit of normal (i.e., 1100 ng/dL). Moreover, while some individual subjects did have Cₘₐₓ T values above the normal upper limit, the vast majority of these peaks were in the range of 1200 to 1400 ng/dL. No subject in any treatment arm experienced a Cₘₐₓ in excess of 1500 ng/dL.

Median serum T half-life (T_{1/2}) was approximately 15 hours for Regimens 1 and 2; for Regimen 3, T_{1/2} was 8 hours. In each regimen, serum DHT concentrations increased in concert with serum T levels. The mean DHT:T ratios (R_{avg}) in all periods were modestly above the normal ranges as determined by liquid chromatography-mass spectroscopy (LC/MS/MS) (i.e., 0.03-0.1), but were clinically insignificant.

TU dosed at 200 mg T equivalents, BID with food yielded the most promising results with 75% of the subjects achieving a serum T C_{avg} above 300 ng/dL (lower normal eugonadal limit). Similarly, 75% of the subjects achieved an average serum T within the normal range (i.e., 0.03 - 0.1 ng/dL). Those subjects that did not achieve a C_{avg} of at least 300 ng/dL were all above 200 ng/dL, indicating that a modest increase in the TU dose would have been effective oral T replacement therapy in these subjects.

Serum T and DHT concentrations increased in concert in the majority of subjects regardless of T-ester dose with excellent dose linearity for oral TU was observed when data were corrected for serum T at baseline. Although DHT:T ratios were modestly elevated, any elevation was considered clinically insignificant. Less inter-subject variability was observed with the formulation than equivalent formulations of other T-esters (e.g., TE). Furthermore, in the "BID" dosing regimens, there was no difference in mean peak serum T concentrations or in the 12-hour AUCs between the morning and evening dose.

Concerning safety, although headache was reported as an adverse effect, in each treatment regimen, no adverse event was reported by more than one subject. No serious adverse events or deaths occurred during the study, and no subjects prematurely discontinued the study due to adverse events. Hence, all adverse events were considered to be of mild intensity.

### Example 2 - Seven-Day study

Formulation B was studied for its acute tolerability and steady-state serum pharmacokinetic profile at two doses administered twice-daily to hypogonadal men. The study was designed as an open-label, repeat dose, cross-over, pharmacokinetic study (with food effect examined in one arm).

Twenty nine (29) hypogonadal men were enrolled after giving written informed consent, 24 of which completed the study. Each subject who completed the study received a regimen of Formulation B as follows:
1. 7 daily doses of 600 mg T as TU BID (300 mg/dose), i.e., 3 capsules/dose
2. 8 daily doses of 400 mg T as TU BID (200 mg/dose)

Doses were administered as capsules to subjects 30 minutes after initiation of meals (breakfast and dinner), except for Day 8, when the morning dose was administered fasting.

Peak exposure (Cₘₐₓ) to T and total exposure (AUC) to T were dose proportional after correction for the endogenous baseline T. The time of peak T concentrations (Tₘₐₓ) occurred at approximately 4 hours post-dose with each of the treatments. As well, the serum concentrations of both TU and DHTU rise and fall within the dosage interval with concentrations at the beginning and end of the dosing interval being less than 20% of the peak concentration for TU and less than 25% of the peak concentration for DHTU. Baseline T concentrations due to endogenous T production decreased progressively for each treatment. The observation is consistent with a progressive and persistent suppression of gonadotropins by exogenous T, thereby resulting in a decreased production of endogenous T. At least partial suppression was maintained over a 14-day washout period.

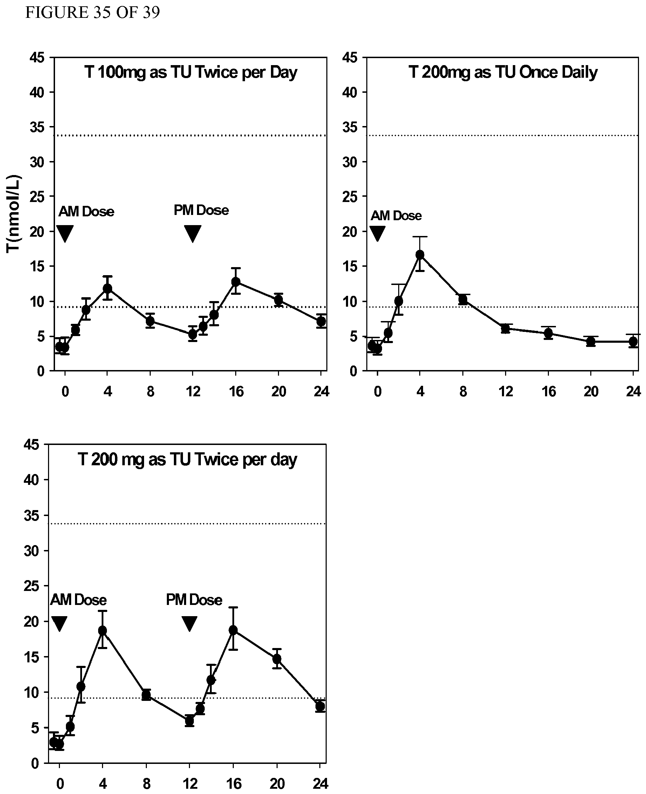

Again, serum T pharmacokinetics did not show diurnal variation with serum T concentrations. The night dose (administered at approximately 8 PM) produced a similar concentration-time profile as the morning dose (administered at approximately 8 AM) (Figure 35). On account of the similarity between concentrations after AM and PM dosing (assessed in Regimen 1), 12-hour PK data from Regimen 2 (fed) were used to accurately predict a full 24-hour PK profile in response to 200 mg T (as TU), BID dosing. The simulated results indicated that (a) 77% of the subjects achieved a serum T C_{avg} in the eugonadal range over the 24-hour period based on AUC thereby meeting the current FDA efficacy requirement of 75% for a T-replacement product; and (b) none of the subjects experienced a Cₘₐₓ in excess of 1500 ng/dL, which is exceeds current FDA criteria that less than 85% of subjects have a Cₘₐₓ of greater than 1500 ng/dL for a T-replacement product. Hence, also consistent with current FDA mandated efficacy endpoints, no subjects had a Cₘₐₓ in excess of 2500 ng/dL and less than 5% of the subjects studied had a Cₘₐₓ in the range of 1800 - 2500 ng/dL. It is noteworthy that these results were achieved in the absence of any dose adjustment.

**Table 21 provides a comparison of steady state AM and PM pharmacokinetics of T with BID Dosing:**

| Table 21. | Treatment Regimen 1 | | | |
|---|---|---|---|---|
| | 300 mg T, as TU, BID | | | |
| | AM Dose | | PM Dose | |
| | Mean | ± SEM | Mean | ± SEM |
| Cₘₐₓ (ng/dL) | 1410 | ± 146 | 1441 | ± 118 |
| Tₘₐₓ (hr, time after dose) | 4.50 | ± 0.39 | 5.9 | ± 0.5 |
| Cₘᵢₙ (ng/dL) | 305 | ± 30 | 324 | ± 36 |
| AUC₀₋₁₂ (ng•hr/dL) | 9179 | ± 754 | 9830 | ± 659 |
| C_{avg} (ng/dL) | 765 | ± 63 | 819 | ± 55 |
| FI ratio | 1.37 | ± 0.09 | 1.36 | ± 0.09 |
| Cₘᵢₙ/Cₘₐₓ ratio | 0.256 | ± 0.029 | 0.243 | ± 0.022 |

Administration of TU with a high-fat meal produced a similar serum T-concentration-time profile as administration with a standard meal. In contrast, administration of TU under fasting conditions resulted in greater than 50% decrease in serum T exposures (Cₘₐₓ and AUC). Table 22.In all cases, a strong correlation between the observed Cₘₐₓ and the calculated C_{avg} was observed, suggesting that targeting of a particular C_{avg} with the oral T-ester formulation can result in predictable peak T levels after dosing.

| **Table 22.** | **After High Fat Breakfast** | | **While Fasting** | | **Geometric Mean of Individual Ratios** |
|---|---|---|---|---|---|
| | Arithmetic Mean | Geometric Mean | Arithmetic Mean | Geometric Mean | |
| Cₘₐₓ (ng/dL) | 955 | 854 | 394 | 365 | 0.426 |
| AUC₀₋₁₂ (ng•hr.dL) | 6217 | 5682 | 2894 | 2692 | 0.471 |
| Administration under fed conditions (high fat breakfast) was used as the reference | | | | | |

DHT concentrations tracked T concentrations, although DHT concentrations were only 11-34% of the T concentrations. Conversion of T to DHT showed a slight nonlinearity, increasing at a less than a concentration-proportional rate compared to T. The DHT/T ratio was least when T concentrations were highest, and the DHT/T ratio prior to starting TU treatment was approximately 0.1, while during treatment, at steady-state, the mean ratio was 0.24 and ranged from approximately 0.1 to 0.35 depending on the time of sampling after oral TU was administered.

Mean estradiol concentration prior to starting the oral TU treatment was approximately 11 pg/mL, and ranged from 19 pg/mL to 33 pg/mL on Day 7 of the various treatments (pre-dose concentrations). Pre-dose steady-state estradiol concentrations were approximately 20-30 pg/mL.

### Example 3 - Four-Week study

Formulation B was also studied was to determine the time required to reach steady-state when hypogonadal men are treated for 28 days with twice daily dosing of 200 mg T (as TU) (i.e., 2 capsules/dose). The study was designed as an open-label, repeat dose, pharmacokinetic study.

Fifteen (15) hypogonadal men were enrolled after giving written informed consent, and all completed the study. Each subject received twice-daily doses of 200 mg T as TU for 28 days.

For each subject, the "Day 28" serial PK sampling day was scheduled for Day 32 of the study. Therefore, each dose-compliant subject received 31 daily doses of 400 mg T as TU (i.e., 200 mg T, BID), and a final morning dose of 200 mg T as TU. Doses were administered as capsules, with subjects instructed to take doses 30 minutes after initiation of meals (breakfast and dinner).

**Table 23 provides the relevant PK data from the study:**

| Table 6.^{a} | T | DHT | DHT/T | E₂ |
|---|---|---|---|---|
| Cₘₐₓ or Rₘₐₓ^{b} | 995 ±436 (43.9%) ng/dL | 151 ±75 (49.5%) ng/dL | 0.380 ±0.181 (47.7%) ratio | 30.6 ±14.9 (48.7%) pg/mL |
| Tₘₐₓ | 4.87 ±1.96 (40.3%) hr | 5.87 ±2.80 (47.7%) hr | 5.87 ±6.02 (102.7%) hr | 6.67 ±3.09 (46.3%) hr |
| Cₘᵢₙ or Rₘᵢₙ^{b} | 199 ±108 (54.2%) ng/dL | 64.6 ±47.6 (73.8%) ng/dL | 0.131 ±0.047 (36.0%) ratio | 15.4 ±9.2 (59.9%) pg/mL |
| C_{avg} or R_{avg}^{b} | 516 ±226 (43.7%) ng/dL | 109 ±61 (55.8%) ng/dL | 0.245 ±0.077 (31.5%) ratio | 22.0 ±10.9 (49.8%) pg/mL |
| AUC₀₋₁₂ | 6197 ±2708 (43.7%) ng•hr/dL | 1312 ±732 (55.8%) ng•hr/dL | 2.94 ±0.93 (31.5%) hr | 264 ±131 (49.8%) pg•hr/mL |
| Cₘᵢₙ/Cₘₐₓ or Rₘᵢₙ/Rₘₐₓ^{b} | 23.5% ±16.2% (69.0%) % | 41.5% ±17.0% (40.9%) % | 37.3% ±11.5% (30.8%) % | 50.2% ±15.1% (30.0%) % |
| Absolute Change in C_{baseline}^{c} | -168 ±188 (112.2%) ng/dL | 3.50 ±16.80 (480.1%) ng/dL | 0.197 ±0.116 (59.0%) ratio | -0.405 ±5.345 (1320.8%) pg/mL |
| Percent Change in C_{baseline}^{c} | -53.4% ± 79.5% (148.8%) % | 18.8% ±95.0% (506.6%) % | 267% ± 170% (63.8%) % | -1.9% ± 41.5% (2224.6%) % |
| Fluctuation Index | 156% ± 64% (40.8%) % | 84.7% ± 30.6% (36.1%) % | 96.0% ± 29.7% (30.9%) % | 74.5% ± 41.6% (55.9%) % |
| λ_{z} | 0.0726 ± 0.0676 (93.1%) 1/hr | 0.0793 ± 0.0373 (47.1%) 1/hr | NA | 0.0544 ± 0.0176 (32.4%) 1/hr |
| T_{1/2} | 29.0 ±32.7 (112.8%) hr | 10.8 ± 5.8 (53.6%) hr | NA | 14.0 ± 5.3 (37.8%) hr |
| ^{a} Results expressed as mean ± SEM. Co-efficient over variation is expressed as % in parentheses. | | | | |
| ^{b} Rₘₐₓ, Rₘᵢₙ, R_{avg} are the Maximum ratio, the Minimum ratio and the Time Averaged ratio, respectively for the DHT/T ratio (analogous to Cₘₐₓ, Cₘᵢₙ and C_{avg}) | | | | |
| ^{c} Change in Baseline determined as concentration (or ratio) in the final sample of Day 28 - concentration (or ratio) in the pre-treatment sample (Day 0). | | | | |

86.7% of subjects achieved serum T C_{avg} within the normal range, with no subjects having Cₘₐₓ concentrations greater than 1800 ng/dL, and with just 13.3% of subjects having Cₘₐₓ concentrations greater than 1500 ng/dL. (Note: No dosing adjustments were made during the conduct of this study to titrate subjects to be within the targeted efficacy and safety ranges.) The half-life of T in response to TU in the formulation tested was appreciably longer than has been reported for T alone or for TU given orally in prior art formulations. For example, in clinical studies of an oral TU formulation consistent with the invention described herein, an elimination half-life (α phase) of about approximately 5 hours was observed compared to a value estimated to be roughly half that (i.e., 2 to 3 hours) based on published serum T profiles after oral dosing of a prior art formulation of TU. A long elimination (i.e., terminal) half-life of 29 hrs was also observed with the inventive oral TU formulation. Endogenous T production was suppressed, however, by the administration of exogenous T, with only limited suppression occurring for the first 3 days, and requiring 5-7 days of continued treatment for maximal suppression.

Concentrations of T and DHT reached steady state by Day 7 of treatment. Concentrations of T and DHT were greater on Day 3 than on Day 5, indicating that a period was required for the exogenously administered T to suppress endogenous T production thus enabling achievement of steady-state in response to oral TU. Indeed, addition of the exogenous T suppressed endogenous T levels from 276 ng/dL pretreatment to 108 ng/dL after 28 days of supplementary T treatment.

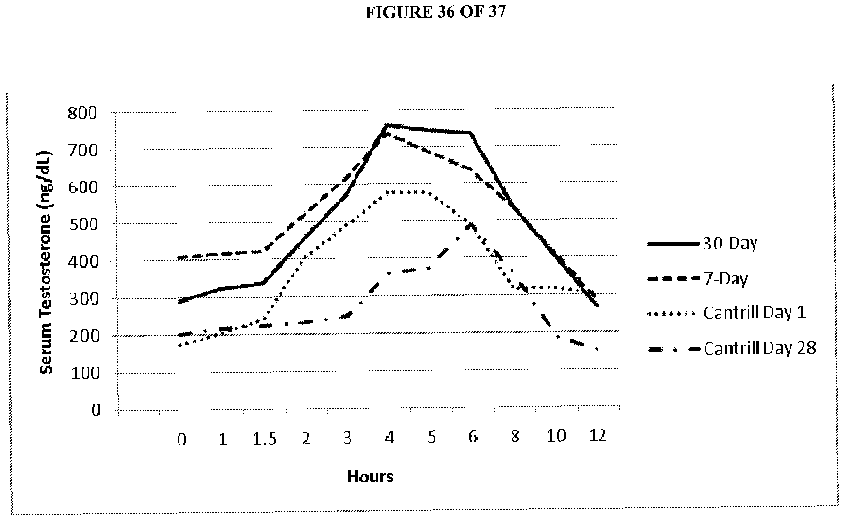

Significantly, however, once steady state was achieved for serum T in response to twice-daily oral TU, little to no decline in serum T response was observed over time (i.e., no trend toward lower serum T level with continued TU dosing). For example, the C_{avg} at Day 15 was substantially similar to the C_{avg} observed at day 28 (Figure 36). By contrast, oral TU formulations in the art have been reported to trend toward a lower mean T over time (Cantrill, J. A. Clinical Endocrinol (1984) 21: 97-107). In hypogonadal men treated with a formulation of oral TU, known in the art, it has been reported that the serum T response observed after 4 weeks of therapy was about 30% less than that observed on the initial day of therapy in hypogonadal men--most of whom had a form of primary hypogonadism and thus low baseline levels of serum T (e.g., <100 ng/dL), so the decrease in T cannot be explained by suppression of endogenous T alone].

Serum DHT concentrations closely tracked T concentrations, with DHT and DHT/T values increasing 4 to 7 fold during treatment. Average DHT/T ratio over a 12-hour dosing interval was 0.245, although values over the dosing interval ranged from a mean maximum ratio of 0.380 to a mean minimum ratio of 0.131. DHT concentrations returned to pretreatment levels within 36 hours of discontinuing treatment with oral TU. However, T concentrations did not return to pretreatment levels as quickly, ostensibly because of the suppression of endogenous T production/release is not as rapidly reversed.

Concentrations of estradiol (E2) showed a monotonic, progressive increase to the steady state, which was also reached by Day 7 of treatment. E2 concentrations also showed systematic variation over the dosing interval that tracked the changes in T. The mean Cₘₐₓ, C_{avg}, and Cₘᵢₙ values for E2 were 30.6 pg/mL, 22.0 pg/mL and 15.5 pg/mL, respectively. E2 concentrations returned to pretreatment levels within 36 hours of discontinuing treatment with oral TU.

Mean Cₘₐₓ, C_{avg}, and Cₘᵢₙ concentrations at steady state (morning dose of Day 28) for T were 995 ng/dL, 516 ng/dL and 199 ng/dL, respectively. Median Tmax for T occurred at 5.0 hours post dose. Cₘᵢₙ averaged 23.5% of Cₘₐₓ, resulting in a Fluctuation Index of 156%. The elimination half-life of T could only be evaluated in about half the subjects, and its median value in those subjects was 18.4 hours (mean T_{1/2} was 29 hours).

### Example 4 - Food Effects study

Any effect of dietary fat on the pharmacokinetics of Formulation B in hypogonadal men was studied in an open-label, two-center, five-way crossover study. After a washout period of 4-10 days, a single dose of 300 mg of T (475mg TU, 3 capsules of Formulation B) was administered to sixteen hypogonadal men with serum a baseline T level 205.5+25.3 ng/dL (mean ± SE, range 23-334.1 ng/dL). Subjects were randomized to receive the drug in the fasting state or 30 minutes after consumption of meals containing ~800 calories with specific amounts of fat (wt %): very low fat (6-10%); low fat (20%); "normal" diet fat (30%); or high fat (50%). The "normal" diet was, a priori, established as the comparator (i.e., reference diet) for purposes of statistical comparisons. Serial blood samples were collected for a total of 24 hours after drug administration to determine serum testosterone and dihydrotestosterone (DHT) levels by liquid chromatography-mass spectroscopy (LC/MS/MS).

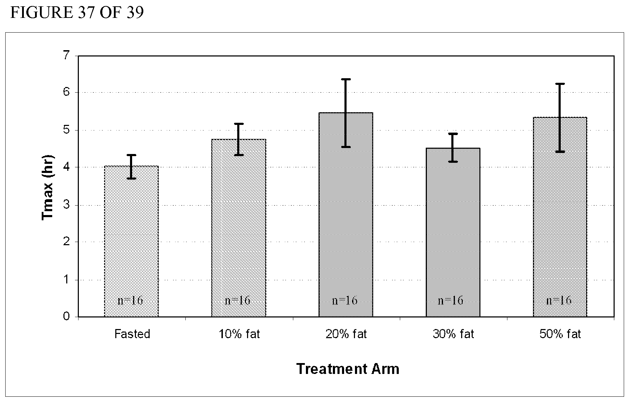

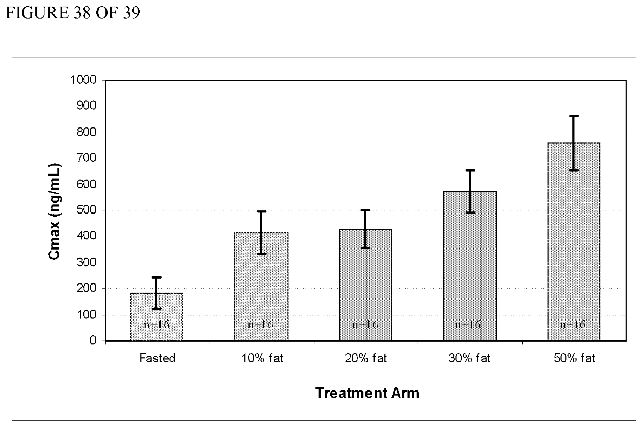

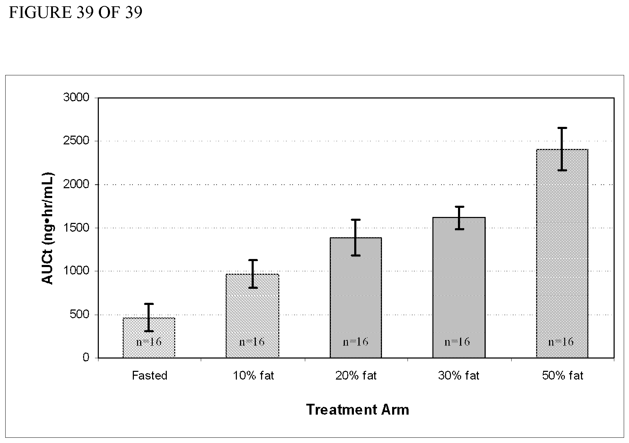

Pharmacokinetic parameters (Table 24, Figures 37-39) observed for serum T in response to a single, high-dose of oral TU were found to be similar for a low-fat and normal fat diet - in fact so much so that they were bioequivalent (i.e., the 90% confidence interval was between 85 - 125%). Similar serum T PK parameters were also observed when the normal- and high-fat meals were compared. And although the high-fat meal yielded a greater serum T response (albeit not statistically different), the mean ratio of least square means fell within 70-143% when compared to the normal-fat meal - a clinically insignificant difference of <30%.

| **Table 7.** | **Serum T pharmacokinetic parameters (mean + SD) in response to oral TU administered with different diets** | | | | |
|---|---|---|---|---|---|
| | Fasting | 6-10% Fat | 20% Fat | 30% Fat | 50% Fat |
| C_{Avg}¹ (ng/dL) | 526±324 | 781±385 | 884±505 | 1010±356 | 1260±477 |
| C_{Max} (ng/dL) | 948±798 | 1370±732 | 1520±711 | 1760±598 | 2140±901 |
| T_{Max} (hr) | 4.1+0.96 | 4.9+1.8 | 6.3+3.9 | 5.1+1.5 | 6.4±4.9 |
| AUC (ng^{∗}h/dL) | 7796±3673 | 10855±428 5 | 12477±502 8 | 13639±377 3 | 16464±5584 |

| | | | | | |
|---|---|---|---|---|---|
| C_{Avg} is calculated as AUC_{0-∞}/τ (τ = dosing interval = 12 hours for BID dosing) | | | | | |

Variability in PK response appeared to be highest following the first dose, or first few doses of oral TU, and decreased as therapy continued. Consequently, any impact of dietary fat across the range of low-normal-high on serum T PK parameters is likely to be insignificant during chronic dosing. This stance is consistent with the PK findings from the 7-day treatment (Example 2) and from the 30-day treatment (Example 3), where repeat dose studies of oral TU where the PK under the differing meal conditions still showed similar results for Cmax and Cavg distributions [both studies administered 200 mg T (as TU), BID].

Statistical comparisons of the serum T response observed after oral TU was taken without food or with a very low fat, low fat, or high fat diet versus a normal fat diet (i.e., reference diet) revealed that there was no statistically significant difference at the p<0.05 level between the low-fat or high-fat diets versus the normal diet. Conversely, administration of oral TU as a SEDDS formulation while fasting or with a very low-fat breakfast yielded serum T PK parameters significantly different (i.e., lower) from a normal diet. Accordingly, the fat content of meals taken with the inventive formulations can differ substantially from "normal", without a clinically significant impact on the levels of T obtained. Thus, a patient is permitted flexibility in eating habits from meal to meal, and from day to day, which could not have been heretofore possible with known oral TU formulations. Oral TU formulations known in the art have heretofore been unable to achieve any meaningful serum T levels in the fasted state.

### Example 5 - In vitro dissolution tests

Dissolution studies of formulations of the present invention were studied in vitro to assess their correlation with the PK profiles observed in vivo. In a first study, the dissolution of Formulation B was studied. Andriol Testocaps^{®} (40 mg TU per softgel dissolved in a mixture of castor oil and propylene glycol laurate) was included for comparison. The study was conducted with essentially equivalent doses of TU, i.e., 1 capsule of Formulation B (158.3 mg TU) and 4 softgels of Testocaps (4x40 mg = 160 mg TU). The dissolution (i.e., the release of TU from the respective formulations) was studied in Fed State Simulated Intestinal Fluid (FeSSIF) medium, which simulates intestinal fluid upon stimulation by a meal. FeSSIF contains sodium hydroxide, glacial acetic acid, potassium chloride, lecithin, and sodium taurocholate. The final emulsion is adjusted to pH 5.0.

That data are presented in Tables 25 and 26 demonstrate that the inventive formulation released approximately 40% TU within the first 30 minutes and about 60% of the total capsule after 4 hours. For the Testocaps^{®}, however, there is little to no drug released (1%) for the entire 4 hours. The observed major difference in the dissolution of TU from these two formulations can be attributed, at least in part, to the presence of the hydrophlic surfactant, e.g.,. Cremophor RH40, in Formulation B. In contrast, Andriol Testocaps^{®} ( incorporate an oil (Castor Oil) and a lipophilic surfactant (Propylene Glycol Laureate) only.

| Table 25. **% Release of TU from Formulation B** | | | | |
|---|---|---|---|---|
| Time (Hours) | % Released | | | |
| | 1 | 2 | 3 | Average |
| 0.5 | 39.3 | 39.2 | 34.6 | 37.7 |
| 1 | 46.2 | 43.6 | 44.3 | 44.7 |
| 2 | 52.8 | 50.9 | 49.8 | 51.2 |
| 4 | 62.7 | 61.7 | 61.3 | 61.9 |
| Infinity | 96.0 | 100.1 | 90.9 | 95.6 |

| Table 26. **% Release of TU from Andriol Testocaps^{®}** | | | | |
|---|---|---|---|---|
| Time (Hours) | % Released | | | |
| | 1 | 2 | 3 | Average |
| 0.5 | 0.0 | 0.0 | 0.0 | 0.0 |
| 1 | 0.0 | 0.0 | 0.0 | 0.0 |
| 2 | 0.0 | 0.9 | 0.0 | 0.3 |
| 4 | 1.3 | 1.1 | 1.3 | 1.3 |
| Infinity | 3.9 | 3.6 | 1.5 | 3.0 |

In a second study, Formulation A was subjected to a similar assay, but using a 5% Triton X100 potassium phosphate buffer (pH 6.8) as a dissolution medium. The results are provided in Table 27 below. In this study, 98% of the TU from the inventive formulation was released within the first 15 minutes of dissolution and once again the presence of the hydrophilic surfactant Cremophor RH40 has certainly facilitated this fast dissolution and TU release.

| Table 10. **% Release of TU from Formulation A** | | | | | | | |
|---|---|---|---|---|---|---|---|
| Time (M) | % Released | | | | | | |
| | 1 | 2 | 3 | 4 | 5 | 6 | Average |
| 0 | 0.0 | 0.0 | 0.0 | 0.0 | 0.0 | 0.0 | 0.0 |
| .25 | 98.9 | 96.9 | 97.7 | 95.7 | 96.6 | 101.0 | 97.8 |
| 0.5 | 98.9 | 97.8 | 98.4 | 98.3 | 97.5 | 100.0 | 98.5 |
| 1.0 | 99.5 | 98.2 | 98.0 | 98.4 | 98.1 | 100.2 | 98.7 |

In yet another embodiment of the present invention, the pharmaceutical compositions disclosed herein may also be suitable for ameliorating some of the side-effects of certain strategies for male contraception. For example, progestin-based male contraception substantially suppresses luteinizing hormone (LH) and follicle-stimulating hormone (FSH), and thereby suppresses spermatogenesis, resulting in clinical azoospermia (defined as less than about 1 million sperm/mL semen for 2 consecutive months). However, administration of progestins also has the undesirable side-effect of significantly reducing steady-state serum testosterone levels.

In such situations, for example, it may be preferable to provide preparations of progestin concomitantly with testosterone or a testosterone derivative (e.g., TU). More preferably, a pharmaceutical preparation according to the invention is provided, comprising progestin-in an amount sufficient to suppress LH and FSH production-in combination with testosterone. In some embodiments, the pharmaceutical preparation is for once-daily, oral delivery.

Formulations of the present invention can provide extended release formulations that can deliver testosterone into the serum over several hours. Indeed, the half-life of serum testosterone according to the invention is between 3 and 7 hours, preferably greater than 4, 5, or 6 hours. The serum half-life of testosterone in men, by contrast, is considered to be in the range of 10 to 100 minutes.

Without being bound by or limited to theory, it is believed that the inventive formulations achieve these results, in one aspect, by enhancing absorption of a medicament therein by the intestinal lymphatic system rather than by way of portal circulation. In another aspect, again without being bound by or limited to theory, it is believed that by using an ester of testosterone, the time required for deesterification to occur contributes to a longer T half-life.

Oral dosages of the present invention can be taken by a patient in need of testosterone therapy once every about twelve hours to maintain desirable levels of serum testosterone. In a more preferred embodiment, oral dosages are taken by a patient in need of testosterone therapy once every about twenty four hours. In general, "desirable" testosterone levels are those levels found in a human subject characterized as not having testosterone deficiency.

### Other Embodiments

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. Any equivalent embodiments are intended to be within the scope of this invention. Indeed, various modifications of the invention in addition to those shown and described herein will become apparent to those skilled in the art from the foregoing description, which do not depart from the spirit or scope of the present inventive discovery. Such modifications are also intended to fall within the scope of the appended claims.

All references cited in this specification are hereby incorporated by reference. The discussion of the references herein is intended merely to summarize the assertions made by their authors and no admission is made that any reference constitutes prior art relevant to patentability. Applicant reserves the right to challenge the accuracy and pertinence of the cited references.

### EMBODIMENTS

1. A method of treating chronic testosterone deficiency in a subject in need thereof comprising the steps of:
   a. administering daily to the subject a dose of an oral pharmaceutical composition comprising a testosterone ester solubilized in a carrier comprising at least one lipophilic surfactant and at least one hydrophilic surfactant;
   b. measuring the serum testosterone concentration in the subject; and
   c. increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL.
2. The method of embodiment 1, wherein the initial amount of testosterone ester in the oral pharmaceutical composition is equivalent to about 200 mg of testosterone.
3. The method of embodiment 2, wherein the amount of testosterone ester in the administered oral pharmaceutical composition is increased by the equivalent of about 25 - 50 mg of testosterone when the serum testosterone concentration in the subject is less than about 250 ng/dL.
4. The method of embodiment 2, wherein the amount of testosterone ester in the administered oral pharmaceutical composition is decreased by the equivalent of about 25 - 50 mg of testosterone when the serum testosterone concentration in the subject is greater than about 700 ng/dL.
5. The method of embodiment 1, wherein the oral pharmaceutical composition comprises testosterone undecanoate.
6. The method of embodiment 5, wherein the initial dose of the oral pharmaceutical composition administered comprises about 317 mg of testosterone undecanoate.
7. The method of embodiment 6, wherein the dose of testosterone undecanoate in the administered oral pharmaceutical composition is increased by about 40 mg to about 80 mg when the measured serum testosterone concentration in the subject is less than about 250 ng/dL.
8. The method of embodiment 6, wherein the dose of testosterone undecanoate in the administered oral pharmaceutical composition is decreased by about 40 mg to about 80 mg when the measured serum testosterone concentration in the subject is greater than about 700 ng/dL.
9. The method of embodiment 1, wherein the oral pharmaceutical composition is administered twice daily.
10. The method of embodiment 1, wherein the serum testosterone concentration is measured two to six hours after administering the oral pharmaceutical composition.
11. The method of embodiment 1, wherein the serum testosterone concentration is measured three to five hours after administering the oral pharmaceutical composition.
12. The method of embodiment 1, wherein the serum testosterone concentration is measured via a radioimmunoassay, an immunometric assay, or a liquid chromatography tandem mass spectrometry (LC-MS/MS) assay.
13. The method of embodiment 12, wherein the serum testosterone concentration is measured via a liquid chromatography tandem mass spectrometry (LC-MS/MS) assay.
14. The method of embodiment 1, wherein the serum testosterone concentration is measured after at least fourteen days of daily treatment with the oral pharmaceutical composition.
15. The method of embodiment 1, wherein the serum testosterone concentration is measured after at least thirty days of daily treatment with the oral pharmaceutical composition.
16. The method of embodiment 1, wherein the oral pharmaceutical composition is administered within 30 minutes of consuming a meal wherein at least about 20 percent of the calories are derived from fat.
17. The method of embodiment 1, wherein steps a.-c. are repeated until the serum testosterone concentration in the subject is between about 250 and about 700 ng/dL.
18. The method of embodiment 1, wherein the oral pharmaceutical composition comprises:
   a. about 10-20 percent by weight of solubilized testosterone ester;
   b. about 5-20 percent by weight of hydrophilic surfactant;
   c. about 50-70 percent by weight of lipophilic surfactant; and
   d. about 10- 15 percent by weight of digestible oil,
   wherein the oral pharmaceutical composition is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.
19. The method of embodiment 18, wherein the testosterone ester is a short-chain (C₂-C₆) or a medium-chain (C₇-C₁₃) fatty acid ester.
20. The method of embodiment 19, wherein the testosterone ester is a medium-chain fatty acid ester selected from the group consisting of testosterone cypionate, testosterone octanoate, testosterone enanthate, testosterone decanoate, and testosterone undecanoate or combinations thereof.
21. The method of embodiment 20, wherein the testosterone ester is testosterone undecanoate.
22. The method of embodiment 18, wherein the hydrophilic surfactant exhibits an HLB of 10 to 45.
23. The method of embodiment 18, wherein the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof.
24. The method of embodiment 23, wherein the hydrophilic surfactant is a hydrogenated castor oil ethoxylate.
25. The method of embodiment 18, wherein the lipophilic surfactant exhibits an HLB of less than 10.
26. The method of embodiment 25, wherein the lipophilic surfactant exhibits an HLB of less than 5.
27. The method of embodiment 26, wherein the lipophilic surfactant exhibits an HLB of 1 to 2.
28. The method of embodiment 18, wherein the lipophilic surfactant is a fatty acid selected from the group consisting of octanoic acid, decanoic acid, undecanoic acid, lauric acid, myristic acid, palmitic acid, palmitoleic, stearic acid, oleic acid, linoleic acid, alpha- and gamma linolenic acid, arachidonic acid, and combinations thereof.
29. The method of embodiment 18, wherein the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil and combinations thereof.
30. The method of embodiment 18, wherein the oral pharmaceutical composition comprises one or more additional lipid-soluble therapeutic agents.
31. The method of embodiment 30, wherein the one or more additional lipid-soluble therapeutic agents are selected from the group consisting of a synthetic progestin, an inhibitor of type-I and/or type II 5 α-reductase, an inhibitor of CYP3A4, finasteride, dutasteride and combinations thereof.
32. The method of embodiment 30, wherein the one or more additional lipid-soluble therapeutic agents comprises a second testosterone ester.
33. The method of embodiment 18, wherein the oral pharmaceutical composition is filled into a hard or soft gelatin capsule.
34. The method of Embodiment 18, wherein the oral pharmaceutical composition is a liquid, semi-solid or solid dosage form.
35. The method of embodiment 18, wherein the oral pharmaceutical composition exhibits a percent (%) in vitro dissolution profile in 5% Triton X-100 solution in phosphate buffer, pH 6.8, indicating release from the composition of substantially all of the solubilized testosterone ester within about 1 hour.
36. The method of embodiment 1, wherein the oral pharmaceutical composition comprises:
   a. about 15-20 percent by weight of solubilized testosterone ester;
   b. about 5-20 percent by weight of hydrophilic surfactant;
   c. about 50-70 percent by weight of lipophilic surfactant; and
   d. about 10- 15 percent by weight of digestible oil,
   wherein the oral pharmaceutical composition is free of ethanol and exhibits a percent (%) in vitro dissolution profile in 5% Triton X-100 solution in phosphate buffer, pH 6.8 that indicates release from the composition of substantially all of the solubilized testosterone ester within about 2 hours.
37. The method of embodiment 36, wherein the testosterone ester is a short-chain (C₂-C₆) or a medium-chain (C₇-C₁₃) fatty acid ester.
38. The method of embodiment 37, wherein the testosterone ester is a medium-chain fatty acid ester selected from the group consisting of testosterone cypionate, testosterone octanoate, testosterone enanthate, testosterone decanoate, and testosterone undecanoate or combinations thereof.
39. The method of embodiment 38, wherein the testosterone ester is testosterone undecanoate.
40. The method of embodiment 36, wherein the hydrophilic surfactant exhibits an HLB of 10 to 45.
41. The method of embodiment 36, wherein the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof.
42. The method of embodiment 41, wherein the hydrophilic surfactant is a polyoxyethylene (40) hydrogenated castor oil.
43. The method of embodiment 36, wherein the lipophilic surfactant exhibits an HLB of less than 10.
44. The method of embodiment 36, wherein the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil and combinations thereof.
45. The method of embodiment 36, wherein the oral pharmaceutical composition comprises one or more additional lipid-soluble therapeutic agents.
46. The method of embodiment 45, wherein the one or more additional lipid-soluble therapeutic agents are selected from the group consisting of a synthetic progestin, an inhibitor of type-I and/or type II 5 α-reductase, an inhibitor of CYP3A4, finasteride, dutasteride and combinations thereof.
47. The method of embodiment 45, wherein the one or more additional lipid-soluble therapeutic agents comprises a second testosterone ester.
48. The method of embodiment 36, wherein the oral pharmaceutical composition is filled into a hard or soft gelatin capsule.
49. The method of embodiment 36, wherein the oral pharmaceutical composition is a liquid, semi-solid or solid dosage form.
50. The method of embodiment 36, wherein the oral pharmaceutical composition exhibits a percent (%) in vitro dissolution profile in 5% Triton X-100 solution in phosphate buffer, pH 6.8, indicating release from the composition of substantially all of the solubilized testosterone ester within about 1 hour.
51. The method of either of embodiments 18 or 36, wherein the pharmaceutical composition is free of monohydric alcohol.
52. The method of embodiment 51, wherein the monohydric alcohol is chosen from C₂-C₁₈ aliphatic or aromatic alcohol.
53. The method of embodiment 52, wherein the monohydric alcohol is chosen from ethanol and benzyl alcohol.
54. The method of embodiment 1, wherein the oral pharmaceutical composition comprises testosterone undecanoate solubilized in a carrier comprising at least one lipophilic surfactant and at least one hydrophilic surfactant in a total lipophilic surfactant to total hydrophilic surfactant ratio (w/w) falling in the range of about 6: 1 to 3.5: 1, which composition, upon once- or twice- daily oral administration, provides an average serum testosterone concentration at steady state falling in the range of about 300 to about 1100 ng/dL.
55. The method of embodiment 54, wherein the oral pharmaceutical composition comprises at least one hydrophilic surfactant comprises Cremophor RH 40 (polyoxyethyleneglycerol trihydroxystearate).
56. The method of embodiment 54, wherein the lipophilic surfactant comprises oleic acid.
57. The method of embodiment 54, wherein the oral pharmaceutical composition comprises about 18 to 22 percent by weight of solubilized testosterone undecanoate.
58. The method of embodiment 54, wherein the testosterone undecanoate is solubilized in a carrier substantially free of ethanol.
59. The method of embodiment 54, wherein the oral pharmaceutical composition comprises 15 to 17 percent by weight of the at least one hydrophilic surfactant.
60. The method of embodiment 54, wherein the oral pharmaceutical composition comprises 50 to 55 percent by weight of the at least one lipophilic surfactant.
61. A method of treating chronic testosterone deficiency in a subject in need thereof comprising the steps of:
   a. administering daily to the subject a morning dose and an evening dose of an oral pharmaceutical composition comprising testosterone undecanoate,
      wherein each dose is administered within about 30 minutes of consuming a meal, for a period of at least thirty days;
   b. measuring the serum testosterone concentration in the subject about three to five hours following the morning administration of the oral pharmaceutical composition;
   c. increasing each dose of testosterone undecanoate administered in step a. byabout80 mg when the measured serum testosterone concentration in the subject is less than about 250 ng/dL, decreasing each dose of testosterone undecanoate administered in step a. by about 40 mg to about 80 mg when the measured serum testosterone concentration in the subject is greater than about 700 ng/dL, and maintaining each dose of testosterone undecanoate administered in step a. when the measured serum testosterone concentration in the subject is between about 250 ng/dL and 700 ng/dL; and
   d. repeating steps a.-c. until the serum testosterone concentration in the subject is between about 250 and 700 ng/dL.
62. The method of embodiment 61, wherein the oral pharmaceutical composition comprises about 19.8 percent by weight of solubilized testosterone undecanoate, about 51.6 percent by weight of oleic acid, about 16.1 percent by weight of polyoxyethylene (40) hydrogenated castor oil, about 10 percent by weight of borage seed oil, about 2.5 percent by weight of peppermint oil, and about 0.03 percent by weight of butylated hydroxytoluene (BHT).
63. The method of embodiment 61, wherein each morning and evening dose initially comprises about 317 mg of testosterone undecanoate.
64. The method of embodiment 1, wherein the initial amount of testosterone ester in the oral pharmaceutical composition is administered in one or more capsules.
65. The method of embodiment 1, wherein the initial amount of testosterone ester in the oral pharmaceutical composition is administered in two capsules.
66. The method of embodiment 1, wherein the oral pharmaceutical composition comprises:
   a. 10-20 percent by weight of solubilized testosterone ester;
   b. about 5-20 percent by weight of hydrophilic surfactant;
   c. about 50-70 percent by weight of lipophilic surfactant; and
   d. about 1-10 percent by weight of polyethylene glycol 8000,
   wherein the oral pharmaceutical composition is free of ethanol, and exhibits a percent (%) in vitro dissolution profile in 5% Triton X-100 solution in phosphate buffer, pH 6.8, that indicates release from the composition of substantially all of the solubilized testosterone ester within about 2 hours.
67. The method of embodiment 66, comprising 15-20 by weight of solubilized testosterone ester.
68. The method of embodiment 67, wherein said solubilized testosterone ester is testosterone undecanoate.
69. The method of embodiment 66, wherein said hydrophilic surfactant is hydrogenated castor oil ethoxylate.
70. The method of embodiment 66, wherein said lipophilic surfactant is glyceryl monolinoleate.
71. The method of embodiment 66, wherein said oral pharmaceutical composition comprises:
   a. 15 percent by weight of solubilized testosterone undecanoate;
   b. 16 percent by weight of polyoxyethylene (40) hydrogenated castor oil;
   c. 63 percent by weight of glyceryl monolinoleate; and
   d. 6 percent by weight of polyethylene glycol 8000.
72. A method of treating a population of humans suffering from chronic testosterone deficiency comprising the steps of:
   a. administering daily to the subject a dose of an oral pharmaceutical composition comprising a testosterone ester solubilized in a carrier comprising at least one lipophilic surfactant and at least one hydrophilic surfactant;
   b. measuring the serum testosterone concentration in the subject; and
   c. increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL,
   wherein, after treatment, less than 25% of the population has a serum testosterone C_{avg} below 300 ng/dL, less than 25% of the population has a serum testosterone C_{avg} above 1000 ng/dL, and 75% of the population has a serum testosterone C_{avg} between 300 ng/dL and 1000 ng/dL.
73. A method of treating a population of humans suffering from chronic testosterone deficiency comprising the steps of:
   a. administering daily to the subject a dose of an oral pharmaceutical composition comprising a testosterone ester solubilized in a carrier comprising at least one lipophilic surfactant and at least one hydrophilic surfactant;
   b. measuring the serum testosterone concentration in the subject; and
   c. increasing the dose of testosterone ester administered in step a. when the measured serum testosterone concentration in the subject is less than about 250 ng/dL, decreasing each dose of testosterone ester administered in step a. when the measured serum testosterone concentration in the subject is greater than about 700 ng/dL, and maintaining each dose of testosterone ester administered in step a. when the measured serum testosterone concentration in the subject is between about 250 ng/dL and about 700 ng/dL,
   wherein, after treatment, less than 85% of the population has a serum testosterone Cₘₐₓ below 1500 ng/dL, less than 15% of the population has a serum testosterone Cₘₐₓ above 1500 ng/dL, less than 5% of the population has a serum testosterone Cₘₐₓ above 1800 ng/dL, and 0% of the population has a serum testosterone Cₘₐₓ above 2500 ng/dL.

## Claims

**1.** An oral pharmaceutical composition comprising testosterone undecanoate for use in a method of treating chronic testosterone deficiency in a subject in need thereof, the method comprising the steps of:
a. administering daily to the subject a morning dose and an evening dose of the oral pharmaceutical composition-comprising testosterone undecanoate, wherein each dose is administered within about 30 minutes of consuming a meal, for a period of at least thirty days;
b. measuring the serum testosterone concentration in the subject about three to five hours following the morning administration of the oral pharmaceutical composition;
c. increasing each dose of testosterone undecanoate administered in step a. by about 80 mg when the measured serum testosterone concentration in the subject is less than about 250 ng/dL,
decreasing each dose of testosterone undecanoate administered in step a. by about 40 mg to about 80 mg when the measured serum testosterone concentration in the subject is greater than about 700 ng/dL, and
maintaining each dose of testosterone undecanoate administered in step a. when the measured serum testosterone concentration in the subject is between about 250 ng/dL and 700 ng/dL; and
d. repeating steps a.-c. until the serum testosterone concentration in the subject is between about 250 and 700 ng/dL.

**2.** An oral pharmaceutical composition comprising testosterone undecanoate for use in a method of treating chronic testosterone deficiency in a subject in need thereof comprising the steps of:
a. administering twice daily to the subject a dose of the oral pharmaceutical composition comprising testosterone undecanoate solubilized in a carrier comprising at least one lipophilic surfactant and at least one hydrophilic surfactant;
b. measuring the serum testosterone concentration in the subject;
c. increasing each dose of testosterone ester administered in step a. when the measured serum testosterone concentration in the subject is less than about 250 ng/dL,
decreasing each dose of testosterone ester administered in step a. when the measured serum testosterone concentration in the subject is greater than about 700 ng/dL,
and maintaining each dose of testosterone ester administered in step a. when the measured serum testosterone concentration in the subject is between about 250 ng/dL and about 700 ng/dL;
repeating steps a.-c. until the serum testosterone concentration in the subject is between about 250 and 700 ng/dL.

**2.** The composition for use according to claim 1 or 2, wherein the oral pharmaceutical composition comprises:
a. 10-20 percent by weight of solubilized testosterone undecanoate;
b. 5-20 percent by weight of a hydrophilic surfactant;
c. 50-70 percent by weight of a lipophilic surfactant; and
d. 10-15 percent by weight of a digestible oil.

**4.** The composition for use according to claim 3, wherein the hydrophilic surfactant is selected from the group consisting of polyoxyethylene sorbitan fatty acid esters, hydrogenated castor oil ethoxylates, polyethylene glycol mono- and di- glycerol esters of caprylic, capric, palmitic and stearic acids, fatty acid ethoxylates, polyethylene glycol esters of alpha-tocopherol and its esters and combinations thereof.

**5.** The composition for use according to claim 4, wherein the hydrophilic surfactant is a hydrogenated castor oil ethoxylate.

**6.** The composition for use according to claim 3, wherein the lipophilic surfactant is a fatty acid selected from the group consisting of octanoic acid, decanoic acid, undecanoic acid, lauric acid, myristic acid, palmitic acid, pamitoleic, stearic acid, oleic acid, linoleic acid, alpha- and gamma linolenic acid, arachidonic acid and combinations thereof.

**7.** The composition for use according to claim 3, wherein the digestible oil is a vegetable oil selected from the group consisting of soybean oil, safflower seed oil, corn oil, olive oil, castor oil, cottonseed oil, arachis oil, sunflower seed oil, coconut oil, palm oil, rapeseed oil, black currant oil, evening primrose oil, grape seed oil, wheat germ oil, sesame oil, avocado oil, almond oil, borage oil, peppermint oil and apricot kernel oil and combinations thereof.

**8.** The composition for use according to claim 1, wherein the oral pharmaceutical composition comprises
about 19.8 percent by weight of solubilized testosterone undecanoate,
about 51.6 percent by weight of oleic acid,
about 16.1 percent by weight of polyoxyethylene (40) hydrogenated castor oil,
about 10 percent by weight of borage seed oil,
about 2.5 percent by weight of peppermint oil, and
about 0.03 percent by weight of butylated hydroxytoluene (BHT).

**9.** The composition for use according to claim 1, wherein each morning and evening dose initially comprises about 317 mg of testosterone undecanoate.

**10.** The composition for use according to claim 1 or 2, wherein the serum testosterone concentration is measured via a radioimmunoassay, an immunometric assay, or a liquid chromatograph tandem mass spectrometry (LC-MS/MS) assay.

**11.** The composition for use according to claim 2, wherein the initial dose of the composition administered comprises about 317 mg of testosterone undecanoate.

**12.** The composition for use according to claim 11, wherein the dose of testosterone undecanoate in the administered oral pharmaceutical composition is increased by about 40 mg to about 80 mg when the measured serum testosterone concentration in the subject is less than about 250 ng/dL.

**13.** The composition for use according to claim 11, wherein the dose of testosterone undecanoate in the administered oral pharmaceutical composition is decreased by about 40 mg to about 80 mg when the measured serum testosterone concentration in the subject is greater than about 700 ng/dL.

**14.** The composition for use according to claim 2, wherein the oral pharmaceutical composition is administered within 30 minutes of consuming a meal wherein at least about 20 percent of the calories are derived from fat.
